# EUROPÄISCHE PATENTANMELDUNG

(11) **EP 0 854 145 A2**
(43) Veröffentlichungstag der Anmeldung: **22.07.1998**
(21) Anmeldenummer: 97121931.6
(22) Anmeldetag: 12.12.1997
(51) Int. Cl.: C07D 487/04, A61K 31/44, C07D 213/00, C07D 249/00

(54) **Vitronectinrezeptor-Antagonisten, deren Herstellung sowie deren Verwendung**

(30) Priorität: 20.12.1996 DE 19961053645
(71) Anmelder: HOECHST AKTIENGESELLSCHAFT, 65929 Frankfurt am Main (DE); Genentech, Inc., South San Francisco, CA 94080-4990 (US)
(72) Erfinder: Wehner, Volkmar, Dr., 97657 Sandberg (DE); Stilz, Hans Ulrich, Dr., 65929 Frankfurt (DE); Peyman, Anuschirwan, Dr., 65779 Kelkheim (DE); Scheunemann, Karlheinz, Dr., 65835 Liederbach (DE); Ruxer, Jean-Marie, Dr., 92130 Issy les Moulineaux (FR); Carniato, Denis, Dr., 91460 Marcoussis (FR); Lefrancois, Jean-Michel, 931390 Livry Gargan (FR); Gadek, Thomas Richard, Dr., Oakland, CA 94611 (US); McDowell, Robert, Dr., San Francisco, CA 94114 (US)
(74) Vertreter: Kujath, Eckard

(57) **Zusammenfassung**

Gegenstand der vorliegenden Erfindung sind Verbindungen der Formel I,

A-B-D-E-F-G (I)

worin A, B, D, E, F und G die in den Patentansprüchen angegebenen Bedeutungen besitzen, ihre Herstellung und ihre Verwendung als Heilmittel. Die erfindungsgemäßen Verbindungen werden als Vitronectinrezeptor-Antagonisten und als Inhibitoren der Knochenresorption verwendet.

## Beschreibung

Gegenstand der vorliegenden Erfindung sind Verbindungen der Formel I

A-B-D-E-F-G (I)

in der A, B, D, E, F und G die unten angegebenen Bedeutungen haben, sowie deren physiologisch verträgliche Salze und solche Verbindungen enthaltende pharmazeutische Zubereitungen, deren Herstellung und Verwendung als Vitronectinrezeptor-Antagonisten zur Behandlung und Prophylaxe von Krankheiten, die auf der Wechselwirkung zwischen Vitronectinrezeptoren und deren Liganden bei Zell-Zell- oder Zell-Matrix-Interaktionsprozessen beruhen, wie z.B. Entzündungen, Krebs, Tumormetastasierung, cardiovaskuläre Erkrankungen wie Arteriosklerose oder Restenose, Retinopathien, Nephropathien, und Krankheiten, die auf einem unerwünschten Maß an Knochenresorption beruhen, wie z. B. Osteoporose.

Die menschlichen Knochen unterliegen einem fortwährenden dynamischen Umbauprozeß, der Knochenresorption und Knochenaufbau beinhaltet. Diese Prozesse werden von dafür spezialisierten Zelltypen gesteuert. Knochenaufbau beruht auf der Ablagerung von Knochenmatrix durch Osteoblasten, Knochenresorption beruht auf dem Abbau von Knochenmatrix durch Osteoclasten. Die Mehrzahl der Knochenerkrankungen beruhen auf einem gestörten Gleichgewicht zwischen Knochenbildung und Knochenresorption. Osteoporose ist charakterisiert durch einen Verlust an Knochenmatrix. Aktivierte Osteoclasten sind vielkernige Zellen mit einem Durchmesser bis zu 400 µm, die Knochenmatrix abtragen. Aktivierte Osteoclasten lagern sich an die Oberfläche der Knochenmatrix an und sezernieren proteolytische Enzyme und Säuren in die sogenannte "sealing zone", dem Bereich zwischen ihrer Zellmembran und der Knochenmatrix. Die saure Umgebung und die Proteasen bewirken den Abbau des Knochens.

Studien haben gezeigt, daß die Anlagerung von Osteoclasten an den Knochen durch Integrin-Rezeptoren auf der Zelloberfläche von Osteoclasten gesteuert wird.

Integrine sind eine Superfamilie von Rezeptoren, zu denen unter anderen der Fibrinogenrezeptor α_{IIb}β₃ auf den Blutplättchen und der Vitronectinrezeptor αᵥβ₃ gehören. Der Vitronectinrezeptor αᵥβ₃ ist ein membranständiges Glycoprotein, das auf der Zelloberfläche einer Reihe von Zellen wie Endothelzellen, Zellen der glatten Gefäßmuskulatur, Osteoclasten und Tumorzellen exprimiert wird. Der Vitronectinrezeptor αᵥβ₃, der auf der Osteclastenmembran exprimiert wird, steuert den Prozeß der Anlagerung an den Knochen und der Knochenresorption und trägt somit zur Osteoporose bei. αᵥβ₃ bindet hierbei an Knochenmatrixproteine wie Osteopontin, Knochensialoprotein und Thrombospontin, die das Tripeptidmotiv Arg-Gly-Asp (oder RGD) enthalten.

Die erfindungsgemäßen Verbindungen der Formel I inhibieren als Vitronectinrezeptor-Antagonisten die Knochenresorption durch Osteoclasten. Knochenkrankheiten, gegen die die erfindungsgemäßen Verbindungen eingesetzt werden können, sind vor allem Osteoporose, Hypercalcämie, Osteopenie, z. B. hervorgerufen durch Metastasen, Zahnerkrankungen, Hyperparathyroidismus, periarticulare Erosionen bei rheumathoider Arthritis und Paget-Krankheit. Ferner können die Verbindungen der Formel I zur Linderung, Vermeidung oder Therapie von Knochenerkrankungen, die durch eine Glucocortikoid-, Steroid- oder Corticosteroid-Therapie oder durch einen Mangel an Sexualhormon(en) hervorgerufen werden, eingesetzt werden. Alle diese Erkrankungen sind durch Knochenverlust gekennzeichnet, der auf dem Ungleichgewicht zwischen Knochenaufbau und Knochenabbau beruht.

Horton und Mitarbeiter beschreiben RGD-Peptide und einen anti-Vitronectinrezeptor Antikörper (23C6), die den Zahnabbau durch Osteoclasten und das Wandern von Osteoclasten inhibieren (Horton et al.; Exp. Cell. Res. 1991, 195, 368). Sato et al. beschreiben in J. Cell Biol. 1990, 111, 1713 Echistatin, ein RGD-Peptid aus Schlangengift, als potenten Inhibitor der Knochenresorption in einer Gewebekultur und als Hemmstoff der Osteoclasten-Anheftung an den Knochen. Fischer et al. (Endocrinology, 1993, 132, 1411) konnten an der Ratte zeigen, daß Echistatin die Knochenresorption auch in vivo hemmt.

Der Vitronectinrezeptor αᵥβ₃ auf humanen Zellen der glatten Gefäßmuskulatur der Aorta stimuliert die Wanderung dieser Zellen in das Neointima, was schließlich zu Arteriosklerose und Restenose nach Angioplastie führt (Brown et al., Cardiovascular Res. 1994, 28, 1815).

Brooks et al. (Cell 1994, 79, 1157) zeigen, daß Antikörper gegen αᵥβ₃ oder αᵥβ₃-Antagonisten eine Schrumpfung von Tumoren bewirken können, indem sie die Apoptose von Blutgefäßzellen während der Angiogenese induzieren. Cheresh et al. (Science 1995, 270, 1500) beschreiben anti αᵥβ₃-Antikörper oder αᵥβ₃-Antagonisten, die bFGF induzierte Angiogeneseprozesse im Rattenauge inhibieren, was therapeutisch bei der Behandlung von Retinopathien nützlich sein könnte.

In der Patentanmeldung WO 94/12181 werden substituierte aromatische oder nichtaromatische Ringsysteme, in WO 94/08577 substituierte Heterocyclen als Fibrinogenrezeptor-Antagonisten und Inhibitoren der Blättchenaggregation beschrieben. Aus EP-A-0 528 586 und EP-A-0 528 587 sind Aminoalkyl- oder Heterocyclyl-substituierte Phenylalanin-Derivate, aus WO 95/32710 Arylderivate als Hemmstoffe der Knochenresorption durch Osteoclasten bekannt. Die WO 96/00574 und WO 96/26190 beschreiben Benzodiazepine als Vitronectin- bzw. Integrinrezeptor-Antagonisten. In der WO 96/00730 werden Fibrinogenrezeptorantagonisten-Template, insbesondere Benzodiazepine, die an einen Stickstoff tragenden 5-Ring geknüpft sind, als Vitronectinrezeptor-Antagonisten beschrieben. In den deutschen Patentanmeldungen P 19629816.4, P 19629817.2 und P 19610919.1 sowie der EP-A-0 796 855 werden substituierte aromatische Ringsysteme bzw. 5-Ringheterozyklen als Vitronectinrezeptor-Antagonisten beschrieben.

Gegenstand der vorliegenden Erfindung sind Verbindungen der Formel I,

A-B-D-E-F-G (I)

worin bedeuten:
A 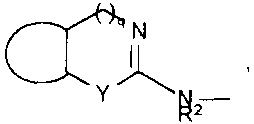 wobei 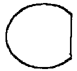
ein 5- bis 10-gliedriges mono- oder polycyclisches, aromatisches oder nicht aromatisches Ringsystem darstellt, das 1 bis 4 Heteroatome aus der Reihe N, O, S enthalten kann und gegebenenfalls ein- oder mehrfach mit R¹², R¹³, R¹⁴ und R¹⁵ substituiert sein kann;
B eine direkte Bindung, (C₁-C₈)-Alkandiyl, (C₅-C₁₀)-Arylen, (C₃-C₈)-Cycloalkylen, -C≡C-, -NR²-, -NR²-C(O)-,-NR²-C(O)-NR²-, -NR²-C(S)-NR²-, -O-C(O)-, -NR²-S(O)-, -NR²-S(O)₂-, -O-, -S-, -CR²=CR³-, die jeweils ein- oder zweifach durch (C₁-C₈)-Alkyl substituiert sein können, wie z. B. -methyl-phenylmethyl-, -ethyl-NR²-C(O)- etc.;
D eine direkte Bindung, (C₁-C₈)-Alkandiyl, (C₅-C₁₀)-Arylen, -O-, -NR²-, -CO-NR²-, -NR²-CO-, -NR²-C(O)-NR²-,-NR²-C(S)-NR²-, -OC(O)-, -C(O)O-, -S(O)-, -S(O)₂-, -S(O)₂-NR²-, -S(O)-NR²-, -NR²-S(O)-, -NR²-S(O)₂-, -S-,-CR²=CR³-, -C≡C-, die jeweils ein- oder zweifach durch (C₁-C₈)-Alkyl, -CR²=CR³-, (C₅-C₆)-Aryl substituiert sein können, wie z.B. methyl-phenyl-CH=CH-, ethyl-O- etc., wobei, wenn B eine direkte Bindung ist, D nicht gleich -CO-NR²-, -C(O)O-, -S(O)-, -S(O)₂-, -S(O)-NR²-, -S(O)₂-NR²- sein kann;
E
   a) ein Templat aus der Reihe der Fibrinogenrezeptorantagonisten bedeutet, das nachfolgenden Patentanmeldungen, Patentschriften bzw. Literaturstellen entnommen ist:
      Adir et Compagnie
         FR 928004, June 30, 1992, Fauchere, J. L., et al..
         EP 0578535, June 29, 1993, Fauchere, J. L., et al..
         CA 2128560, Jan. 24, 1995, Godfroid, J. J., et al..
      Asahi Breweries, Ltd.
         JP 05239030, Sep. 17, 1993.
      Asahi Glass
         WO 90/02751, Ohba, M. et al., Sept. 8, 1989.
         WO 90/115950, Mar. 22, 1990, Ohba, M., et al..
         EP 0406428, 1/9/91.
         WO 92/09627, Isoai, A. et al., Nov. 29, 1991.
      Chiron
         WO 93/07169, (Der 93-134382/16), Mar. 15, 1993, Devlin, J. J., et al..
      Ciba Geigy
         EP 0452210, (Der 91-305246/42) Apr, 5, 1990.
         EP 0452257, Mar. 26, 1991, Allen, M. C., et al..
      COR Therapeutics
         WO 90/15620, June 15, 1990.
         EP 0477295, Apr. 1, 1992: Scarborough, R. M., et al..
         WO 92/08472, May 29, 1992, Scarborough, R. M., et al..
         WO 93/223356, April 27, 1993, Swift, R. L., et al..
         EP 0557442, Sept. 1, 1993, Scarborough, R. M., et al..
         Scarborough, R. M.; Rose, J. W.; Hsu, M. A.; Phillips, D. R.; Fried, V. A.; Campbell, A. M.; Nunnizzi, L.; Charo, I. F., Barbourin, A GPIIb-IIIa-Specfiic Integrin Antagonist from the Venom of Sistrurus M. Barbouri, J. Biol. Chem, 266,9359,1991.
      Daiichi Pharm Co Ltd.
         JP 05078344-A, (Der 93-140339/17), Mar. 30, 1993.
      DuPont Merck
         WO 93/07170, Apr. 15, 1993.
         WO 94/11398, May 26,1994, Wells, G. J., et al..
         IL 109237, Jul. 31, 1994.
         WO 94/22909, (Der 94-333113/41) Oct. 13, 1994: DeGrado W. F., et al..
         WO 94/22910, (Der 94-333114/41) Oct. 13, 1994: DeGrado W. F., et al..
         WO 94/22494, (Der 94-332838/41) Oct. 13, 1994: DeGrado W. F., et al..
         EP 625164, Nov. 23,1994: Degrado, W. F., et al..
         WO 95/14682, June 1, 1995, Wityak, J., et al..
         WO 95/14683, June 1, 1995, Wityak, J., et al..
         WO 95/18111, July 6, 1995, DeGrado, W.F., et al..
         WO 95/23811, Sep. 8, 1995, DeGrado, W.F., et al..
         Mousa, S.A.; Bozarth, J.M.; Forsythe, M.S.; Jackson, S.M., Leamy, A.; Diemer, M. M.; Kapil, R. P; Knabb, R. M.; Mayo, M. C.; Pierce, S. K.; al., e., Antiplatelet and Antithrombotic Efficacy of DMP 728, a Novel Platelet GPIIb/IIIa Receptor Antagonist, Circulation, 89, 3, 1994.
         Jackson, S.; DeGrado, W.; Dwivedi, A.; Parthasarathy, A.; Higley, A.; Krywko, J.; Rockwell, A.; Markwalder, J.; Wells, G.; Wexler, R.; Mousa, S.; Harlow, R., Template-Constrained Cyclic Peptides: Design of High-Affinity Ligands for GPIIb/IIIa, J. Amer. Chem. Soc., 116,3220,1994.
      E. Merck
         EP 0608759, Jan. 19, 1994, Gante, J. et al..
         EP 0623615, Apr. 19, 1994, Raddatz, P. et al..
         EP 0645376, Sep. 15, 1994, Gante, J. et al..
         EP 0668278, Feb. 14, 1995, Juraszyk, H. et al..
         EP 0697408, Aug. 10, 1995, Juraszyk, H. et al..
         DE 4310643, (Der 94-311172/39), Oct. 6, 1994, Jonczyk, A., et al..
         WO 9404093, Okt. 27, 1994, Jonczyk, A., et al..
         EP 0632053, Jan. 4, 1995, Jonczyk, A., et al..
         EP 576898, Jan. 5, 1994, Jonczyk, A., et al..
         EP 0608759 A, Aug. 3, 1994, Gaute, J. P, et al..
         HU 9400249, Mai 30, 1994, Gante, J., et al..
      Ellem Ind Farma Spa
         GB 2207922, Aug. 3, 1988.
      Farmitalia Carlo Erba SRL
         EP 611765 (Der 94-265375/33), Aug. 24, 1994, Cozzi, P, et al..
      Fuji Photo Film
         JP 04208296-A (Der. 92-303598/38), Nov. 30, 1990.
         JP 04213311-A (Der. 92-305482/38), Nov. 27, 1990.
         JP 04217693-A (Der. 92-312284/38), Oct. 23, 1990.
         JP 04221394-A (Der. 92-313678/38), Oct. 26, 1990.
         JP 04221395-A (Der. 92-313679/38), Oct. 26.1990.
         JP 04221396-A (Der. 92-313680/38), Oct. 26, 1990.
         JP 04221397-A (Der. 92-313681/38), Dec. 20, 1990.
         EP 0482649 A2, April 29,1992, Kojima, M. et al..
         EP 0488258A2, June 3,1992, Komazawa, H., et al..
         EP 0503301-A2, Feb. 14, 1991, Ktaguchi, H. et al..
         JP 05222092, May 21, 1993, Nishikawa, N., et al..
         JP 06239885, (Der 94-313705/39), Aug. 30, 1993, Nishikawa, N., et al..
         WO 9324448, (Der 93-405663/50), Dec. 9,1993, Nishikawa, N., et al..
         JP 06228189, (Der 94-299801/37), Aug. 16, 1994.
         EP 0619118, (Der 94-311647/39), Oct. 12, 1994, Nishikawa, N., et al..
      Fujisawa
         EP 0513675, May 8,1992, N.Umekita, et al..
         WO 9409030-A1, Apr. 28, 1994, Takasugi, H., et al..
         EP 0513675, (Der 92-383589/47).
         WO 9500502, Jan. 5, 1995, Oku, T., et al..
         WO 95/08536, March 30, 1995, Ohkubo, M., et al..
         FR 144633: Thromb Haem. 69, 706, 1993.
         Cox, D.; Aoki, T.; Seki, J.; Motoyama, Y; Yoshida, K., Pentamidine: A Specific Nonpeptide GPIIb/IIIa Antagonist, Thromb. Haem., 69, 707, 1993.
      Genentech
         WO 90/15072 (Der 91007159).
         WO 91/01331 (Der 91058116), July 5,1990, P.L.Barker, et al..
         WO 91/04247, Sept. 24,1990, T. R. Webb.
         WO 91/11458 (Der 91252610), Jan. 28, 1991, P.L. Barker, et al..
         WO 92/07870, Oct. 24, 1991 J. P. Burnier, et al..
         WO 92/17492, Oct. 15, 1992, Burnier, J. P, et al..
         US 5250679, Oct. 5, 1993, Blackburn, B.K., et al..
         US 5403836, Apr. 4, 1995, Blackburn, B.K., et al..
         US 5565449 Oct. 15, 1996, Blackburn, B.K. et al..
         CA 2106314, Oct. 6, 1992, Burnier, J. P, et al..
         WO 93/08174, Oct. 15,1991, B. K. Blackburn, et al..
         CA 2106314, Oct. 6,1992, Burnier, J. P, et al..
         EP 0555328, Aug. 18, 1993, J. P. Burnier, et al..
         WO 95/04057, Feb. 9, 1995, Blackburn, B. K., et al..
         Scarborough, R. M., Naughton, M. A., Teng, W., Rose, J. W., Phillips, D. R., Nannizzi, L, Arfsten, A., Campbell, A. M., and Charo, I. F., J. Biol. Chem. 268, 1066, 1993.
         Dennis, M. S.; Henzel, W. J.; Pitti, R. M.; T., L. M.; Napier, M. A.; Deisher, T. A.; Bunting, S.; Lazarus, R., Platelet Glycoprotein IIb-IIIa Protein Antagonists from Snake Venoms: Evidence for a Family of Platelet-Aggregation Inhibitors, Proc. Natl. Acad. Sci. USA, 87, 2471, 1989.
         Barker, P. L.; Bullens, S.; Bunting, S., Burdick, D. J.; Chan, K. S.; Deisher, T.; Eigenbrot, C.; Gadek, T. R.; Gantzos, R.; Lipari, M. T.; Muir, C. D.; Napier, M. A.; Pitti, R. M.; Padua, A.; Quan, C.; Stanley, M.; Struble, M.; Tom, J. Y K.; Burnier, J., P, Cyclic RGD Peptide Analogues as Antiplatelet Antithrombotics, J. Med. Chem., 35, 2040, 1992.
         McDowell,. R. S.; Gadek, T. R., Structural Studies of Potent Constrained RGD Peptides, J. Amer. Chem Soc. , 114, 9245, 1992.
      Glaxo
         EP 0537980, Oct. 13, 1992, B. Porter, et al..
         EP 0542363, Nov. 10, 1992, Porter, B., et al..
         WO 93/10091, May 27, 1993, Porter, B., et al..
         WO 93/14077, July 22, 1993, Porter, B., et al..
         WO93/22303, Jan. 11, 1993,Middlemiss, D., et al..
         WO 93/14077, Jan. 15, 1993, B. Porter, et al..
         EP 0609282 A1, Aug. 10, 1994, Porter, B., et al..
         EP 0612313, Aug. 31, 1994, Porter, B., et al..
         EP 903911769, Apr. 20, 1994, Middlemiss, D., et al..
         EP 0637304 A1, Feb. 8, 1995, Middlemiss, D., et al..
         Hann, M. M.; Carter, B.; Kitchin, J.; Ward, P.; Pipe, A.; Broomhead, J.; Horuby, E.; Forster, M.; Perry, C., An Investigation of the Bioactive Conformation of ARG-GLY-ASP Containing Cyclic Peptides and Snake Venom Peptides which Inhibit Human Platelet Aggregation, In Molecular Recognition: Chemical and Biochemical Problems II", S. M. Roberts, Ed., The Royal Society of Chemistry, Cambridge, 1992.
         Ross, B. C. Nonpeptide Fibrinogen Receptor Antagonists", (SAR leading to the discovery of GR 144053), In Seventh RSC-SCI Medicinal Chemistry Symposium, The Royal Society of Chemistry Fine Chemicals and Medicinals Group and SCI Fine Chemicals Group, Churchill College, Cambridge, 1993, L20.
         Pike, N. B.; Foster, M. R.; Hornby, E. J.; Lumley, P., Effect of the Fibrinogen Receptor Antagonist GR144053 Upon Platelet Aggregation Ex Vivo Following Intravenous and Oral Administration to the Marmoset and Cynomologous Monkey, Thromb. Haem., 69, 1071, 1993.
      Hoffmann-La Roche
         AU 9344935, (Der 94-118783/15), Mar. 10, 1994.
         EP 0592791, Apr. 20, 1994, Bannwarth. W., et al..
      Kogyo Gijutsuin
         JP 06179696, Jun. 28, 1994, Maruyama, S., et al..
      Kyowa Hakko Kogyo KK
         JP 05078244-A, März. 30, 1993.
      Laboratoire Chauvin
         WO 9401456, Jan. 20,1994, Regnouf, D. V J., et al..
      La Jolla Cancer Res. Fndn
         WO 9500544, Jan. 5, 1994, Pierschbacher, M. D., et al..
         US 079441, Jan. 5, 1994, Pierschbacher, M. D., et al..
      Lilly/COR
         EP 0635492, Jan. 25, 1995, Fisher, M. J., Happ, A. M., Jakubowski, J. A.. Knnick, M. D., Kline, A. D., Morin, Jr., J. M., Sall, M. A., Vasileff, R.T.
         EP 0655439, Nov. 9, 1994, Denney, M.L. et al.
      Medical University of South Carolina
         EP 587770, März 23,1994, Halushka, P. V, Spicer, K. M..
      Merck
         EP 0368486 (Der 90-149427/20), Nov. 10, 1988.
         EP 0382451 (Der 90248531).
         EP 0382538 (Der 90248420).
         EP 0410537, Jul. 23, 1990, R. F. Nutt, et al..
         EP 0410539, Jul. 25, 1990, R. F. Nutt, et al..
         EP 0410540, Jul. 25, 1990, R. F. Nutt, et al..
         EP 0410541, Jul. 25, 1990, R. F. Nutt, et al..
         EP 0410767, Jul. 26, 1990, R. F. Nutt, et al..
         EP 0411833, Jul. 26, 1990, R. F. Nutt, et al..
         EP 0422937, Okt. 11, 1990, R. F. Nutt, et al..
         EP 0422938, Okt. 11, 1990, R. F. Nutt, et al..
         EP 0487238, Okt. 13. 1991, T. M. Connolly, et al..
         EP 0437367 (Der 91209968), M. Sato, et al..
         WO 9409029, Apr. 28, 1994, Nutt, R. F. and Veber, D. F..
         EP 618225, (Der 94-304404/38) Oct. 5, 1994.
         EP 0479481, Sept. 25, 1991, M. E. Duggan, et al..
         EP 0478362, Sept. 27, 1991 M. E. Duggan, et al..
         EP 0512831, Mai, 7, 1992, Duggan, M. E., et al..
         EP 0540334, Okt. 29, 1992, G. D. Hartman, et al..
         US 5264420-A, Nov. 23, 1993.
         US 5272158, Dez. 21, 1993, Hartmann G. D., et al..
         US 5281585, Jan. 25, 1994, Ihle, N., et al..
         GB 945317 A, März 17, 1994 (Priority US 34042A, Mar. 22, 1993).
         GB 2271567 A, Apr. 20, 1994, Hartman, G. D., et al..
         WO 9408962, Apr. 28, 1994, Hartmann, G.D., et al..
         WO 9409029, (Der 94-151241/18) Apr. 28, 1994, Hartman, G. D., et al..
         US 5321034, Juni 14, 1994, Duggan, M. E., et al..
         US 5334596, Aug. 2, 1994, Hartman, G. D., et al..
         US 5328900, Jul. 12, 1994, Klein, S. I., et al..
         US 5332726, Jul. 26, 1994, Klein, S. I., et al..
         US 5451578, Sep. 19, 1995, Claremon, D. A., et al..
         US 5455243, Okt. 3, 1995, Duggan, M. E., et al..
         WO 9418981, (Der 94-293975/36) Sep. 1, 1994, Claremon, D. A., et al..
         GB 2276384, (Der 94-287743/36) Sep. 28, 1994, Claremon, D. A., Liverton, N..
         WO 9422825, Oct. 13, 1994, Claremon, D. A.. Liverton, N. J..
         WO 9504531, Feb. 16, 1995, Hartman, G D., et al..
         WO 95/17397, Juni 29, 1995, Hartmann, G.D., et al..
         Nutt, R. F.; Brady, S. F.; Colton, C. D.; Sisko, J. T.; Ciccarone, T. M.; Levy, M. R.; Duggan, M. E; Imagire, I. S.; Gould, R. J.; Anderson, P. S.; Veber, D. F., Development of Novel, Highly Selective Fibrinogen Receptor Antagonists as Potentially Useful Antithrombotic Agents, In Peptides, Chemistry and Biology, Proc. 12th Amer. Peptide Symp., J. A. Smith and J. E. Rivier, Ed., ESCOM, Leiden, 1992; 914.
         Hartman, G. D.; Egbertson, M. S.; Halszenko, W.; Laswell, W. L.; Duggan, M. E.; Smith, R. L.; Naylor, A. M.; Manno, P.D.; Lynch, R. J.; Zhang, G.; Chang, C. T. C.; Gould, R. J., Non-peptide Fibrinogen Receptor Antagonists. 1. Discovery and Design of Exosite Inhibitors, J. Med. Chem., 35, 4640, 1992.
         Gould, R. J.; Barrett, S.; Ellis, J. D.; Holahan, M. A.; Stranieri, M. T.;Theoharides, A. D.; Lynch, J. J.; Friedman, P.A.; Duggan, M. E.; Ihle, N. C.; Anderson, P.S.; Hartman, G. D., Characterization of L-703,014, A Novel Fibrinogen Receptor Antagonist, Following Oral Administration to Dogs, Thromb. Haem., 69, 539, 1993.
      Merrell Dow
         WO 93/24520, Mai 14,1993, Harbeson, S. L., et al..
         WO 9324520, Dez. 9, 1993, Harbeson, Bitonti, J., A..
         WO 9429349, Dez. 22, 1994, Harbeson, Bitonti,J., A..
      Nippon Steel Corp
         WO 9405696, März. 17, 1993, Sato, Y, et al..
         EP 628571, Dez. 14, 1994, Sato, Y, et al..
         WO 9501371, Jan. 12, 1995, Sato, Y, et al..
      ONO Pharmaceuticals
         JP 05286922 (Der 93-383035/48)
      Roche
         EP 038,362, Feb. 19, 1990, M. Muller, et al..
         EP 0372486, Juni 13, 1990, Allig, L., et al..
         EP 0381033, Juli 8, 1990, Allig, L., et al..
         EP 0384362, Aug. 29, 1990, Allig, L., et al..
         EP 0445796, Sept. 11, 1991, Allig, L., et al..
         EP 0505868, Sept. 30, 1992, Allig, L., et al..
         US 5273982-A, (Der 94-006713/01) Dec. 28, 1993.
         US 5430024, Juli 4, 1995, Allig, L., et al..
         EP 0468231, Juli 2, 1991, Ackermann, J., et al..
         EP 0656348, Nov. 26, 1994, Allig, L., et al..
         Allig, L.; Edenhofer, A.; Hadvary, P.; Hurzeler, M.; Knopp, D.; Muller, M.; Steiner, B.; Trzeciak, A.; Weller, T., Low Molecular Weight, Non-peptide Fibrinogen Receptor Antagonists, J. Med. Chem. , 35, 4393, 1992.
      Rhone-Poulenc Rorer
         US 4952562, Sept. 29, 1989, S. I. Klein, et al..
         US 5064814, (Der 91-353169/48) Apr. 5, 1990.
         WO 9104746, Sept. 25, 1990, S. I. Klein et al..
         WO 91/05562, Okt. 10, 1989, S. I. Klein et al..
         WO 91/07976, (Der 91-192965) Nov. 28, 1990, S. I. Klein, et al..
         WO 91/04746, S. I. Klein, et al..
         WO 92/18117, Apr. 11, 1991, S. I. Klein, et al..
         US 5086069, (Der 92-064426/08) Apr. 2, 1992.
         WO 92/17196, März 30, 1992,. S. I. Klein, et al..
         US 5328900, (Der 94-221950/27) Jul. 12, 1992.
         US 5332726, (Der 94-241043/29) Jul. 26, 1994.
         WO 93/11759, Dez. 7, 1992, S. I. Klein, et al..
         EP 0577775, Jan. 12, 1994, Klein, S. I., et al..
         WO 95/10295, Apr. 20, 1995, Klein, S.I., et al..
         CA 2107088, Sept. 29, 1992, Klein, S.I., et al..
      Sandoz
         EP 0560730, März 8, 1993, G. Kottirisch and R. Metternich.
         G. Kottirisch, et al.. Biorg. Med. Chem. Lett 3, 1675-1680, 1993.
      Schering AG
         EP 530937, März. 10, 1993, Noeski-Jungblut, C., et al..
      Searle / Monsanto
         EP 0319506, (Der 89-3195506) Dec.2, 1988, S.P. Adams, et al..
         EP 0462,960, Juni 19. 1991, Tjoeng, F.S., et al..
         US 4857508, S. P. Adams, et al..
         EP 0502536, (Der 92-301855) März 3, 1991, R. B. Garland, et al..
         EP 0319506, Dez. 2, 1988, S. P. Adams, et al..
         US 4992463, Aug. 18, 1989.
         US 5037808, Apr. 23,1990.
         EP 0454651 A2, Okt 30, 1991, Tjoeng, F. S., et al..
         US 4879313, Juli 20, 1988.
         WO 93/12074, Nov. 19, 1991, N. Abood, et al..
         WO 93/12103, Dez. 11, 1991, P. R. Bovy, et al..
         US 5091396, Feb. 25, 1992, Tjoeng, F. S., et al..
         WO 92/15607, März. 5, 1992, Garland, R. B., et al..
         WO 93/07867, Apr. 29, 1993, P. R. Bovy, et al..
         US 888686, Mai 22, 1992, Bovy, P. R., et al..
         CA 2099994, Sept. 7, 1992, Garland, R, B., et al..
         EP 0513810, Mai 15, 1992, Garland, R. B., et al..
         US 5254573, Okt. 19, 1993, Bovy, P. R., et al..
         EP 0539343, Oct. 14, 1992, P. R. Bovy, et al..
         WO 93/12074, Nov. 27, 1992, N. A. Abood, et. al..
         WO 93/12103, Dez. 11, 1992, P. R. Bovy, et al..
         EP 0539343, Apr. 28, 1993, Bovy, P. R., et al..
         EP 0542708, Mai, 19, 1993, Bovy. P. R., et al..
         WO 94/00424, Jan. 6, 1994, Abood, N. A., et al..
         WO 93/16038, Aug. 16, 1993, Miyano. M., et al..
         WO 93US7975, Aug. 17, 1993, Zablocki, J. A., Tjoeng, F. S..
         WO 93/18058, Sept. 16, 1993, Bovy, P. R., et al..
         US 5254573, Okt. 19, 1993, Bovy, P. R., et al..
         US 5272162, Dez. 21, 1993, Tjoeng, F. S., et al..
         EP 0574545, Dez. 22, 1993, Garland, R. B., et al..
         WO 9401396, Jan. 20, 1994, Tjoeng, F. S., et al..
         WO 9405694, (Der 94-101119/12) März 17, 1994, Zablocki, et al..
         US 5314902, Mai 24, 1994, Adams, S. P, et al..
         WO 9418162, Aug. 18, 1994, Adams, S. P., et al..
         WO 9419341, Sept. 1, 1994, Tjoeng, F. S., et al..
         US 5344837, (Der 94-285503/35), Sept. 6, 1994, Zablocki, J. A., et al..
         EP 614360, Sept. 14, 1994, Bovy, P. R., et al..
         WO 9420457, (Der 94-302907/37), Sep. 15, 1994, Tjoeng, F. S., et al..
         WO 9421602, (Der 94-316876/39), Sept. 29, 1994, Tjoeng, F. S., et al..
         WO 9422820, Okt. 13, 1994, Abood, N. A., et al..
         EP 630366, Dez. 28, 1994, Bovy, P. R., et al..
         US 5378727, Jan. 3, 1995, Bovy, P. R., et al..
         WO 95/06038, März 2, 1995, Bovy P.R., et al..
         WO 93/08164, Apr. 29, 1993, Bovy, P.R., et al..
         K. F. Fok, et al.., Int. J. Peptide Prot. Res., 38, 124-130, 1991, SAR of RGDY analogs. J. A. Zablocki, et al.. J. Med. Chem. 35, 4914-4917, 1992, SAR summary of guanidinoalkanoyl-Asp-Phe analogs.
         Tjoeng, F. S.; Fok, K. F.; Zupec, M. E.; Garland, R. B.; Miyano, M.; Panzer-Knodle, S.; King, L. W.; Taite, B. B.; Nicholson, N. S.; Feigen, L. P; Adams, S. P, Peptide Mimetics of the RGD Sequence, In Peptides, Chem. and Biol. Proc. 12th Amer. Peptide Symp., J. A. Smith and J. E. Rivier, Ed., ESCOM, Leiden, 1992; 752.
         Nicholson, N.; Taite, B.; Panzer-Knodle, S.; Salyers, A.; Haas, N.; Szalony, J.; Zablocki, J.; Feigen, L.; Glenn, K.; Keller, B.; Broschat, K.; Herin, M.; Jacqmin, P; lesne, M., An Orally Active Glycoprotein IIb/IIIa Antagonist-SC-54684, Thromb. Haem, 69, 975, 1993.
      Smithkline Beecham Corporation
         WO 91/07429, Mai 30, 1991, Ali, F., et al..
         WO 92/07568, Mai 14, 1992, Callahan, J. F., et al..
         WO 92/13552, Aug. 20, 1992, Ali, F., et al..
         WO 93/00095, Jan. 7, 1993, Bondinell, W. E., et al..
         WO 93/09133, Mai 13, 1993, Callahan, J. F., et al..
         WO 94/12478, Juni 9, 1994, Keenan, R. M. C., et al..
         WO 94/14775, Juli 7, 1994, Bondinell, W. E., et al..
         WO 94/22440, Okt. 13, 1994, Callahan, J. F., et al..
         WO 94/14776, Juli 7, 1994, Bondinell, W. E., et al..
         WO 94/15913, Juli 21, 1994, Samanen, J..
         WO 94/29273, Dez. 22, 1994, Samanen, J..
         WO 95/18619, Juli 13, 1995, Bondinell, W. E., et al..
         WO 96/06087, Feb. 29, 1996, Kwon, C., et al..
         WO 96/00730, Jan. 11, 1996, Ali, F., et al..
      Sumitomo Pharm. Co. Ltd
         WO 9501336, Juni 6, 1994, Ikeda, Y, et al..
      Sumitomo Seiyaku KK
         JP 06025290, (Der 94-077374/10), Feb. 1, 1994.
      Taisho Pharm. (Teijin, Ltd)
         JP 05230009, (Der 93-317431/40), Feb. 24, 1992.
         JP 9235479, Feb. 24, 1992.
         WO 94/17804, Aug. 18, 1994, Mizushima, Y.
         EP 0634171, Jan. 18, 1995, Nizushima, M..
      Takeda
         EP 0529858, Apr. 3, 1993, H. Sugihara, et al..
         EP 0606881, Jul. 20, 1994.
         EP 0614664, Sept. 14, 1994, Miyake, A., et al..
      Tanabe
         WO 89/07609, T. J. Lobl, et al..
         WO 92/00995, Juli 9, 1991, T. J. Lobl, et al..
         WO 93/08823, Nov. 6, 1991, T. C. McKenzie.
         CA 2087021, Jan. 10, 1991, Lobl, T. J., et al..
         WO 92/08464, Nov. 15, 1991, T. C. McKenzie, et al..
      Telios / La Jolla Cancer Research
         US 4578079, Nov. 22, 1983, E. Ruoslahti, and M. Pierschbacher.
         US 4614517, Juni 17, 1985, E. Ruoslahti, and M. Pierschbacher.
         US 4792,525, Juni 17, 1985, E. Ruoslahti, and M. Pierschbacher.
         US 4879237, (Der 90-154405/20) Mai, 24, 1985.
         WO 91/15515, (Der 91-325173/44) Apr. 6, 1990.
         US 5041380, 1991, E. Ruoslahti, and M. Pierschbacher.
         WO 95/00544 Jan. 5, 1995, Craig, W. S., et. al..
         Cheng, S.; Craig, W. S.; Mullen, D.; Tschopp, J. F.; Dixon, D.; Pierschbacher, M. F., Design and Synthesis of Novel Cyclic RGD-Containing Peptides as highly Potent and Selective Integrin αvβ3 Antagonists, J Medicin. Chem. 37, 1, 1994.
         Collen, D.; Lu, H. R.; Stassen, J.-M.; Vreys, I.; Yasuda, T.; Bunting, S.; Gold, H. K., Antithrombotic Effects and Bleeding Time Prolongation with Synthetic Platelet GPIIb/IIIa Inhibitors in Animal Models of Platelet-Mediated Thrombosis, Thrombosis and Haemostasis, 71, 95, 1994.
      Temple U.
         WO 9409036, (Der 94-151248/18), Apr. 28, 1994.
      Terumo KK
         JP 6279389, Okt. 4, 1994, Obama, H., et al..
      Karl Thomae / Boehringer Ingelheim
         EP 0483667, Mai 6, 1992, Himmelsbach, F., et al..
         EP 0496378, Jan. 22, 1992, Himmelsbach, F., et al..
         EP 0503548, Sep. 16, 1992, Himmelsbach, F., et al..
         AU A-86926/91, Mai 7, 1992, Himmelsbach, F., et al..
         EP 0528369, Feb. 24, 1993, Austel, V, et al..
         EP 0537696, Apr. 21, 1993, Linz, G., et al..
         DE 4124942, Jan. 28, 1993, Himmelsbach, F., et al..
         DE 4129603, März 11, 1993, Pieper, H, et al..
         EP 0547517 A1, (Der 93-198544) June 23, 1993, Soyka, R., et al..
         EP 0567966, Nov. 3, 1993, Himmelsbach, F., et al..
         EP 0567967, Nov. 3 1993, Weisenberger, J., et al..
         EP 0567968, Nov. 3, 1993, Linz, G., et al..
         EP 0574808, Juni 11, 1993, Pieper, H., et al..
         Der 93-406657/51, Austel, V , et al..
         EP 587134, (Der 94-085077111) Mar. 16, 1994, Himmelsbach, F., et al..
         EP 589874, Apr. 6, 1994, Grell, W., et al..
         (P534005), DE 4234295, Apr. 14, 1994, Pieper, H., et al..
         EP 0592949, Apr. 20, 1994, Pieper, H. D., et al..
         EP 0596326, Mai, 11, 1994, Maier, R., et al..
         DE 4241632, Juni 15, 1994, Himmelsbach, F., et al..
         EP 0525629, Juli 22, 1992, Himmelsbach, F., et al..
         EP 0531883, Sep. 3, 1992, Austel., V, et al..
         EP 0604800 A, Juli 6, 1994, Himmelsbach, F., et al..
         DE 4302051, (Der 94-235999/29) July 28, 1994.
         EP 0608858 A, Aug. 3, 1994, Linz, G. D., et al..
         DE 4304650, (Der 94-256165/32), Aug. 18, 1994, Austel, V., et al..
         EP 611660, Aug. 24, 1994, Austel, V., et al..
         EP 0612741, Feb. 21, 1994, Himmelsbach, F., et al..
         DE 4305388, (Der 94-264904/33), Aug. 25, 1994, Himmelsbach, F., et al..
         EP 612741, (Der 94-265886/33), Aug. 31, 1994, Himmelsbach, F., et al..
         EP 0639575 A, Feb. 22, 1995, Linz, G., et al..
         DE 4324580, Jan. 26, 1995, Linz, G., et al..
         EP 0638553, Feb. 15, 1995, Himmelsbach, F., et al..
         WO 95/24405, Sep. 14, 1995, Himmelsbach, F., et al..
         WO 96/02514, Feb. 1, 1996, Himmelsbash, F., et al..
         WO 96/02504, Feb. 1, 1996, Himmelsbach F., et al..
         DE 4427838, Feb. 8, 1996, Himmelsbach, F., et al..
         WO 96/05194, Feb. 22, 1996, Himmelsbach, F., et al..
         DE 4431868, März 14, 1996, Pieper, H. et al..
         DE 4429079, Feb. 22, 1996, Himmelsbach, F. et al..
         F. Himmelsbach, V. Austel, G. Kruger, H. Pieper, H. Weisenberger, T. H. Muller, und W. G. Eisert, in Xllth Int. Symp. on Med. Chem. Basel, Book of Abstracts, 47, 1992.
         V. Austel, W. Eisert, F. Himmelsbach, G. Kruger, G. Linz, T. Muller, H. Pieper, und J. Weisenberger, Natl. Mtg. Amer. Chem. Soc. Book of Abstracts, Denver, Div. Med. Chem., 1993.
         Muller, T. H.; Schurer, H.; Waldmann, L.; Bauer, E.; Himmelsbach, F.; Binder, K., Orally Activity of BIBU 104, a Prodrug of the Non-peptide Fibrinogen Receptor Antagonist BIBU 52, in Mice and Monkeys, Thromb. Haem., 69, 975, 1993.
      Univ. Califorrnia
         WO 94/14848, July 7, 1994, Zanetti, M.
      Univ. New York
         WO 94/00144, Juni 29,1993, Ojima, I., et al..
      Yeda Res. and Dev. Co.
         WO 93/09795, (Der 93-182236/22), Lido, O., et al..
      Zeneca
         WO 9422834, Okt. 13, 1994, Wayne, M. G., et al..
         WO 9422835, Okt. 13, 1994, Wayne, M. G., et al..
         EP 632016, Jan. 4, 1995, Brewster, A. G.., et al..
         EP 632019, Jan. 4, 1995, Brown, G., Shute, R. E..
         EP 632020, Jan. 4, 1995, Brown, G., Shute, R. E..
         WO 95/00472, Jan. 5, 1995, Brewster, A. G., et al..
      oder
   b) ein Templat, definiert analog den Templaten aus der Reihe der Fibrinogenrezeptorantagonisten, bedeutet, das nachfolgenden Patentanmeldungen entnommen ist:
      Smithkline Beecham Corp.
         WO 96/00574, Jan. 11, 1996, Cousins, R.D. et al.
      Fujisawa Pharmaceutical Co.
         WO 95/29907, Nov. 9, 1995, Kawai, Y. et al.,
      Eli Lilly
         US 5 488 058, Jan. 30, 1996, Palkowitz, A.D. et al.
         US 5 484 798, Jan. 16, 1996, Bryant, H.U. et al.;
      oder auch solche Template bedeutet, die sich aus den in den vorstehenden Patentanmeldungen, Patentschriften und Publikationen beschriebenen Templaten strukturell ableiten lassen;
F ist wie D definiert;
G 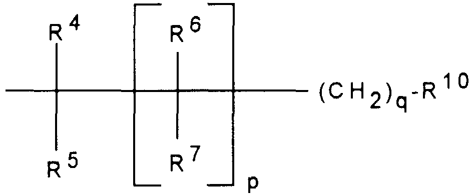
   - R², R³: unabhängig voneinander H, (C₁-C₁₀)-Alkyl, das gegebenenfalls durch Fluor ein- oder mehrfach substituiert ist, (C₃-C₁₂)-Cycloalkyl, (C₃-C₁₂)-Cycloalkyl-(C₁-C₈)-alkyl, (C₅-C₁₄)-Aryl, (C₅-C₁₄)-Aryl-(C₁-C₈)-alkyl, R⁸OC(O)R⁹, R⁸R⁸NC(O)R⁹, R⁸C(O)R⁹,
   - R⁴, R⁵, R⁶, R⁷: unabhängig voneinander H, Fluor, OH, (C₁-C₈)-Alkyl, (C₃-C₁₄)-Cycloalkyl, (C₃-C₁₄)-Cycloalkyl-(C₁-C₈)-alkyl, oder R⁸OR⁹, R⁸SR⁹, R⁸CO₂R⁹, R⁸OC(O)R⁹, R⁸-(C₅-C₁₄)-Aryl-R⁹, R⁸N(R²)R⁹, R⁸R⁸NR⁹, R⁸N(R²)C(O)OR⁹, R⁸S(O)ₙN(R²)R⁹, R⁸OC(O)N(R²)R⁹, R⁸C(O)N(R²)R⁹, R⁸N(R²)C(O)N(R²)R⁹, R⁸N(R²)S(O)ₙN(R²)R⁹, R⁸S(O)ₙR⁹, R⁸SC(O)N(R²)R⁹, R⁸C(O)R⁹, R⁸N(R²)C(O)R⁹, R⁸N(R²)S(O)ₙR⁹;
   - R⁸: H, (C₁-C₈)-Alkyl, (C₃-C₁₄)-Cycloalkyl, (C₃-C₁₄)-Cycloalkyl-(C₁-C₈)-alkyl, (C₅-C₁₄)-Aryl, (C₅-C₁₄)-Aryl-(C₁-C₈)-alkyl, wobei die Alkylreste durch Fluor ein- oder mehrfach substituiert sein können;
   - R⁹: eine direkte Bindung oder (C₁-C₈)-Alkandiyl;
   - R¹⁰: C(O)R¹¹, C(S)R¹¹, S(O)ₙR¹¹, P(O)(R¹¹)ₙ oder ein vier- bis achtgliedriger, gesättigter oder ungesättigter Heterocyclus, der 1, 2, 3 oder 4 Heteroatome aus der Reihe N, O, S enthält, wie z.B. Tetrazolyl, Imidazolyl, Pyrazolyl, Oxazolyl, Thiadiazolyl;
   - R¹¹: OH, (C₁-C₈)-Alkoxy, (C₅-C₁₄)-Aryl-(C₁-C₈)-alkoxy, (C₅-C₁₄)-Aryloxy, (C₁-C₈)-Alkylcarbonyloxy-(C₁-C₄)-alkoxy, (C₅-C₁₄)-Aryl-(C₁-C₈)-alkylcarbonyloxy-(C₁-C₆)-alkoxy, NH₂, Mono- oder Di-((C₁-C₈)-alkyl)-amino, (C₅-C₁₄)-Aryl-(C₁-C₈)-alkylamino, (C₁-C₈)-Dialkylaminocarbonylmethyloxy, (C₅-C₁₄)-Aryl-(C₁-C₈)-dialkylaminocarbonylmethyloxy oder (C₅-C₁₄)-Arylamino oder den Rest einer L- oder D-Aminosäure;
   - R¹², R¹³, R¹⁴, R¹⁵: unabhängig voneinander H, (C₁-C₁₀)-Alkyl, das gegebenenfalls durch Fluor ein- oder mehrfach substituiert ist, (C₃-C₁₂)-Cycloalkyl, (C₃-C₁₂)-Cycloalkyl-(C₁-C₈)-alkyl, (C₅-C₁₄)-Aryl, (C₅-C₁₄)-Aryl-(C₁-C₈)-alkyl, H₂N, R⁸ONR⁹, R⁸OR⁹, R⁸OC(O)R⁹, R⁸R⁸NR⁹, R⁸-(C₅-C₁₄)-Aryl-R⁹, HO-(C₁-C₈)-Alkyl-N(R²)R⁹, R⁸N(R²)C(O)R⁹, R⁸C(O)N(R²)R⁹, R⁸C(O)R⁹, R²R³N-C(=NR²)-NR², R²R³N-C(=NR²), =O, =S;
   wobei zwei benachbarte Substituenten aus R¹² bis R¹⁵ zusammen ferner -OCH₂O-,-OCH₂CH₂O-, -OC(CH₃)₂O- bedeuten können;
   - Y: NR², O, S;
   - n: 1 oder 2;
   - p, q: unabhängig voneinander 0 oder 1;
   in allen ihren stereoisomeren Formen und Mischungen davon in allen Verhältnissen, und ihre physiologisch verträglichen Salze,
   wobei Verbindungen ausgenommen sind, in denen E
   a) ein 6-gliedriges aromatisches Ringsystem, das bis zu 4 N-Atome enthalten kann und mit 1 bis 4 gleichen oder verschiedenen beliebigen Substituenten substituiert sein kann, bedeutet, oder
   b) 4-Methyl-3-oxo-2,3,4,5-tetrahydro-1-H-1,4-benzodiazepin bedeutet.

Unter einem Templat aus der Reihe der Fibrinogenrezeptorantagonisten versteht man den Mittelteil der Molekülstruktur (eines Fibrinogenrezeptorantagonisten), an den im Falle der Fibrinogenrezeptorantagonisten über Spacer eine basische und eine saure Gruppe angeknüpft sind, wobei die basische und/oder saure Gruppe gegebenenfalls in geschützter Form (Pro-Drug) vorliegen.

In den Fibrinogenrezeptorantagonisten ist die basische Gruppe im allgemeinen eine N-haltige Gruppe, wie z.B. Amidin oder Guanidin, die saure Gruppe im allgemeinen eine Carboxylfunktion, wobei die basische und saure Gruppe jeweils in geschützter Form vorliegen können.

Ein Fibrinogenrezeptorantagonist ist ein Wirkstoff, der die Bindung von Fibrinogen an den Blutplättchenrezeptor GPIIbIIIa inhibiert.

Ein Fibrinogenrezeptorantagonist besteht aus einem Mittelteil (Templat), an das über Spacer eine basische und eine saure Gruppe angeknüpft sind, wobei die basische und/oder saure Gruppe gegebenenfalls in geschützter Form (Pro-Drug) vorliegen.

Alkylreste können geradkettig oder verzweigt sein. Dies gilt auch, wenn sie Substituenten tragen oder als Substituenten anderer Reste auftreten, beispielsweise in Alkoxy-, Alkoxycarbonyl- oder Aralkylresten. Beispiele für geeignete (C₁-C₁₀)-Alkylreste sind: Methyl, Ethyl, Propyl, Butyl, Pentyl, Hexyl, Heptyl, Octyl, Decyl, Isopropyl, Isopentyl, Neopentyl, Isohexyl, 3-Methylpentyl, 2,3,5-Trimethylhexyl, sec-Butyl, tert-Pentyl. Bevorzugte Alkylreste sind Methyl, Ethyl, Propyl, Isopropyl, Butyl, Isobutyl, sec-Butyl und tert-Butyl.

Auch Alkenyl- und Alkinylreste können geradkettig und verzweigt sein. Beispiele für Alkenylreste sind Vinyl, 1-Propenyl, Allyl, Butenyl, 3-Methyl-2-butenyl, für Alkinylreste Ethinyl, 1-Propinyl oder Propargyl.

Cycloalkylreste können monocyclisch oder polycyclisch, z.B. bicyclisch oder tricyclisch, sein. Monocyclische Cycloalkylreste sind beispielsweise Cyclopropyl, Cyclobutyl, Cyclopentyl, Cyclohexyl, Cycloheptyl, Cyclooctyl, Cyclododecyl die aber auch durch beispielsweise (C₁-C₄)-Alkyl substituiert sein können. Als Beispiele für substituierte Cycloalkylreste seien 4-Methylcyclohexyl und 2,3-Dimethylcyclopentyl genannt.

Beispiele für Grundkörper monocyclischer (C₁₀-C₁₄)-Cycloalkylreste in R⁴, R⁵, R⁶, R⁷ sind beispielsweise Cyclodecan und Cyclododecan.

Bicyclische und tricyclische Cycloalkylreste können unsubstituiert sein oder in beliebigen geeigneten Positionen durch eine oder mehrere Oxogruppen und/oder eine oder mehrere gleiche oder verschiedene (C₁-C₄)-Alkylgruppen, z.B. Methyl- oder Isopropylgruppen, bevorzugt Methylgruppen, substituiert sein. Die freie Bindung des bi- oder tricyclischen Restes kann sich in einer beliebigen Position des Moleküls befinden, der Rest kann also über ein Brückenkopfatom oder ein Atom in einer Brücke gebunden sein. Die freie Bindung kann sich auch in einer beliebigen stereochemischen Position befinden, beispielsweise in einer exo- oder einer endo-Position.

Ein Beispiel für ein bicyclisches Ringsystem ist Dekalin (Decahydronaphthalin), für ein mit einer Oxogruppe substituiertes System 2-Decanon.

Beispiele für Grundkörper bicyclischer Ringsysteme sind das Norbornan (= Bicyclo[2.2.1]heptan), das Bicyclo[2.2.2]octan und das Bicyclo[3.2.1]octan. Ein Beispiel für ein mit einer Oxogruppe substituiertes System ist der Campher (= 1,7,7-trimethyl-2-oxobicyclo[2.2.1]heptan).

Beispiele für Grundkörper tricyclischer Systeme sind das Twistan (= Tricyclo[4.4.0.0^{3,8}]decan, das Adamantan (= Tricyclo[3.3.1.1^{3,7}]decan), das Noradamantan (= Tricyclo[3.3.1.0^{3,7}]-nonan), das Tricyclo[2.2.1.0^{2,6}]heptan, das Tricyclo[5.3.2.0^{4,9}]dodecan, das Tricyclo[5.4.0.0^{2,1}]undecan oder das Tricyclo[5.5.1.0^{3,11}]tridecan.

Beispiele für Grundkörper tricyclischer (C₁₀-C₁₄)-Cycloalkylreste in R⁴, R⁵, R⁶, R⁷ sind das Twistan (= Tricyclo[4.4.0.0.^{3,8}]decan, das Adamantan (= Tricyclo[3.3.1.1.^{3,7}]-nonan), das Tricyclo[5.3.2.0^{4,9}]dodecan, das Tricyclo[5.4.0.0^{2,9}]undecan oder das Tricyclo[5.5.1.0^{3,11}]tridecan.

Halogen bedeutet Fluor, Chlor, Brom, Iod.

Beispiele für 6-gliedrige aromatische Ringsysteme sind Phenyl, Pyridyl, Pyridazinyl, Pyrimidinyl, Pyrazinyl, 1,3,5-Triazinyl, 1,2,4-Triazinyl, 1,2,3-Triazinyl, Tetrazinyl.

Aryl ist beispielsweise Phenyl, Naphthyl, Biphenylyl, Anthryl oder Fluorenyl, wobei 1-Naphthyl, 2-Naphthyl und insbesondere Phenyl bevorzugt sind. Arylreste, insbesondere Phenylreste, können ein- oder mehrfach, bevorzugt ein-, zwei oder dreifach, durch gleiche oder verschiedene Reste aus der Reihe (C₁-C₈)-Alkyl, insbesondere (C₁-C₄)-Alkyl, (C₁-C₈)-Alkoxy, insbesondere (C₁-C₄)-Alkoxy, Halogen, wie Fluor, Chlor und Brom, Nitro, Amino, Trifluormethyl, Hydroxy, Methylendioxy, -OCH₂CH₂O- -OC(CH₃)₂O-, Cyano, Hydroxycarbonyl, Aminocarbonyl, (C₁-C₄)-Alkoxycarbonyl, Phenyl, Phenoxy, Benzyl, Benzyloxy, (R¹⁷O)₂P(O), (R¹⁷O)₂P(O)-O- oder Tetrazolyl substituiert sein, wobei R¹⁷ H, (C₁-C₁₀)-Alkyl, (C₆-C₁₄)-Aryl oder (C₆-C₁₄)-Aryl-(C₁-C₈)-alkyl bedeutet.

In monosubstituierten Phenylresten kann sich der Substituent in der 2-, der 3- oder der 4-Position befinden, wobei die 3- und die 4-Position bevorzugt sind. Ist Phenyl zweifach substituiert, können die Substituenten in 1,2-, 1,3-oder 1,4-Position zueinander stehen. Bevorzugt sind in zweifach substituierten Phenylresten die beiden Substituenten in der 3- und der 4-Position, bezogen auf die Verknüpfungsstelle, angeordnet.

Arylgruppen können ferner mono- oder polycyclische aromatische Ringsysteme darstellen, worin 1 bis 5 C-Atome durch 1 bis 5 Heteroatome ersetzt sein können, wie z.B. 2-Pyridyl, 3-Pyridyl, 4-Pyridyl, Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl, β-Carbolinyl, oder ein benz-anelliertes, cyclopenta-, cyclohexa- oder cyclohepta-anelliertes Derivat dieser Reste. Diese Heterocyclen können mit den gleichen Substituenten wie die vorstehend genannten carbocyclischen Arylsysteme substituiert sein.

In der Reihe dieser Arylgruppen sind bevorzugt mono- oder bicyclische aromatische Ringsysteme mit 1 bis 3 Heteroatome aus der Reihe N, O, S, die mit 1 bis 3 Substituenten aus der Reihe (C₁-C₆)-Alkyl, (C₁-C₆)-Alkoxy, F, Cl, NO₂, NH₂, CF₃, OH, (C₁-C₄)-Alkoxycarbonyl, Phenyl, Phenoxy, Benzyloxy oder Benzyl substituiert sein können.

Besonders bevorzugt sind hierbei mono- oder bicyclische aromatische 5- bis 10-Ringsysteme mit 1 bis 3 Heteroatomen aus der Reihe N, O, S, die mit 1 bis 2 Substituenten aus der Reihe (C₁-C₄)-Alkyl, (C₁-C₄)-Alkoxy, Phenyl, Phenoxy, Benzyl oder Benzyloxy substituiert sein können.

L- oder D-Aminosäuren können natürliche oder unnatürliche Aminosäuren sein. Bevorzugt sind α-Aminosäuren. Beispielsweise seien genannt (vgl. Houben-Weyl, Methoden der organischen Chemie, Band XV/1 und 2, Georg Thieme Verlag, Stuttgart, 1974):
Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hlle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, tert-Butylglycin (Tbg), Neopentylglycin (Npg), Cyclohexylglycin (Chg), Cyclohexylalanin (Cha), 2-Thienylalanin (Thia), 2,2-Diphenylaminoessigsäure, 2-(p-Tolyl)-2-phenylaminoessigsäure, 2-(p-Chlorphenyl)-aminoessigsäure;

ferner:
Pyrrolidin-2-carbonsäure; Piperidin-2-carbonsäure; 1,2,3,4-Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Azabicyclo[2.2.2]octan-3-carbonsäure; 2-Azabicyclo[2.2.1]-heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro(bicyclo[2.2.1]heptan)-2,3-pyrrolidin-5-carbonsäure; Spiro(bicyclo[2.2.2]octan)-2,3-pyrrolidin-5-carbonsäure; 2-Azatricyclo[4.3.0.1^{6,9}]decan-3-carbonsäure; Decahydrocyclohepta[b]pyrrol-2-carbonsäure; Decahydrocycloocta[c]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure, Hydroxypyrrolidin-2-carbonsäure, die alle gegebenenfalls substituiert sein können (siehe folgende Formeln):

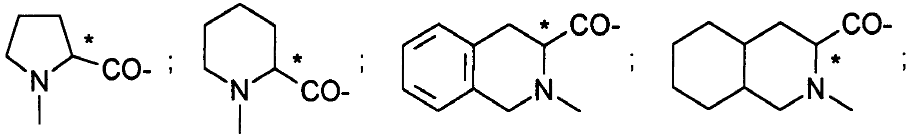

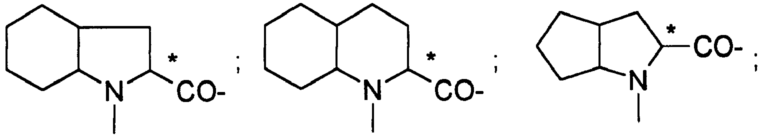

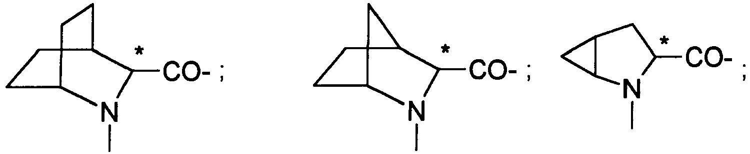

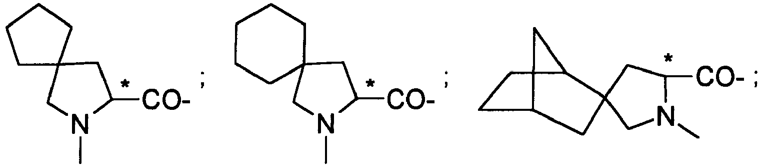

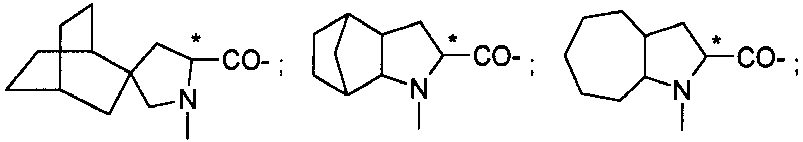

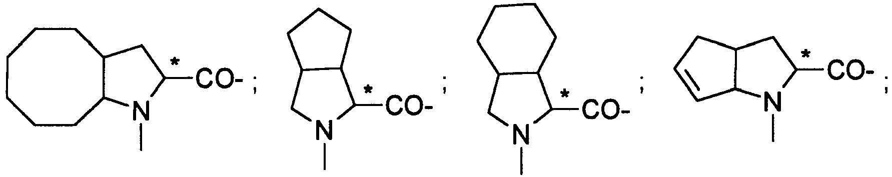

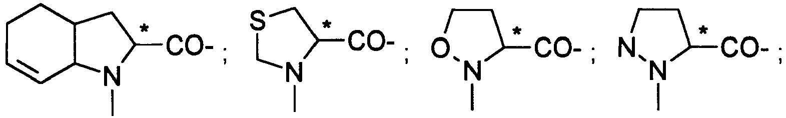

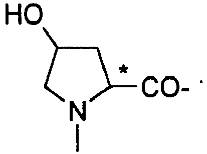

Die oben genannten Resten zugrundeliegenden Heterocyclen sind beispielsweise bekannt aus US-A-4,344,949; US-A 4,374,847;
US-A 4,350,704; EP-A 29,488; EP-A 31,741; EP-A 46,953; EP-A 49,605; EP-A 49,658; EP-A 50,800; EP-A 51,020; EP-A 52,870; EP-A 79,022; EP-A 84,164; EP-A 89,637; EP-A 90,341; EP-A 90,362; EP-A 105,102; EP-A 109,020; EP-A 111,873; EP-A 271,865 und EP-A 344,682.

Ferner können die Aminosäuren auch als Ester bzw. Amide vorliegen, wie z. B. Methylester, Ethylester, Isopropylester, Isobutylester, tert-Butylester, Benzylester, unsubstituiertes Amid, Ethylamid, Semicarbazid oder ω-Amino-(C₂-C₈)-alkylamid.

Funktionelle Gruppen der Aminosäuren können geschützt vorliegen. Geeignete Schutzgruppen wie z. B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, Z(NO₂), Z(Halₙ), Bobz, Iboc, Adpoc, Mboc, Acm, tert-Butyl, OBzl, ONbzl, OMbzl, Bzl, Mob, Pic, Trt.

Physiologisch verträgliche Salze der Verbindungen der Formel I sind insbesondere pharmazeutisch verwendbare oder nicht-toxische Salze. Solche Salze werden beispielsweise von Verbindungen der Formel I, welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin, Ethanolamin oder Tris-(2-hydroxy-ethyl)-amin. Verbindungen der Formel I, welche basische Gruppen, z. B. eine Aminogruppe, eine Amidinogruppe oder eine Guanidinogruppe enthalten, bilden mit anorganischen Säuren, wie z.B. Salzsäure, Schwefelsäure oder Phosphorsäure, und mit organischen Carbon- oder Sulfonsäuren, wie z. B. Essigsäure, Citronensäure, Benzoesäure, Maleinsäure, Fumarsäure, Weinsäure, Milchsäure, Methansulfonsäure oder p-Toluolsulfonsäure Salze.

Die erfindungsgemäßen Verbindungen der Formel I können optisch aktive Kohlenstoffatome, die unabhängig voneinander R- oder S-Konfigurationen haben können, enthalten und können somit in Form reiner Enantiomerer oder reiner Diastereomerer oder in Form von Enantiomerengemischen oder Diastereomerengemischen vorliegen. Sowohl reine Enantiomere und Enantiomerengemische in allen Verhältnissen als auch Diastereomere und Diastereomerengemische in allen Verhältnissen sind Gegenstand der vorliegenden Erfindung.

Die erfindungsgemäßen Verbindungen der Formel I können, unabhängig voneinander als E/Z-Isomerengemische vorliegen. Gegenstand der vorliegenden Erfindung sind sowohl reine E- bzw. Z-Isomere als auch E/Z-Isomerengemische. Diastereomere, einschließlich E/Z-Isomere können durch Chromatographie in die Einzelisomeren aufgetrennt werden. Racemate können entweder durch Chromatographie an chiralen Phasen oder durch Racematspaltung in die beiden Enantiomere aufgetrennt werden.

Die erfindungsgemäßen Verbindungen der Formel I können darüber hinaus bewegliche Wasserstoffatome enthalten, also in verschiedenen tautomeren Formen vorliegen. Auch alle diese Tautomeren sind Gegenstand der vorliegenden Erfindung.

Bevorzugt sind Verbindungen der Formel I, die selektive Vitronectinrezeptor-Antagonisten, insbesondere in Bezug zum Fibrinogenrezeptor, darstellen, d.h. die stärkere Inhibitoren des Vitronectinrezeptors als des Fibrinogenrezeptors sind.

Bevorzugt sind insbesondere Verbindungen der Formel I, die selektive Vitronectinrezeptor-Antagonisten darstellen und in denen der Abstand zwischen R¹⁰ und dem ersten N-Atom in A 12 bis 13 kovalente Bindungen entlang des kürzesten Weges zwischen diesen Atomen beträgt, wie nachfolgend exemplarisch gezeigt für
A= 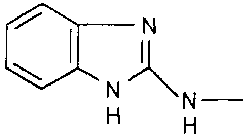
und R¹⁰=COOH dargestellt:

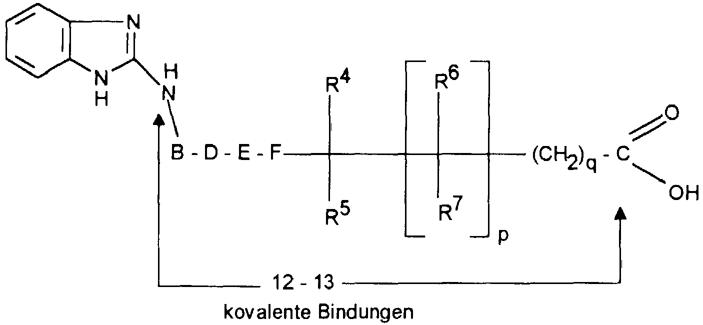

Bevorzugt sind ferner Verbindungen der Formel I, in denen mindestens ein Rest aus der Reihe R⁴, R⁵, R⁶, R⁷ einen lipophilen Rest darstellt.

Beispiele für lipophile Reste in der Reihe R⁴, R⁵, R⁶, R⁷ sind z.B. Neopentyl, Cyclohexyl, Adamantyl, Cyclohexyl-(C₁-C₈)-alkyl, Adamantyl-(C₁-C₈)-alkyl, Phenyl, Naphthyl, Phenyl-(C₁-C₈)-alkyl, Naphthyl-(C₁-C₈)-alkyl, Cyclohexylmethylcarbonylamino, 1-Adamantylmethyloxycarbonylamino oder Benzyloxycarbonylamino bzw. allgemein Reste, in denen R⁸ z.B. Neopentyl, Cyclohexyl, Adamantyl, Cyclohexyl-(C₁-C₈)-alkyl, Adamantyl-(C₁-C₈)-alkyl, Phenyl, Naphthyl, Phenyl-(C₁-C₈)-alkyl bedeutet.

Bevorzugt sind ferner Verbindungen der Formel I, in der bedeuten:
A den Rest 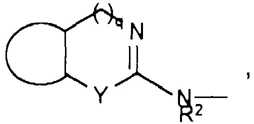 wobei 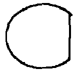
ein 5- bis 10-gliedriges mono- oder polycyclisches, aromatisches oder nicht aromatisches Ringsystem darstellt, das 1 bis 4 Heteroatome aus der Reihe N, O, S enthalten kann und gegebenenfalls ein- oder mehrfach mit R¹², R¹³, R¹⁴ und R¹⁵ substituiert sein kann;
B eine direkte Bindung, (C₁-C₆)-Alkandiyl, (C₅-C₈)-Arylen, (C₃-C₈)-Cycloalkylen, -C≡C-, -NR²-, -NR²-C(O)-, -NR²-C(O)-NR²-, -NR²-S(O)-, -NR²-S(O)₂-, -O-, -CR²=CR³-, die jeweils ein- oder zweifach durch (C₁-C₆)-Alkyl substituiert sein können;
D eine direkte Bindung, (C₁-C₈)-Alkandiyl, (C₅-C₈)-Arylen, -O-, NR²-, -CO-NR²-, -NR²-CO-, -NR²-C(O)-NR²-,-OC(O)-, -C(O)O-, S(O)₂-, -S(O)₂-NR²-, -NR²-S(O)₂-, -S-, -CR²=CR³-, -C≡C-, die jeweils ein- oder zweifach durch (C₁-C₈)-Alkyl, -CR²=CR³-, (C₅-C₆)-Aryl substituiert sein können, wobei, wenn B eine direkte Bindung ist, D nicht gleich -CO-NR²-, -C(O)O, -SO₂-, -S(O)₂-NR²- sein kann;
E ein Templat aus der Reihe der Fibrinogenrezeptorantagonisten, entnommen aus:
   US 5 250 679, Okt. 5, 1993, Blackburn, B.K. et al..
   US 5 403 836, Apr. 4, 1995, Blackburn, B.K. et al..
   US 5 565 449, Okt. 15, 1996, Blackburn, B.K. et al..
   WO 93/08174, Okt. 15, 1991, Blackburn, B.K. et al..
   WO 95/04057, Feb. 9, 1995, Blackburn, B.K. et al..
   EP 0 655 439, Nov. 9, 1994, Denney, M.L. et al..
   WO 94/18981, Sep. 1, 1994, Claremon, D.A. et al..
   WO 94/08962, Apr. 28, 1994, Harmann, G.D. et al..
   EP 0 668 278, Feb. 14, 1995, Juraszyk, H. et al..
   WO 94/12478, June 9, 1994, Keenan, E.Mc. C. et al..
F ist wie D definiert;
   - G: 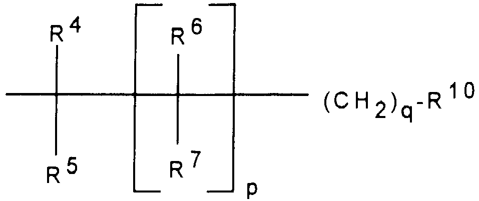
   - R², R³: unabhängig voneinander H, (C₁-C₁₀)-Alkyl, das gegebenenfalls durch Fluor ein- oder mehrfach substituiert ist, (C₃-C₈)-Cycloalkyl, (C₃-C₈)-Cycloalkyl-(C₁-C₆)-alkyl, (C₅-C₁₂)-Aryl, (C₅-C₁₂)-Aryl-(C₁-C₆)-alkyl, R⁸OC(O)R⁹, R⁸R⁸NC(O)R⁹, R⁸C(O)R⁹;
   - R⁴, R⁵, R⁶, R⁷: unabhängig voneinander H, Fluor, OH, (C₁-C₈)-Alkyl, (C₅-C₁₄)-Cycloalkyl, (C₅-C₁₄)-Cycloalkyl-(C₁-C₈)-alkyl, oder R⁸OR⁹, R⁸SR⁹, R⁸CO₂R⁹, R⁸OC(O)R⁹, R⁸-(C₅-C₁₄)-Aryl-R⁹, R⁸N(R²)R⁹, R⁸R⁸NR⁹, R⁸N(R²)C(O)OR⁹, R⁸S(O)ₙN(R²)R⁹, R⁸OC(O)N(R²)R⁹, R⁸C(O)N(R²)R⁹, R⁸N(R²)C(O)N(R²)R⁹, R⁸N(R²)S(O)ₙN(R²)R⁹, R⁸S(O)ₙR⁹, R⁸SC(O)N(R²)R⁹, R⁸C(O)R⁹, R⁸N(R²)C(O)R⁹, R⁸N(R²)S(O)ₙR⁹;
   - R⁸: H, (C₁-C₆)-Alkyl, (C₅-C₁₄)-Cycloalkyl, (C₅-C₁₄)-Cycloalkyl-(C₁-C₆)-alkyl, (C₅-C₁₂)-Aryl, (C₅-C₁₂)-Aryl-(C₁-C₆)-alkyl, wobei die Alkylreste durch Fluor ein- oder mehrfach substituiert sein können;
   - R⁹: eine direkte Bindung oder (C₁-C₆)-Alkandiyl;
   - R¹⁰: C(O)R¹¹, C(S)R¹¹, S(O)ₙR¹¹, P(O)(R¹¹)ₙ oder ein vier- bis achtgliedriger, gesättigter oder ungesättigter Heterocyclus, der 1, 2, 3 oder 4 Heteroatome aus der Reihe N, O, S enthält;
   - R¹¹: OH, (C₁-C₆)-Alkoxy, (C₅-C₁₂)-Aryl-(C₁-C₆)-alkoxy, (C₅-C₁₂)-Aryloxy, (C₁-C₆)-Alkylcarbonyloxy-(C₁-C₄)-alkoxy, (C₅-C₁₂)-Aryl-(C₁-C₆)-alkylcarbonyloxy-(C₁-C₆)-alkoxy, NH₂, Mono- oder Di-((C₁-C₆)-alkyl)-amino, (C₅-C₁₂)-Aryl-(C₁-C₆)-alkylamino, (C₁-C₆)-Dialkylaminocarbonylmethyloxy;
   - R¹², R¹³, R¹⁴, R¹⁵: unabhängig voneinander H, (C₁-C₈)-Alkyl, das gegebenenfalls durch Fluor ein- oder mehrfach substituiert ist, (C₃-C₈)-Cycloalkyl, (C₃-C₈)-Cycloalkyl-(C₁-C₆)-alkyl, (C₅-C₁₂)-Aryl, (C₅-C₁₂)-Aryl-(C₁-C₆)-alkyl, H₂N, R⁸ONR⁹, R⁸OR⁹, R⁸OC(O)R⁹, R⁸-(C₅-C₁₂)-Aryl-R⁹, R⁸R⁸NR⁹, HO-(C₁-C₈)-Alkyl-N(R²)R⁹, R⁸N(R²)C(O)R⁹, R⁸C(O)N(R²)R⁹, R⁸C(O)R⁹, R²R³N-C(=NR²), R²R³N-C(=NR²)-NR², =O, =S;
   wobei zwei benachbarte Substituenten aus R¹² bis R¹⁵ zusammen ferner -OCH₂O-, OCH₂CH₂O-, -OC(CH₃)₂O- bedeuten können;
   - Y: NR², O, S;
   - n: 1 oder 2;
   - p, q: unabhängig voneinander 0 oder 1;
in allen ihren stereoisomeren Formen und Mischungen davon in allen Verhältnissen, und ihre physiologisch verträglichen Salze.

Besonders bevorzugt sind Verbindungen der Formel I, in der bedeuten:
A einen der Reste 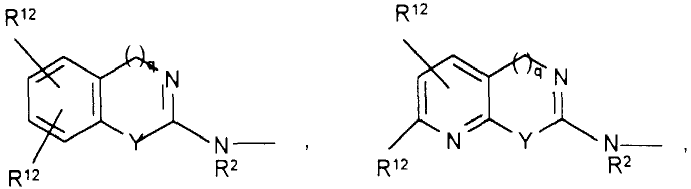 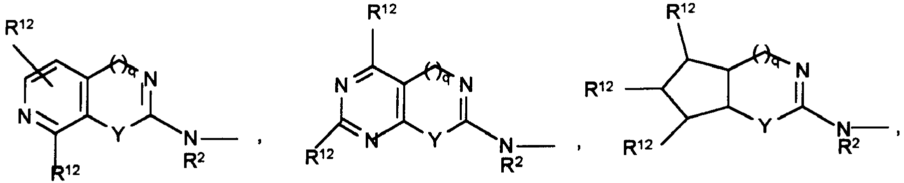 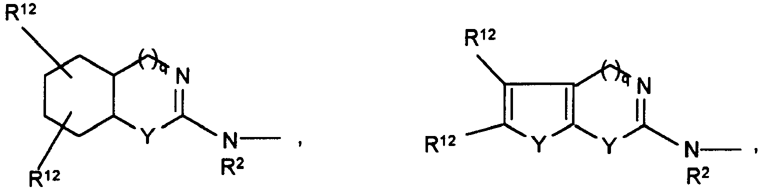 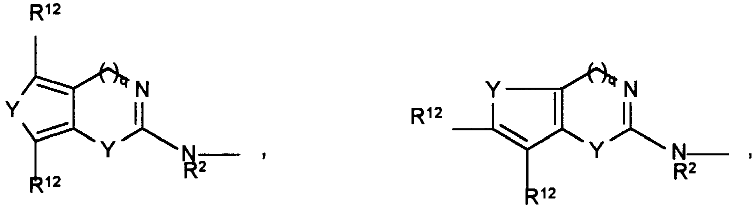 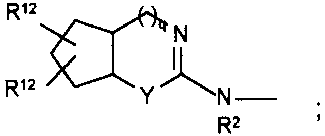
B eine direkte Bindung, (C₁-C₆)-Alkandiyl, (C₅-C₆)-Arylen, (C₅-C₆)-Cycloalkylen, -C≡C-, -NR²-, -NR²-C(O)-, -NR²-S(O)₂-, -O-, -CR²=CR³-, die jeweils ein- oder zweifach durch (C₁-C₆)-Alkyl substituiert sein können;
D eine direkte Bindung, (C₁-C₆)-Alkandiyl, (C₅-C₆)-Arylen, -O-, -NR²-, -NR²-C(O)-, -C(O)NR²-, -NR²-C(O)-NR²-,-OC(O)-, -S(O)₂-NR²-, -NR²-S(O)₂-, -CR²=CR³-, die jeweils ein- oder zweifach durch (C₁-C₆)-Alkyl substituiert sein können, wobei, wenn B eine direkte Bindung ist, D nicht gleich -C(O)NR²-, -S(O)₂-NR²- sein kann;
E
   a) ein Templat aus WO 93/08174, US 5 250 679, US 5 403 836, US 5,565,449, und zwar: 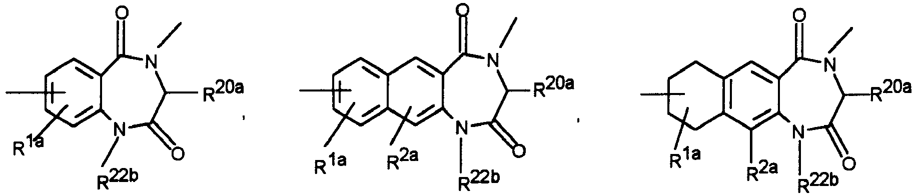 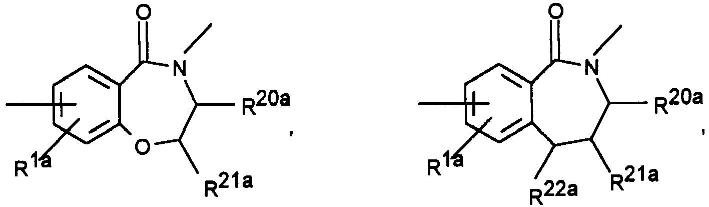 wobei R^{1a}, R^{2a}, R^{20a}, R^{21a}, R^{22a} wie R¹, R², R²⁰, R²¹, R²² in US 5,403,836, Spalte 249, Zeile 9-22; Spalte 252, Zeile 66 bis Spalte 253, Zeile 68 definiert sind und somit bedeuten:
      - R^{1a}, R^{2a},: unabhängig voneinander eine bis drei Gruppen aus der Reihe Wasserstoff, Halogen, Cyano, Carboxamido, Carbamoyloxy, Formyloxy, Formyl, Azido, Nitro, Ureido, Thioureido, Hydroxy, Mercapto, Sulfonamido oder einen gegebenenfalls substituierten Rest aus der Reihe C₁-C₁₂-Alkyl, C₂-C₁₂-Alkenyl, C₃-C₁₂-Alkinyl, C₃-C₁₂-Cycloalkyl, C₆-C₁₄-Aryl, C₆-C₁₀-Aryl-C₁-C₈-alkyl, C₁-C₂-AlkyloxyC₆-C₁₄-Aryloxy und C₁-C₁₂-Acylamino, wobei die Substituenten einen Rest aus der Reihe Halogen, Cyano, Azido, Nitro, Hydroxy, Mercapto, Sulfonamido, Ureido, Thioureido, Carboxamido, Carbamoyloxy, Formyloxy, Formyl, C₁-C₄-Alkoxy, Phenyl und Phenoxy bedeuten;
      - R^{20a}: Wasserstoff, Halogen (Fluor, Chlor, Brom, Iod), C₁-C₄-Alkoxy, C₁-C₄-Alkyl, Phenyl, Benzyl oder Halogen-C₁-C₄-Alkyl bedeutet,
      - R^{21a}, R^{22a},: unabhängig voneinander
      1. Wasserstoff
      2. (C₁-C₁₂)-Alkyl
      3. (C₆-C₁₄)-Aryl,
      4. (C₃-C₁₄)-Cycloalkyl,
      5. (C₁-C₁₂)-Alkyl-(C₆-C₁₄)-aryl,
      6. (C₁-C₁₂)-Alkyl-(C₃-C₁₄)-cycloalkyl bedeutet, wobei die unter 2. bis 6. definierten Reste substituiert sein können mit einem oder mehreren Resten aus der Reihe Halogen (Fluor, Chlor, Brom, Iod); Nitro; Hydroxyl; Carboxyl; Tetrazol; Hydroxamat; Sulfonamid; Trifluorimid; Phosphonat; C₁-C₆-Alkyl; C₆-C₁₄-Aryl; Benzyl; C₃ -C₁₄-Cycloalkyl; COR^{24a}; CONR²⁵R²⁶; wobei
         R^{24a} einen Rest aus der Reihe C₁-C₈-Alkoxy; C₃-C₁₂-Alkenoxy; C₆-C₁₂-Aryloxy; di-C₁-C₈-Alkylamino-C₁-C₈-alkoxy; Acylamino-C₁-C₈-Alkoxy wie beispielsweise Acetylaminoethoxy, Nicotinoylaminoethoxy, Succinamidethoxy oder Pivaloylethoxy; C₆-C₁₂-Aryl-C₁-C₈-alkoxy, wobei die Arylgruppe gegebenenfalls mit ein bis drei Resten aus der Reihe Nitro, Halogen, C₁-C₄-Alkoxy, Amino, Hydroxyl, Hydroxy-C₂-C₈-Alkoxy, Dihydroxy-C₃-C₈-Alkoxy substituiert ein kann;
         R²⁵ und R²⁶ unabhängig voneinander Wasserstoff, C₁-C₁₀-Alkyl, C₃-C₁₀-Alkenyl, C₆-C₁₄-Aryl, C₁-C₆-Alkyl-C₆-C₁₀-aryl bedeuten oder
         R²⁵ und R²⁶ zusammen einen Trimethylen-, Tetramethylen-, Pentamethylen- oder 3-Oxopentamethylenrest bilden;
      7. Q²-L³ bedeutet, wobei
         Q² Wasserstoff oder Q¹ ist; und
         L³ eine chemische Bindung, L¹ oder L² bedeutet;
            Q¹ ein substituierter oder unsubstituierter, positiv geladener, Stickstoff-enthaltender Rest ist,
            L¹ ein zweiwertiger Rest ist, der 3 bis 9 Methylengruppen enthält, wobei eine bis alle Methylengruppen durch eine oder mehrere Alkengruppen, Alkingruppen, Arylgruppen oder funktionelle Gruppen enthaltend Heteroatome aus der Reihe N, O oder S, ersetzt sein können, und
            L² ein gegebenenfalls substituierter, zweiwertiger Rest ist;
      wobei bevorzugte Reste für Q¹, L¹ und L² solche Reste sind, wie sie in US 5,403,836 in Spalte 249, Zeile 27 bis Spalte 251, Zeile 6 (Q¹), Spalte 251, Zeile 7 bisSpalte 252, Zeile 18 (L¹) und Spalte 252, Zeile 19-45 (L²) beschrieben sind;
      - und R^{22b} wie R²²: in US 5,565,449, Spalte 296, Zeile 38 bis Spalte 297, Zeile 38 definiert ist und bedeutet:
      1. Wasserstoff
      2. (C₁-C₁₂)-Alkyl
      3. (C₆-C₁₄)-Aryl,
      4. (C₃-C₁₄)-Cycloalkyl,
      5. (C₁-C₁₂)-Alkyl-(C₆-C₁₄)-aryl,
      6. (C₁-C₁₂)-Alkyl-(C₃-C₁₄)-cycloalkyl bedeutet, wobei die unter 2. bis 6. definierten Reste substituiert sein können mit einem oder mehreren Resten aus der Reihe Halogen (Fluor, Chlor, Brom, Iod); Nitro; Hydroxyl; Carboxyl; Tetrazol; Hydroxamat; Sulfonamid; Trifluorimid; Phosphonat; C₁-C₆-Alkyl; C₆-C₁₄-Aryl; Benzyl; C₃ -C₁₄-Cycloalkyl; COR^{24a}; CONR²⁵R²⁶; wobei
         R^{24a} einen Rest aus der Reihe C₁-C₈-Alkoxy; C₃-C₁₂-Alkenoxy; C₆-C₁₂-Aryloxy; di-C₁-C₈-Alkylamino-C₁-C₈-alkoxy; Acylamino-C₁-C₈-alkoxy wie beispielsweise Acetylaminoethoxy, Nicotinoylaminoethoxy, Succinamidethoxy oder Pivaloylethoxy; C₆-C₁₂-Aryl-C₁-C₈-alkoxy, wobei die Arylgruppe gegebenenfalls mit ein bis drei Resten aus der Reihe Nitro, Halogen, C₁-C₄-Alkoxy, Amino, Hydroxyl, Hydroxy-C₂-C₈-Alkoxy, Dihydroxy-C₃-C₈-Alkoxy substituiert sein kann;
         R²⁵ und R²⁶ unabhängig voneinander Wasserstoff, C₁-C₁₀-Alkyl, C₃-C₁₀-Alkenyl, C₆-C₁₄-Aryl, C₁-C₆-Alkyl-C₆-C₁₀-aryl bedeuten oder
         R²⁵ und R²⁶ zusammen einen Trimethylen-, Tetramethylen-, Pentamethylen- oder 3-Oxopentamethylenrest bilden;
      7. Q²-L³ bedeutet, wobei
         Q² Wasserstoff oder Q¹ ist; und
         L³ eine chemische Bindung, L¹ oder L² bedeutet;
            Q¹ ein substituierter oder unsubstituierter, positiv geladener, Stickstoff-enthaltender Rest ist,
            L¹ ein zweiwertiger Rest ist, der 3 bis 9 Methylengruppen enthält, wobei eine bis alle Methylengruppen durch eine oder mehrere Alkenreste, Alkinreste, Arylreste oder funktionelle Gruppen enthaltend Heteroatome aus der Reihe N, O oder S ersetzt sein können, und
            L² ein gegebenenfalls substituierter, zweiwertiger Rest ist;
      wobei bevorzugte Reste für Q¹, L¹ und L² solche Reste sind, wie sie in US 5,403,836 in Spalte 289, Zeile 9 bis Spalte 293, Zeile 17 (Q¹), Spalte 293, Zeile 18 bis Spalte 295, Zeile 28 (L¹) und Spalte 295, Zeile 29 bis Spalte 296, Zeile 11 (L²) beschrieben sind;
   oder b) ein Templat aus WO 95/04057, und zwar: 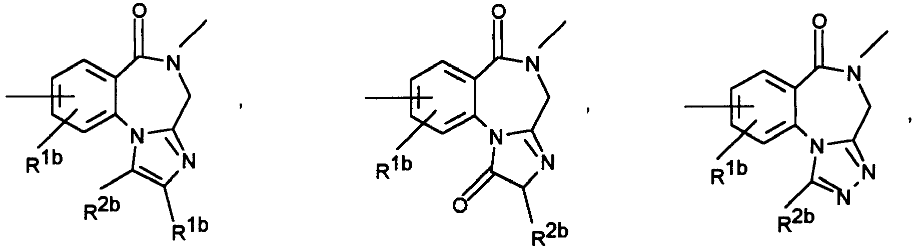 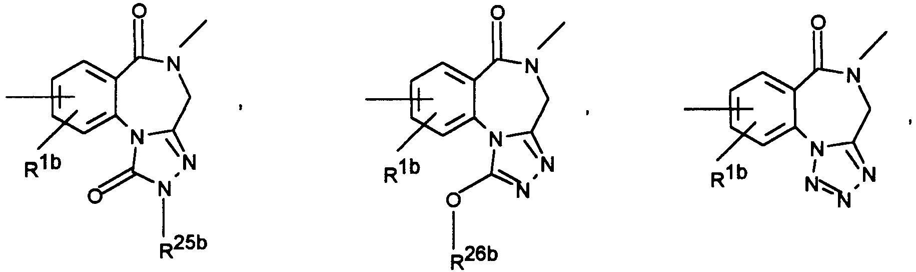 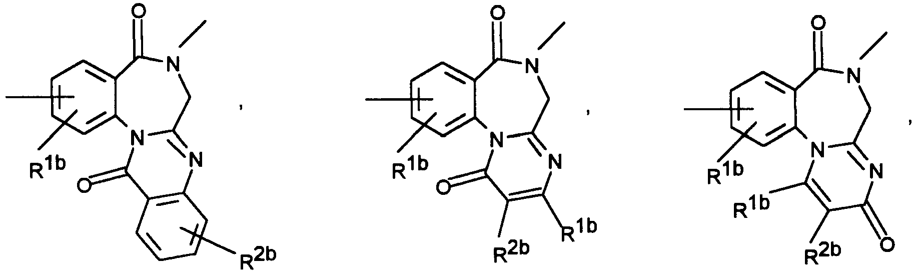 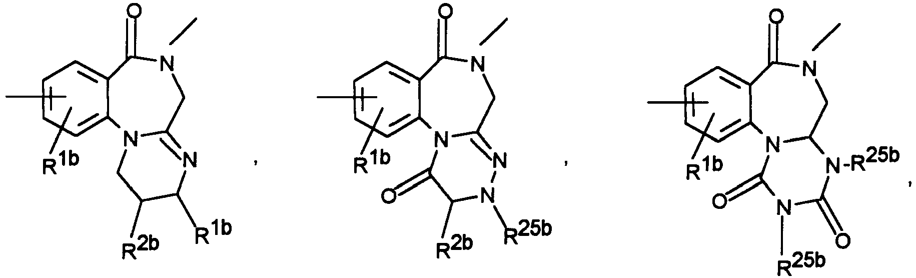 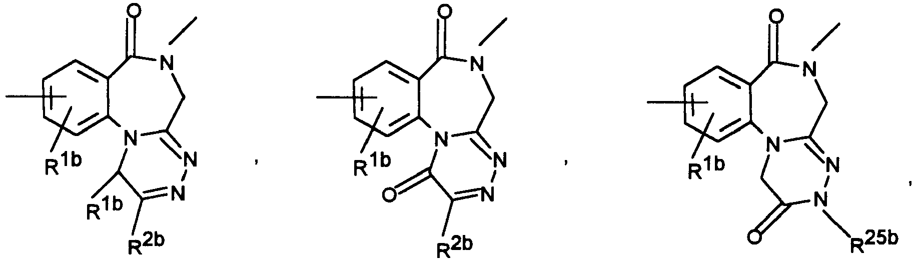 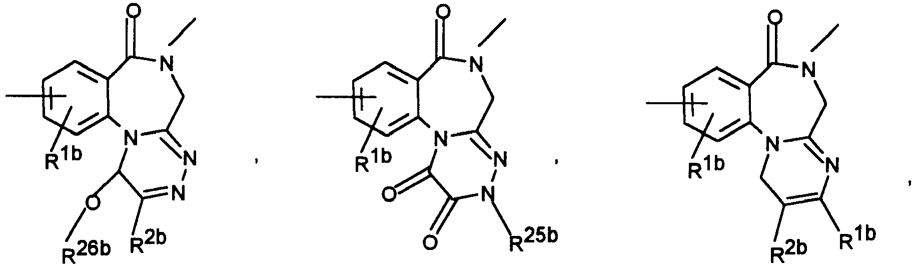 wobei R^{1b} und R^{2b} wie R¹ und R² in US 5,403,836, Spalte 249, Zeile 9 - 22; definiert sind und bedeuten:
      - R^{1b}, R^{2b},: unabhängig voneinander eins bis drei Gruppen aus der Reihe Wasserstoff, Halogen, Cyano, Carboxamido, Carbamoyloxy, Formyloxy, Formyl, Azido, Nitro, Ureido, Thioureido, Hydroxy, Mercapto, Sulfonamido oder ein gegebenenfalls substituierter Rest aus der Reihe C₁-C₁₂-Alkyl, C₂-C₁₂-Alkenyl, C₃-C₁₂-Alkinyl, C₃-C₁₂-Cycloalkyl, C₆-C₁₄-Aryl, C₆-C₁₀-Aryl-C₁-C₈-alkyl, C₁-C₁₂-Alkyloxy,C₆-C₁₄-Aryloxy und C₁-C₁₂-Acylamino, wobei die Substituenten einen Rest aus der Reihe Halogen, Cyano, Azido, Nitro, Hydroxy, Mercapto, Sulfonamido, Ureido, Thioureido, Carboxamido, Carbamoyloxy, Formyloxy, Formyl, C₁-C₄-Alkoxy, Phenyl und Phenoxy darstellen; und
      - R^{25b} und R^{26b} wie R²⁵ und R²⁶: in US 5,565,449 definiert sind und bedeuten:
      - R^{25b} und R^{26b}: unabhängig voneinander
      Wasserstoff, C₁-C₁₀-Alkyl, C₃-C₁₀-Alkenyl, C₆-C₁₄-Aryl, C₁-C₆-Alkyl-C₆-C₁₀-aryl bedeuten oder
      - R^{25b} und R^{26b}: zusammen einen Trimethylen-, Tetramethylen-, Pentamethylen- oder 3-Oxopentamethylenrest bilden;
   oder c)
      ein Templat aus EP-A 0 655 439, und zwar 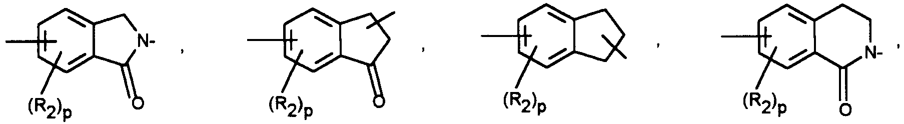 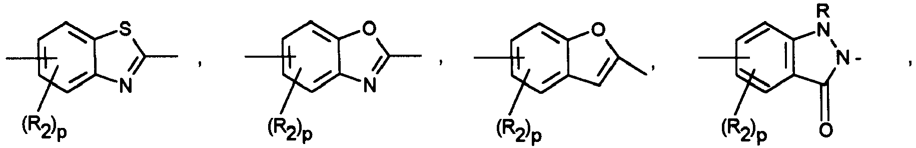 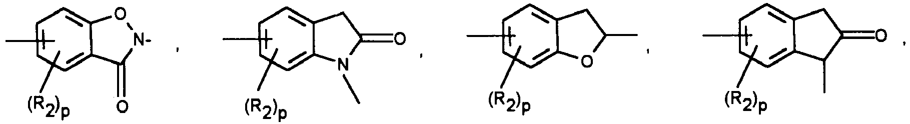 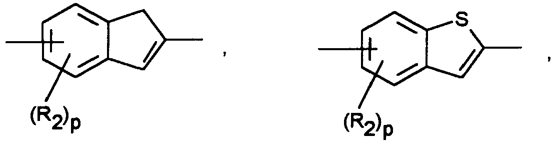 bedeutet,
      wobei
      - (R₂)ₚ: an ein oder mehrere C-Atomen des 6-gliedrigen Rings gebunden ist und unabhängig voneinander einen Rest aus der Reihe H, Alkyl, halogensubstituiertes Alkyl, Hydroxyalkyl, Alkenyl, Alkinyl, Cycloalkyl, Aryl, Aryloxy, Aralkyl, Hydroxy, Alkoxy, Aralkoxy, Carbamyl, Amino, substituiertes Amino, Acyl, Cyano, Halogen, Nitro und Sulfo bedeutet;
      - R: (C₁-C₄)-Alkyl bedeutet
      - p: eine ganze Zahl von 1 bis 3 ist
   oder d)
      ein Templat aus WO 94/12478, und zwar 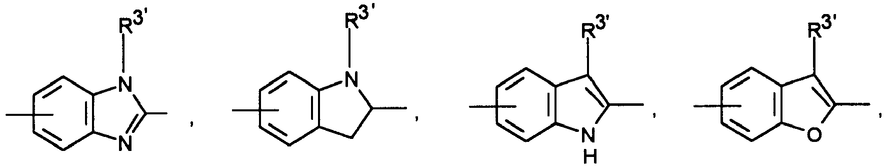 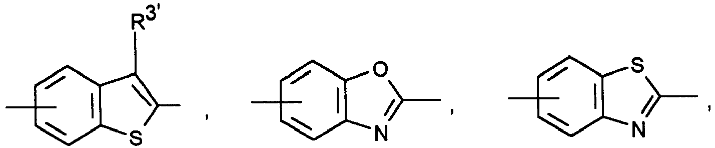 bedeutet
      wobei R^{3'} Wasserstoff, (C₁-C₆)-Alkyl, Aryl-C₁-C₆-alkyl ist,
   oder e) ein Templat aus WO94/18981, und zwar 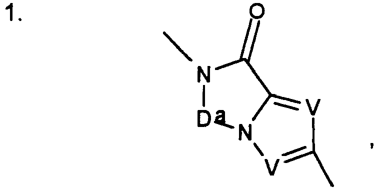
      - worin V: CR^{7a} oder N ist, und
      - D^{a}: CH₂, CH₂-CH₂, CH₂C(R^{7a})₂CH₂ oder 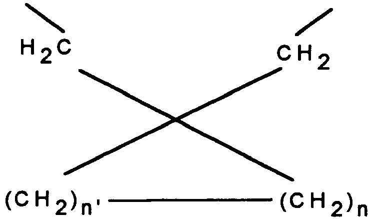
      bedeutet; 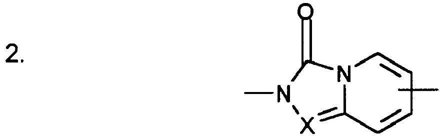
      - worin X: CR^{3a} oder N ist,
      - wobei R^{3a}: CN, C(O)N(R^{7a})R^{8a}, 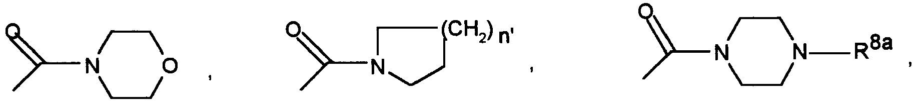 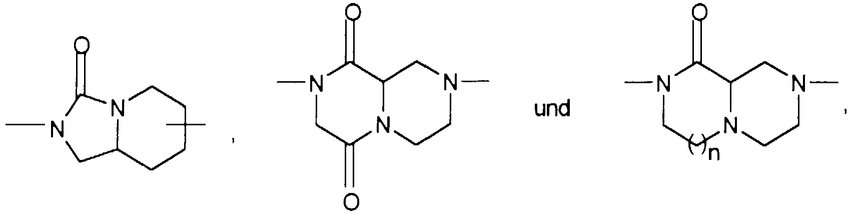
      bedeutet; 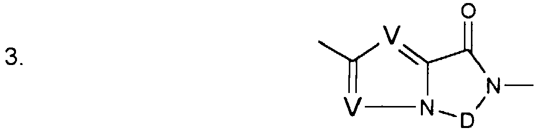
      - worin V: CR^{7a} oder N bedeutet, und
      - D^{a}: CH₂, CH₂-CH₂, CH₂C(R^{7a})₂CH₂ oder 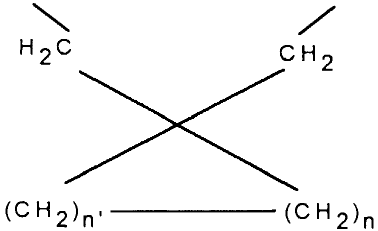
      bedeutet; 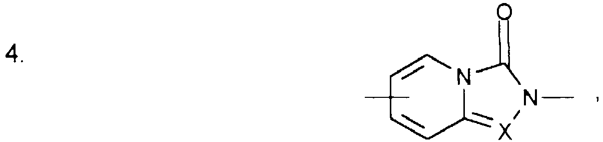
      - worin X: CR^{3a} oder N bedeutet, worin
      - R^{3a}: CN, C(O)N(R^{7a})R^{8a}, 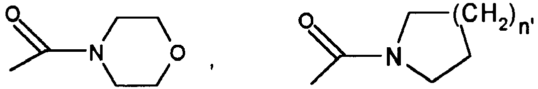
      bedeutet; 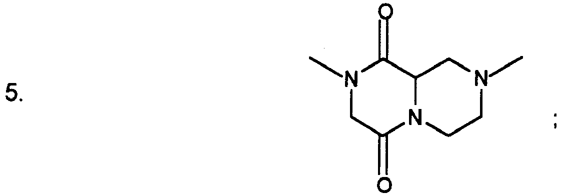 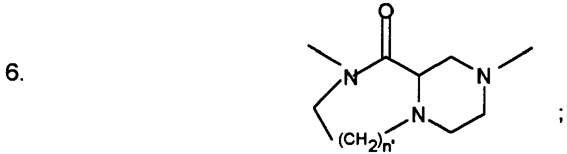 und 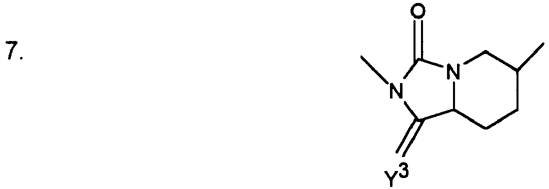
      - wobei Y³: O oder H₂ ist und
      - R^{7a}: Wasserstoff; C₁-C₄-Alkyl, gegebenenfalls substituiert mit OH oder (C₁-C₄)-Alkoxy; C₂-C₆-Alkenyl, gegebenenfalls substituiert mit (C₁-C₄)-Alkoxy; oder OH (C₁-C₄)-Alkylaryl; Aryl gegebenenfalls substituiert mit gleichen oder verschiedenen Resten aus der Reihe Halogen, (C₁-C₄)-Alkoxy, Hydroxy oder (C₁-C₄)-Alkyl, bedeutet
      - R^{8a}: Wasserstoff oder C₁-C₄-Alkyl bedeutet
      - n: eine ganze Zahl von 0 bis 7 und
      - n': eine ganze Zahl von 0 bis 3 ist;
   oder f) ein Templat aus EP-A 0531 883, und zwar 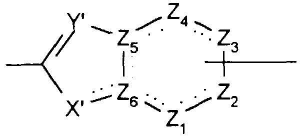 bedeutet,
      wobei bedeuten:
      - X': ein Sauerstoff-, Schwefel- oder Stickstoffatom oder eine -NR^{2b}-Gruppe, wobei
      - R^{2b}: ein Wasserstoffatom, eine geradkettige oder verzweigte Alkylgruppe mit 1 bis 15 C-Atomen, eine geradkettige oder verzweigte Alkenyl- oder Alkinylgruppe mit jeweils 3 bis 10 Kohlenstoffatomen, wobei die Doppel- oder Dreifachbindung nicht unmittelbar an das Stickstoffatom anschließen kann, eine Cycloalkyl- oder Cycloalkylalkylgruppe mit jeweils 3 bis 7 Kohlenstoffatomen im Cycloalkylteil, eine Arylgruppe, eine Alkylgruppe mit 2 bis 6 Kohlenstoffatomen, die ab der β-Position zu dem Stickstoffatom der -NR^{2b}-Gruppe durch eine R^{3b}O-, (R^{3b})₂N-, R^{4b}CO-NR^{3b}-, Alkylsulfonyl-NR^{3b} -, Arylsulfonyl-NR^{3b}-, Alkylsulfenyl-, Alkylsuffinyl-, Alkylsulfonyl- oder R^{5b}-Gruppe substituiert ist, oder eine Alkylgruppe mit 1 bis 6 Kohlenstoffatomen, die durch eine oder zwei Arylgruppen, R^{6b}OCO-, (R^{3b})₂NCO-, R^{5b}-CO-, R^{3b}O-CO-alkylen-NR₃-CO-, (R^{3b})₂N-CO-alkylen-NR^{3b}-CO- oder R^{5b}CO-alkylen-NR^{3b}-CO-Gruppe substituiert ist, in denen R^{3b} und R^{5b} wie nachstehend definiert sind und R^{6b} ein Wasserstoffatom, eine Alkylgruppe mit 1 bis 6 Kohlenstoffatomen, eine Cycloalkylgruppe mit 5 bis 7 Kohlenstoffatomen oder eine Aralkylgruppe darstellt,
      - Y': eine NO-Gruppe, ein Stickstoffatom oder eine gegebenenfalls durch eine Alkylgruppe substituierte Methingruppe,
      - Z₁, Z₂, Z₃ und Z₄,: die gleich oder verschieden sein können, Methingruppen, Kohlenstoffatome, Iminogruppen oder Stickstoffatome, wobei mindestens einer der Reste Z₁ bis Z₄ ein Kohlenstoffatom enthalten muß und eine oder zwei zu einem Stickstoffatom benachbarte Methingruppen jeweils durch Carbonylgruppen ersetzt sein können,
      - Z₅ und Z₆: jeweils ein Kohlenstoffatom oder auch einer der Reste Z₅ oder Z₆ ein Stickstoffatom und der andere der Reste Z₅ oder Z₆ ein Kohlenstoffatom,
      - R^{3b}: ein Wasserstoffatom, eine Alkylgruppe mit 1 bis 6 Kohlenstoffatomen, eine Aryl-, Aralkyl-, Carboxyalkyl- oder Alkoxycarbonylalkylgruppe,
      - R^{4b}: ein Wasserstoffatom, eine Alkyl- oder Alkoxygruppe mit jeweils 1 bis 6 Kohlenstoffatomen, eine Aryl- oder Aralkylgruppe mit 1 bis 6 Kohlenstoffatomen im Alkylteil und
      - R^{5b}: eine Azetidino-, Pyrrolidino-, Hexamethylenimino- oder Heptamethyleniminogruppe oder eine Piperidinogruppe, in der die Methylengruppe in 4-Stellung durch ein Sauerstoffatom, eine Sulfenyl-, Sulfinyl- oder Sulfonylgruppe oder durch eine durch eine R₃-, R₄CO-, Alkylsulfonyl- oder Arylsulfonylgruppe substituierte Iminogruppe ersetzt sein kann, wobei R₃ und R₄ wie vorstehend erwähnt, definiert sind, darstellen;
F eine direkte Bindung, (C₁-C₆)-Alkandiyl, -O-, -CO-NR²-, -NR²-CO-, -NR²-C(O)-NR²-, -OC(O)-, -C(O)O-, -CO-, - S(O)₂-, -S(O)₂-NR²-, -NR²-S(O)₂-, -CR²=CR³-, -C≡C-, die jeweils ein- oder zweifach durch (C₁-C₆)-Alkyl substituiert sein können;
   - G: 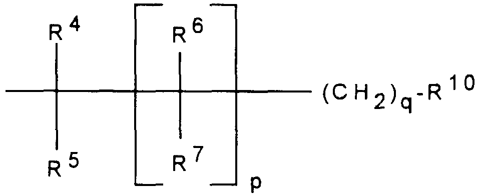
   - R², R³: unabhängig voneinander H, (C₁-C₆)-Alkyl, das gegebenenfalls durch Fluor ein- oder mehrfach substituiert ist, (C₅-C₆)-Cycloalkyl, (C₅-C₆)-Cycloalkyl-(C₁-C₄)-alkyl, (C₅-C₁₀)-Aryl, (C₅-C₁₀)-Aryl-(C₁-C₄)-alkyl, R⁸OC(O)R⁹, R⁸R⁸NC(O)R⁹, R⁸C(O)R⁹;
   - R⁴, R⁵, R⁶, R⁷: unabhängig voneinander H, Fluor, OH, (C₁-C₆)-Alkyl, (C₅-C₁₄)-Cycloalkyl, (C₅-C₁₄)-Cycloalkyl-(C₁-C₆)-alkyl, oder R⁸OR⁹, R⁸CO₂R⁹, R⁸OC(O)R⁹, R⁸-(C₅-C₁₀)-Aryl-R⁹, R⁸NHR⁹, R⁸R⁸NR⁹, R⁸NHC(O)OR⁹, R⁸S(O)ₙNHR⁹, R⁸OC(O)NHR⁹, R⁸C(O)NHR⁹, R⁸C(O)R⁹, R⁸NHC(O)NHR⁹, R⁸NHS(O)ₙNHR⁹, R⁸NHC(O)R⁹, R⁸NHS(O)ₙR⁹, wobei mindestens ein Rest aus der Reihe R⁴, R⁵, R⁶, R⁷ einen lipophilen Rest wie z. B. Benzyloxycarbonylamino, Cyclohexylmethylcarbonylamino etc. bedeutet;
   - R⁸: H, (C₁-C₆)-Alkyl, (C₅-C₁₄)-Cycloalkyl, (C₅-C₁₄)-Cycloalkyl-(C₁-C₄)-alkyl, (C₅-C₁₀)-Aryl, (C₅-C₁₀)-Aryl-(C₁-C₄)-alkyl, wobei die Alkylreste mit 1 bis 6 Fluor-Atomen substituiert sein können;
   - R⁹: eine direkte Bindung oder (C₁-C₆)-Alkandiyl;
   - R¹⁰: C(O)R¹¹;
   - R¹¹: OH, (C₁-C₆)-Alkoxy, (C₅-C₁₀)-Aryl-(C₁-C₆)-alkoxy, (C₅-C₁₀)-Aryloxy, (C₁-C₆)-Alkylcarbonyloxy-(C₁-C₄)-alkoxy, (C₅-C₁₀)-Aryl-(C₁-C₄)-alkylcarbonyloxy-(C₁-C₄)-alkoxy, NH₂, Mono- oder Di-((C₁-C₆)-alkyl)-amino;
   - R¹²: H, (C₁-C₆)-Alkyl, das gegebenenfalls durch Fluor ein- oder mehrfach substituiert ist, (C₃-C₆)-Cycloalkyl, (C₃-C₆)-Cycloalkyl-(C₁-C₄)-alkyl, (C₅-C₁₀)-Aryl, (C₅-C₁₀)-Aryl-(C₁-C₄)-alkyl, H₂N, R⁸OR⁹, R⁸OC(O)R⁹, R⁸-(C₅-C₁₀)-Aryl-R⁹, R⁸R⁸NR⁹, R⁸NHC(O)R⁹, R⁸C(O)NHR⁹, H₂N-C(=NH)-, H₂N-C(=NH)-NH-, =O,
   wobei zwei benachbarte Substituenten R¹² zusammen ferner -OCH₂O-, -OCH₂CH₂O- bedeuten können;
   - Y: NR², O, S;
   - n: 1 oder 2; und
   - p, q: unabhängig voneinander 0 oder 1;
   in allen ihren stereoisomeren Formen und Mischungen davon in allen Verhältnissen, und ihre physiologisch verträglichen Salze.

Ganz besonders bevorzugt sind Verbindungen der Formel I, in der bedeuten:
A einen der Reste 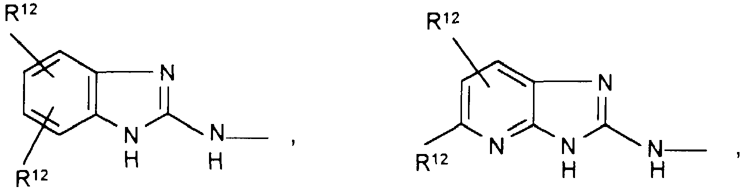 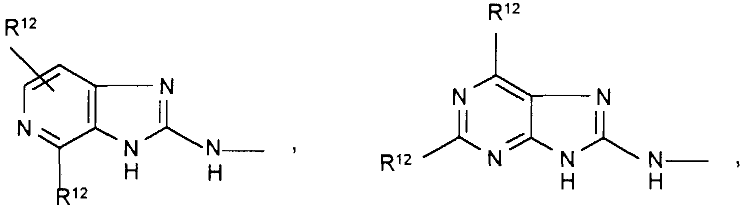 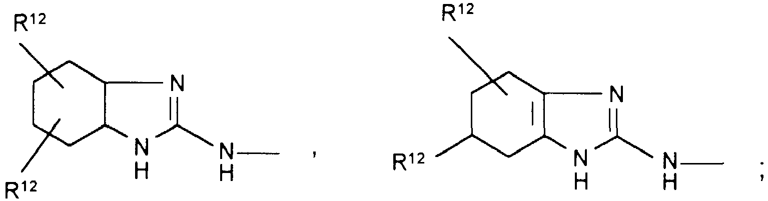
B eine direkte Bindung, (C₁-C₄)-Alkandiyl, Phenylen, Pyridindiyl, Thiophendiyl, Furandiyl, Cyclohexylen, Cyclopen-tylen, -C≡C-, -CR²=CR³-, die jeweils ein- oder zweifach durch (C₁-C₄)-Alkyl substituiert sein können;
D eine direkte Bindung, (C₁-C₄)-Alkandiyl oder Phenylen, -O-, -NR₂-, -NR²-C(O)-, -C(O)-NR²-, -NR²-S(O)₂, -NR²-C(O)-NR²-, -CR²=CR³-, die jeweils ein- oder zweifach durch (C₁-C₄)-Alkyl substituiert sein können wobei, wenn B eine direkte Bindung ist, D nicht gleich -C(O)-NR²- sein kann;
E
   a) ein Templat aus WO 93/08174, US 5 250 679, US 5 403 836, US 5,565,449, und zwar: 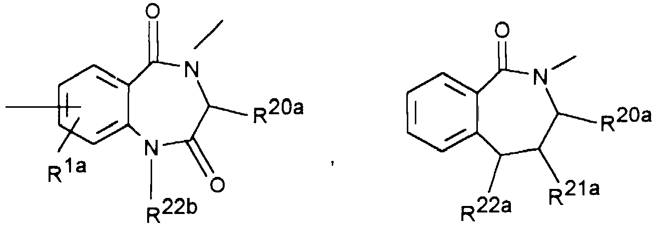 wobei hier R^{1a}, R^{20a}, R^{21a}, R^{22a} und R^{22b} bedeuten:
      - R^{1a}: unabhängig voneinander eins bis drei Gruppen aus der Reihe Wasserstoff und Halogen (Fluor, Chlor, Brom, Iod);
      - R^{20a}: Wasserstoff;
      - R^{21a}, R^{22a},: unabhängig voneinander
      1. Wasserstoff
      2. (C₁-C₆)-Alkyl
      3. (C₆-C₁₂)-Aryl
      4. (C₆-C₁₂)-Cycloalkyl
      5. (C₁-C₆)- Alkyl-(C₆-C₁₂)-aryl
      6. (C₁-C₆)-Alkyl-(C₆-C₁₂)-cycloalkyl bedeutet, wobei die unter 2. bis 6. definierten Reste substituiert sein können mit einem oder mehreren Resten aus der Reihe Fluor, Chlor, Hydroxyl, Hydroxamat, Sulfonamid, (C₁-C₆)-Alkyl, (C₆-C₁₂)-Aryl, Benzyl, (C₆-C₁₂)-Cycloalkyl;
      - R^{22b}: 1. Wasserstoff
      2. (C₁-C₁₂)-Alkyl
      3. (C₆-C₁₄)-Aryl,
      4. (C₃-C₁₄)-Cycloalkyl,
      5. (C₁-C₁₂)-Alkyl-(C₆-C₁₄)-aryl,
      6. (C₁-C₁₂)-Alkyl-(C₃-C₁₄)-cycloalkyl bedeutet, wobei die unter 2. bis 6. definierten Reste substituiert sein können mit einem oder mehreren Resten aus der Reihe Halogen (Fluor, Chlor, Brom, Iod); Nitro; Hydroxyl; Carboxyl; Tetrazol; Hydroxamat; Sulfonamid; Trifluorimid; Phosphonat; C₁-C₆-Alkyl; C₆-C₁₄-Aryl; Benzyl; C₃ -C₁₄-Cycloalkyl; COR^{24a}; CONR²⁵R²⁶; wobei
         R^{24a} einen Rest aus der Reihe C₁-C₈-Alkoxy; C₃-C₁₂-Alkenoxy; C₆-C₁₂-Aryloxy; di-C₁-C₈-Alkylamino-C₁-C₈-alkoxy; Acylamino-C₁-C₈-Alkoxy wie beispielsweise Acetyl-aminoethoxy, Nicotinoylaminoethoxy, Succinamidethoxy oder Pivaloylethoxy; C₆-C₁₂-Aryl-C₁-C₈-alkoxy, wobei die Arylgruppe gegebenenfalls mit ein bis drei Resten aus der Reihe Nitro, Halogen, C₁-C₄-Alkoxy, Amino, Hydroxyl, Hydroxy-C₂-C₈-Alkoxy, Dihydroxy-C₃-C₈-Alkoxy substituiert sein kann;
         R²⁵ und R²⁶ unabhängig voneinander Wasserstoff, C₁-C₁₀-Alkyl, C₃-C₁₀-Alkenyl, C₆-C₁₄-Aryl, C₁-C₆-Alkyl-C₆-₁₀-aryl bedeuten oder
         R²⁵ und R²⁶ zusammen einen Trimethylen-, Tetramethylen-, Pentamethylen- oder 3-Oxopentamethylenrest bilden;
      7. Q²-L³ bedeutet, wobei
         Q² Wasserstoff oder Q¹ ist; und
         L³ eine chemische Bindung oder L¹ bedeutet;
            Q¹ eine Amino-, Amidino-, Aminoalkylenimino-, Iminoalkylenamino- oder Guanidinogruppe, vorzugsweise eine Amidinogruppe bedeutet;
            L¹ C₆-C₁₄-Aryl-C₂-C₄-alkinylen; C₆-C₁₄-Aryl-C₁-C₃-Alkgen; C₆-C₁₄-Aryl-C₁-C₃-alkyloxyen oder -R^{14c}-CO-NR^{6c}R^{15c}- bedeutet, wobei
            R^{6c} Wasserstoff, C₁-C₄-Alkoxy, C₁-C₄-Alkyl oder Halogen-C₁-C₄-alkyl bedeutet;
            R^{14c} eine chemische Bindung, C₁-C₈-Alkylen, C₃-C₇-Cycloalkylen, C₂-C₅-Alkenylen, C₃-C₅-Alkinylen, C₆-C₁₀-Arylen, C₁-C₃-Alkyl-C₆-C₁₂-arylen, C₁-C₂-Alkyl-C₆-C₁₀-aryl-C₁-C₂-alkylen, C₆-C₁₀-Aryl-C₁-C₂-alkylen oder C₆-C₁₀-Aryloxy-C₁-C₂-alkylen bedeutet, und
            R^{15c} eine chemische Bindung, C₁-C₄-Alkylen, C₂-C₄-Alkenylen, C₂-C₄-Alkinylen, C₆-C₁₀-Arylen oder C₁-C₃-Alkyl-C₆-C₁₂-arylen bedeutet;
   oder b) ein Templat aus WO 95/04057, und zwar: 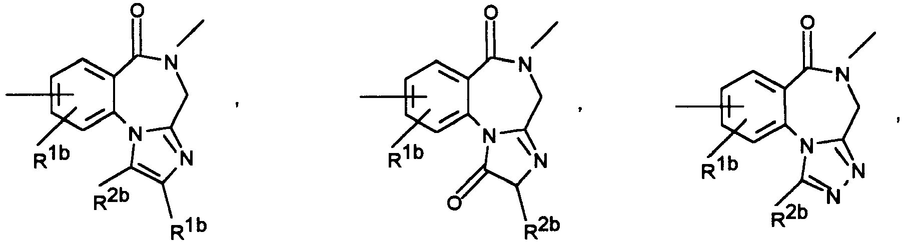 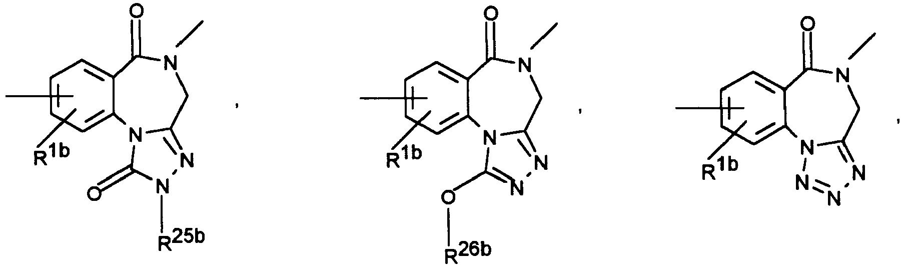 wobei hier R^{1b}, R^{2b}, R^{25b} und R^{26b} bedeuten:
      - R^{1b}, R^{2b}: unabhängig voneinander eine bis drei Gruppen aus der Reihe Wasserstoff und Halogen (Fluor, Chlor, Brom, Iod); und
      - R^{25b} und R^{26b}: unabhängig voneinander Wasserstoff, C₁-C₁₀-Alkyl, C₃-C₁₀-Alkenyl, C₆-C₁₄-Aryl, C₁-C₆-Alkyl-C₆-C₁₀-aryl bedeuten oder
      - R^{25b} und R^{26b}: zusammen einen Trimethylen-, Tetramethylen-, Pentamethylen- oder 3-Oxo-pentamethylenrest bilden;
   oder c) ein Templat aus EP 0 655 439, und zwar: 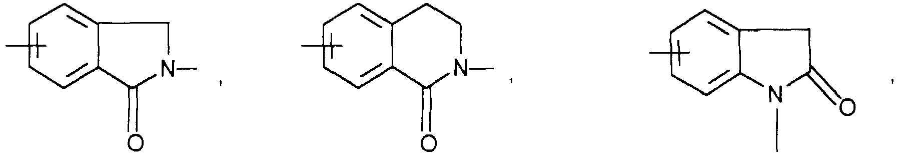 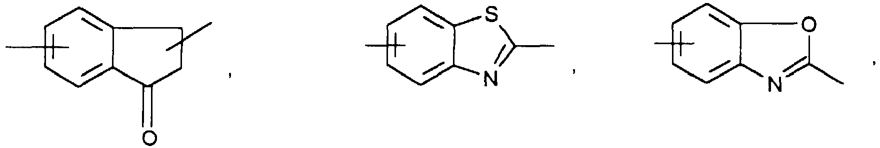 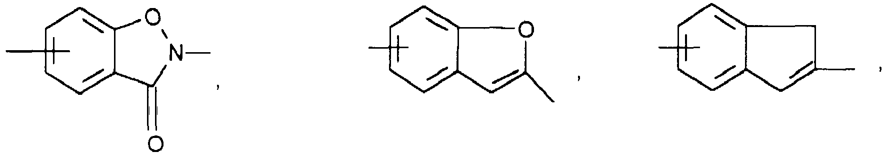 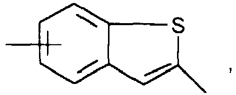
   oder d) ein Templat aus WO 94/12478, und zwar: 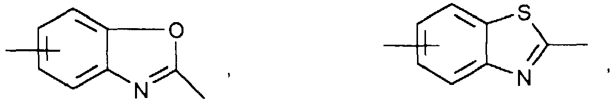
   oder e) ein Templat aus WO 94/18981, und zwar: 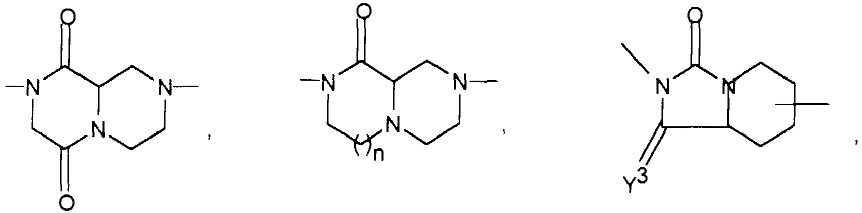 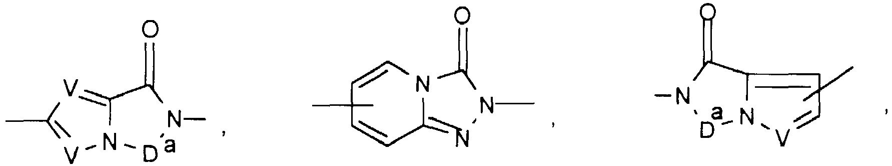 wobei Y³, V und D^{a} wie oben beschrieben, definiert sind;
   oder f) ein Templat aus EP 0 531 883, und zwar: 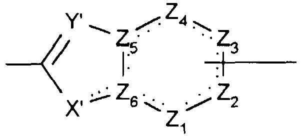 wobei bedeuten:
      - X': ein Sauerstoff-, Schwefel- oder Stickstoffatom oder eine -NR^{2b}-Gruppe, wobei
      - R^{2b}: ein Wasserstoffatom, eine geradkettige oder verzweigte Alkylgruppe mit 1 bis 15 C-Atomen, eine geradkettige oder verzweigte Alkenyl- oder Alkinylgruppe mit jeweils 3 bis 10 Kohlenstoffatomen, wobei die Doppel- oder Dreifachbindung nicht unmittelbar an das Stickstoffatom anschließen kann, eine Cycloalkyl- oder Cycloalkylalkylgruppe mit jeweils 3 bis 7 Kohlenstoffatomen im Cycloalkylteil, eine Arylgruppe, eine Alkylgruppe mit 2 bis 6 Kohlenstoffatomen, die ab der β-Position zu dem Stickstoffatom der -NR^{2b}-Gruppe durch eine R^{3b}O-, (R^{3b})₂N-, R^{4b}CO-NR^{3b}-, Alkylsulfonyl-NR^{3b}-, Arylsulfonyl-NR^{3b}-, Alkylsulfenyl-, Alkylsuffinyl-, Alkylsulfonyl- oder R^{5b}-Gruppe substituiert ist, oder eine Alkylgruppe mit 1 bis 6 Kohlenstoffatomen, die durch eine oder zwei Arylgruppen, R^{6b}OCO-, (R^{3b})₂NCO-, R^{5b} -CO-, R^{3b}O-CO-alkylen-NR^{3b}-CO-, (R^{3b})₂N-CO-alkylen-NR^{3b}-CO- oder R^{5b}CO-alkylen-NR^{3b}-CO-Gruppe substituiert ist, in denen R^{3b} und R^{5b} wie nachstehend definiert sind und R^{6b} ein Wasserstoffatom, eine Alkylgruppe mit 1 bis 6 Kohlenstoffatomen, eine Cycloalkylgruppe mit 5 bis 7 Kohlenstoffatomen oder eine Aralkylgruppe darstellt,
      - Y': eine NO-Gruppe, ein Stickstoffatom oder eine gegebenenfalls durch eine Alkylgruppe substituierte Methingruppe,
      - Z₁, Z₂, Z₃ und Z₄,: die gleich oder verschieden sein können, Methingruppen, Kohlenstoffatome, Iminogruppen oder Stickstoffatome, wobei mindestens einer der Reste Z₁ bis Z₄ ein Kohlenstoffatom enthalten muß und eine oder zwei zu einem Stickstoffatom benachbarte Methingruppe jeweils durch Carbonylgruppen ersetzt sein können,
      - Z₅ und Z₆: jeweils ein Kohlenstoffatom oder auch einer der Reste Z₅ oder Z₆ ein Stickstoffatom und der andere der Reste Z₅ oder Z₆ ein Kohlenstoffatom bedeuten,
      - R^{3b}: ein Wasserstoffatom, eine Alkylgruppe mit 1 bis 6 Kohlenstoffatomen, eine Aryl-, Aralkyl, Carboxyalkyl- oder Alkoxycarbonylalkylgruppe,
      - R^{4b}: ein Wasserstoffatom, eine Alkyl- oder Alkoxygruppe mit jeweils 1 bis 6 Kohlenstoffatomen, eine Aryl- oder Aralkylgruppe mit 1 bis 6 Kohlenstoffatomen im Alkylteil und
      - R^{5b}: eine Azetidino-, Pyrrolidino-, Hexamethylenimino- oder Heptamethyleniminogruppe oder eine Piperidinogruppe, in der die Methylenruppe in 4-Stellung durch ein Sauerstoffatom, eine Sulfenyl-, Suffinyl- oder Sulfonylgruppe oder durch eine durch eine R^{3b}, R^{4b}CO-, Alkylsulfonyl- oder Arylsulfonylgruppe substituierte Iminogruppe ersetzt sein kann, wobei R^{3b} und R^{4b} wie vorstehend erwähnt, definiert sind, darstellen,
F eine direkte Bindung, (C₁-C₆)-Alkandiyl, -O-, -CO-NR²-, -NR²-CO-, -NR²-C(O)-NR²-, -S(O)₂-NR², -NR²-S(O)₂-, -CR²=CR³, -C≡C-, die jeweils ein- oder zweifach durch (C₁-C₄)-Alkyl substituiert sein können;
   - G: 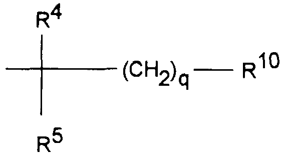
   - R², R³: unabhängig voneinander H, (C₁-C₄)-Alkyl, Trifluormethyl, Pentafluorethyl, (C₅-C₆)-Cycloalkyl, (C₅-C₆)-Cycloalkyl-(C₁-C₄)-alkyl, Phenyl, Benzyl;
   - R⁴: (C₁₀-C₁₄)-Cycloalkyl, (C₁₀-C₁₄)-Cycloalkyl-(C₁-C₄)-alkyl, oder R¹⁶OR⁹, R¹⁶NHR⁹, R¹⁶NHC(O)OR⁹, R¹⁶S(O)ₙNHR⁹, R¹⁶OC(O)NHR⁹, R¹⁶C(O)NHR⁹, R¹⁶C(O)R⁹, R¹⁶NHC(O)R⁹, R¹⁶NHS(O)ₙR⁹
   - R⁵: H, (C₁-C₆)-Alkyl, (C₅-C₆)-Cycloalkyl, (C₅-C₆)-Cycloalkyl-(C₁-C₄)-alkyl, Trifluormethyl, Pentafluorethyl, Phenyl, Benzyl;
   - R⁸: H, (C₁-C₄)-Alkyl, (C₅-C₆)-Cycloalkyl, (C₅-C₆)-Cycloalkyl-(C₁-C₂)-alkyl, Phenyl, Benzyl, Trifluormethyl, Pentafluorethyl;
   - R⁹: eine direkte Bindung oder (C₁-C₄)-Alkandiyl;
   - R¹⁰: C(O)R¹¹;
   - R¹¹: OH, (C₁-C₆)-Alkoxy, Phenoxy, Benzyloxy, (C₁-C₄)-Alkylcarbonyloxy-(C₁-C₄)-alkoxy, NH₂, Mono- oder Di-((C₁-C₆)-alkyl)-amino;
   - R¹²: H, (C₁-C₄)-Alkyl, Trifluormethyl, Pentafluorethyl, (C₅-C₆)-Cycloalkyl, (C₅-C₆)-Cycloalkyl-(C₁-C₂)-alkyl, (C₅-C₆)-Aryl, (C₅-C₆)-Aryl-(C₁-C₂)-alkyl, H₂N, R⁸R⁸NR⁹, R⁸NHC(O)R⁹, H₂N-C(=NH), H₂N-C(=NH)-NH-;
   wobei zwei benachbarte Substituenten R¹² ferner -OCH₂O-, -OCH₂CH₂O-bedeuten können;
   - R¹⁶: (C₁₀-C₁₄)-Cycloalkyl, (C₁₀-C₁₄)-Cycloalkyl-(C₁-C₄)-alkyl, die gegebenenfalls 1 oder 2-fach durch (C₁-C₄)-Alkyl, Trifluormethyl, Phenyl, Benzyl, (C₁-C₄)-Alkoxy, Phenoxy, Benzyloxy, =O oder Mono- oder Di-((C₁ -C₄)-alkyl)-amino substituiert sein können, wobei die Cycloalkylenreste bevorzugt 1-Adamantyl oder 2-Adamantyl sind, die wie vorstehend beschrieben, substituiert sein können;
   - n: 1 oder 2; und
   - q: 0 oder 1;
   in allen ihren stereoisomeren Formen und Mischungen davon in allen Verhältnissen, und ihre physiologisch verträglichen Salze.

Bevorzugt sind auch Verbindungen der Formel I, in denen A, B, D, F und G wie vorstehend für die ganz besonders bevorzugten Verbindungen der Formel I definiert sind und E ein Templat aus WO 95/04057, EP 0 655 439, WO 94/18981, WO 94/08962, EP 0 668 278, WO 94/12478 oder EP 0 531 883 ist, wobei letzteres bevorzugt wie vorstehend für die besonders bevorzugten Verbindungen der Formel I definiert ist, und besonders bevorzugt wie vorstehend für die ganz besonders bevorzugten Verbindungen der Formel I definiert ist.

Ein weiterer Gegenstand der vorliegenden Erfindung besteht darin, daß man einen an sich bekannten Fibrinogenrezeptorantagonisten in einen selektiven Vitronectinrezeptorantagonisten überführen kann, indem man die basische Gruppe (mit Spacer) eines Fibrinogenrezeptorantagonisten durch den Rest A-B-D ersetzt, der wie in Formel I definiert ist, wobei der Abstand zwischen R¹⁰ und dem ersten N-Atom in A 12 bis 13 kovalente Bindungen entlang des kürzesten Weges zwischen diesen Atomen beträgt.

Verbindungen der Formel I können generell, beispielsweise im Zuge einer konvergenten Synthese, durch Verknüpfung zweier oder mehrerer Fragmente, die sich retrosynthetisch aus der Formel I ableiten lassen, hergestellt werden. Bei der Herstellung der Verbindungen der Formel I kann es generell im Laufe der Synthese nötig sein, funktionelle Gruppen, die im jeweiligen Syntheseschritt zu unerwünschten Reaktionen oder Nebenreaktionen führen könnten, durch eine dem Syntheseproblem angepaßte Schutzgruppenstrategie temporär zu blockieren, was dem Fachmann bekannt ist. Die Methode der Fragmentverknüpfung ist nicht auf die nachfolgenden Beispiele beschränkt, sondern allgemein für Synthesen der Verbindungen der Formel I anwendbar.

Beispielsweise können Verbindungen der Formel I des Typs

A-B-D-E-C(O)NR²-G,

mit F = C(O)NR² durch Kondensation einer Verbindung der Formel II

A-B-D-E-M II,

wobei M für Hydroxycarbonyl, (C₁-C₆)-Alkoxycarbonyl, aktivierte Carbonsäurederivate wie Säurechloride, Aktivester oder gemischte Anhydride steht, mit HNR²-G hergestellt werden.

Zur Kondensation zweier Fragmente unter Bildung einer Amidbindung verwendet man vorteilhafterweise die an sich bekannten Kupplungsmethoden der Peptidchemie (siehe z.B. Houben-Weyl, Methoden der Organischen Chemie, Band 15/1 und 15/2, Georg Thieme Verlag, Stuttgart, 1974). Dazu ist es in der Regel nötig, daß vorhandene, nicht reagierende Aminogruppen durch reversible Schutzgruppen während der Kondensation geschützt werden. Gleiches gilt für nicht an der Reaktion beteiligte Carboxylgruppen, die bevorzugt als (C₁-C₆)-Alkyl-, Benzyl- oder tert-Butylester eingesetzt werden. Ein Aminogruppen-Schutz erübrigt sich, wenn die zu generierenden Aminogruppen noch als Nitro- oder Cyanogruppen vorliegen und erst nach der Kupplung durch Hydrierung gebildet werden. Nach der Kupplung werden die vorhandenen Schutzgruppen in geeigneter Weise abgespalten. Beispielsweise können NO₂-Gruppen (Guanidinoschutz), Benzyloxycarbonylgruppen und Benzylester abhydriert werden. Die Schutzgruppen vom tert-Butyltyp werden sauer abgespalten, während der 9-Fluorenylmethyloxycarbonylrest durch sekundäre Amine entfernt wird.

Verbindungen der Formel I mit R¹⁰ = SO₂R¹¹ werden beispielsweise hergestellt, in dem man Verbindungen der Formel I mit R¹⁰ = SH nach literaturbekannten Verfahren (vgl. Houben-Weyl, Methoden der Organischen Chemie, Bd. E12/2, Georg Thieme Verlag, Stuttgart 1985, S. 1058ff) zu Verbindungen der Formel I mit R¹⁰ = SO₃H oxidiert, aus denen dann direkt oder über entsprechende Sulfonsäurehalogenide durch Veresterung oder Knüpfung einer Amidbindung die Verbindungen der Formel I mit R¹⁰ = SO₂R¹¹ (R¹¹ ≠ OH) hergestellt werden. Oxidationsempfindliche Gruppen im Molekül, wie z.B. Amino-, Amidino- oder Guanidinogruppen werden, falls erforderlich, vor Durchführung der Oxidation durch geeignete Schutzgruppen geschützt.

Verbindungen der Formel I mit R¹⁰ = S(O)R¹¹ werden beispielsweise hergestellt, in dem man Verbindungen der Formel I mit R¹⁰ = SH in das entsprechende Sulfid (R¹⁰ = S^{⊝}) überführt und anschließend mit meta-Chlorperbenzoesäure zu den Suffinsäuren (R¹⁰ = SO₂H) oxidiert (vgl. Houben-Weyl, Methoden der Organischen Chemie, Bd. E11/1, Georg Thieme Verlag, Stuttgart 1985, S. 618f), aus denen nach literaturbekannten Methoden die entsprechenden Sulfinsäureester oder -amide R¹⁰ = S(O)R¹¹ (R¹¹ ≠ OH) hergestellt werden können. Generell können auch andere literaturbekannte Methoden zur Herstellung von Verbindungen der Formel I mit R¹⁰ = S(O)ₙR¹¹ (n = 1, 2) Anwendung finden (vgl. Houben-Weyl, Methoden der Organischen Chemie, Bd. E11/1, Georg Thieme Verlag, Stuttgart 1985, S. 618ff oder Bd. E11/2, Stuttgart 1985, S. 1055ff).

Verbindungen der Formel I mit R¹⁰ = P(O)(R¹¹)ₙ (n = 1, 2) werden nach literaturbekannten Verfahren (vgl. Houben-Weyl, Methoden der Organischen Chemie, Bd. E1 und E2, Georg Thieme Verlag, Stuttgart 1982) aus geeigneten Vorstufen aufgebaut, wobei die gewählte Synthesemethode dem Zielmolekül anzupassen ist.

Verbindungen der Formel I mit R¹⁰ = C(S)R¹¹ können nach Literaturverfahren hergestellt werden (vgl. Houben-Weyl, Methoden der Organischen Chemie, Bd. E5/1 und E5/2, Georg Thieme Verlag, Stuttgart 1985).

Verbindungen der Formel I mit R¹⁰ = S(O)ₙR¹¹ (n = 1, 2), P(O)(R¹¹)ₙ(n = 1, 2) oder C(S)R¹¹ können natürlich auch durch Fragmentverknüpfung, wie vorstehend beschrieben, hergestellt werden, was beispielsweise ratsam ist, wenn in F-G der Formel I z.B. eine (käufliche) Aminosulfonsäure, Aminosulfinsäure, Aminophosphonsäure oder Aminophosphinsäure oder daraus abgeleitete Derivate, wie Ester oder Amide, enthalten sind.

Verbindungen der Formel I, in denen A-B- einen Rest der Formel 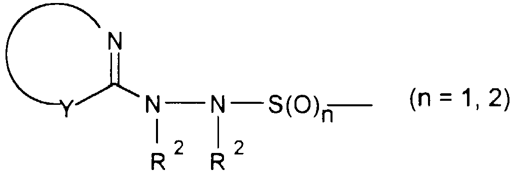 bedeutet, werden nach literaturbekannten Verfahren durch Reaktion von Verbindungen der Formel 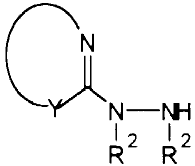 mit Sulfin- oder Sulfonsäurederivaten der Formel IV,

Q-S(O)ₙ-D-E-F-G IV

in denen Q z.B. gleich Cl oder NH₂ bedeutet, analog S. Birtwell et al., J. Chem. Soc. (1946) 491 oder Houben Weyl, Methoden der Organischen Chemie, Bd. E4, Georg Thieme Verlag, Stuttgart 1983; S. 620 ff, hergestellt.

Verbindungen der Formel I, in denen B -NR²-C(O)-NR²-, -NR²-C(O)O-, -NR²-C(O)S- und A die angegebene Bedeutung hat, werden z.B. hergestellt, indem man eine Verbindung der Formel V

Q-D-E-F-G V

in der Q HNR²-, HO- oder HS- bedeutet, mit einem geeigneten Kohlensäurederivat, bevorzugt Phosgen, Diphosgen (Chlorameisensäuretrichlormethylester), Triphosgen (Kohlensäure-bistrichlormethylester), Chlorameisensäureethylester, Chlorameisensäure-i-butylester, Bis-(1-hydroxy-1-H-benzotriazolyl)carbonat oder N,N'-Carbonyldiimidazol, in einem gegenüber den verwendeten Reagentien inerten Lösungsmittel, bevorzugt Dimethylformamid (DMF), Tetrahydrofuran (THF) oder Toluol, bei einer Temperatur zwischen -20°C und dem Siedepunkt des Lösungsmittels, bevorzugt zwischen 0°C und 60°C, zunächst zu einem substituierten Kohlensäurederivat der Formel VI, 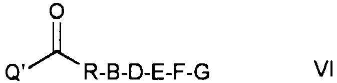 in der R -NR²-, -O- oder -S- und Q' je nach verwendetem Kohlensäurederivat Chlor, Methoxy, Ethoxy, Isobutoxy, Benzotriazol-1-oxy oder 1-Imidazolyl bedeuten, umsetzt.

Die Umsetzung dieser Derivate mit den einen Mono- oder Polycyclus enthaltenden Systemen des Typs VII 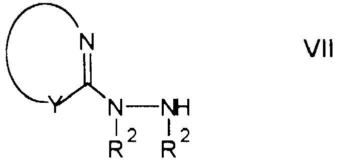 erfolgt in einem protischen oder aprotischen polaren, aber inerten organischen Lösungsmittel. Dabei haben sich bei der Umsetzung der Methylester (Q = OMe) mit den jeweiligen Verbindungen der Formel VII Methanol, Isopropanol oder THF bei Temperaturen von 20°C bis zur Siedetemperatur dieser Lösungsmittel bewährt. Bei den meisten Umsetzungen von Verbindungen der Formel VI mit salzfreien Verbindungen der Formel VII wird vorteilhaft in aprotischen inerten Lösungsmitteln wie THF, Dimethoxyethan, Dioxan gearbeitet. Aber auch Wasser kann unter Gebrauch einer Base (wie beispielsweise NaOH) als Lösungsmittel bei der Umsetzung von Verbindungen der Formel IV mit den Verbindungen VII verwendet werden. Wenn Q = Cl bedeutet, arbeitet man vorteilhaft unter Zusatz einer Base als Säurefänger, zur Abbindung der Halogenwasserstoffsäure.

Verbindungen der Formel I, in denen F -R²N-C(O)-NR²- oder -R²N-C(S)-NR²- ist, werden beispielsweise hergestellt, indem man eine Verbindung der Formel VIII

A-B-D-E-NHR² VIII

mit einem Isocyanat OCN-G oder Isothiocyanat SCN-G nach literaturbekannten Verfahren umsetzt.

Verbindungen der Formel I, in denen F -C(O)NR²-, -SO₂NR²- oder -C(O)O- ist, können z. B. durch Umsetzung von

A-B-D-E-C(O)Q bzw. A-B-D-E-SO₂Q

(Q ist eine leicht nukleophil substituierbare Abgangsgruppe, wie z.B. OH, Cl, OMe etc.) mit HR²N-G bzw. HO-G nach Literaturverfahren erhalten werden.

Verbindungen der Formel I, in denen A einen Mono- oder Polycyclus des Typs 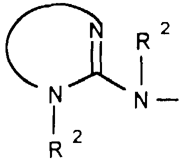 bedeutet, können beispielsweise hergestellt werden, indem man
a) eine Verbindung der Formel IX

   HR²N-D-E-F-G IX

   mit einem Mono- oder Polycyclus des Typs 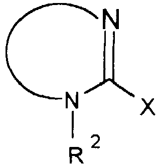 in der X eine nucleophil substituierbare Abgangsgruppe, wie z.B. Halogen oder SH, -SCH₃, SOCH₃, SO₂CH₃ oder HN-NO₂ darstellt, nach literaturbekannten Verfahren (siehe z.B. A.F Mckay et al., J. Med. Chem. 6 (1963) 587, M.N. Buchman et al., J. Am. Chem. Soc. 71 (1949), 766, F. Jung et al., J. Med. Chem. 34 (1991) 1110 oder G. Sorba et al., Eur. J. Med. Chem. 21 (1986), 391) umsetzt,
oder b) eine 1,2-Diaminoverbindung der Formel XVII 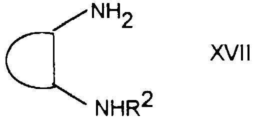 mit einem Isothiocyanat der Formel XIII

   SCN-B-D-E-F-G XIII

   zu einem Thioharnstoffderivat der Formel XVIII 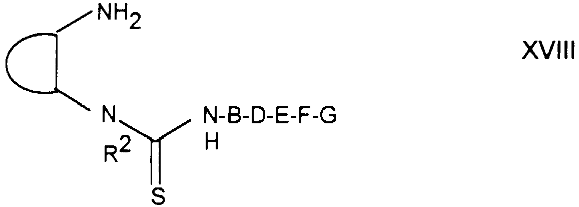 z.B. nach F. Janssens et al., J. Med. Chem. 28 (1985) 1925 umsetzt und anschließend wie dort beschrieben oder z.B. nach A. Mohsen et al., Synthesis (1977) 864 oder V. Ojka et al., Indian J. Chem. Sec. B 32 (3) (1993) 394 zu den Verbindungen der Formel I mit
   umsetzt,
oder c) eine 1-Nitro-2-Aminoverbindung der Formel XIX 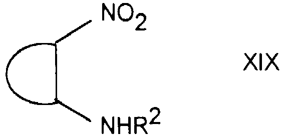 mit einem Isothiocyanat der Formel XIII zu einem Thioharnstoffderivat der Formel XX 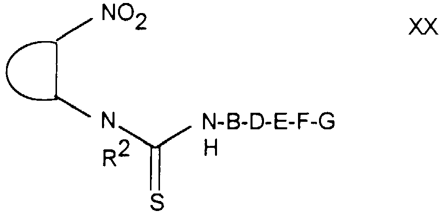 umsetzt, das nach Reduktion der Nitrogruppe mit Pd/C (vgl. z.B. F. Janssens et al., J. Med. Chem. 28 (1985) 1925) wie vorstehend beschrieben zu Verbindungen der Formel I mit 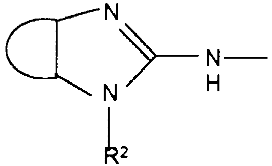 umgesetzt wird.

Verbindungen der Formel I, in denen A einen Mono- oder Polycyclus des Typs 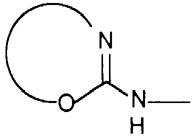 darstellt, werden nach literaturbekannten Verfahren erhalten z.B. aus Verbindungen der Formel 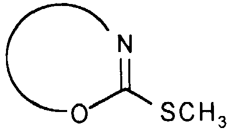 durch Reaktion mit Verbindungen der Formel R²-NH-B-D-E-F-G in Anlehnung an M. Yamato et al., Chem. Pharm. Bull 32(8) (1984) 3053, oder z. B. aus 1,2-Aminoalkoholen der Formel XII 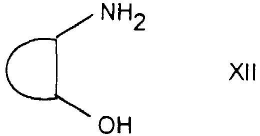 die zunächst durch Reaktion mit Isothiocyanaten der Formel XII

SCN-B-D-E-F-G XIII

zu Thioharnstoffen der Formel XIV 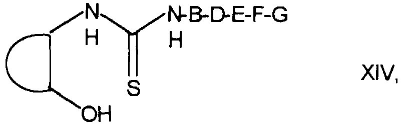 umgesetzt werden, die dann z.B. nach H.S. Chang et al., Chem. Lett. 8 (1986) 1291 oder E.A. Ibrahim et al., J. Heterocycl. Chem. 19(4) (1982) 761 zu den Verbindungen der Formel I mit 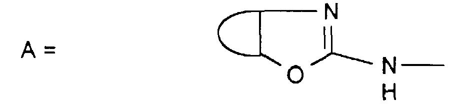 umgesetzt werden.

Verbindungen der Formel I, in denen A einen Mono- oder Polycyclus des Typs 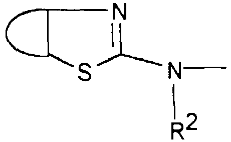 darstellt, werden nach literaturbekannten Verfahren erhalten, z.B. aus 1,2-Aminothiolen der Formel XV 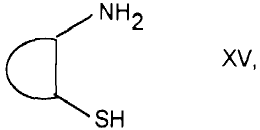 die zunächst durch Reaktion mit Isothiocyanaten der Formel XIII zu den Thioharnstoffen der Formel XVI 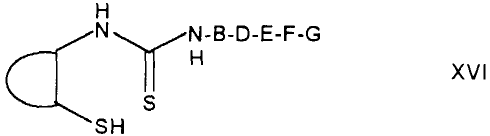 umgesetzt werden, die dann z.B. nach J. Garvin et al., J. Heterocycl. Chem. 28 (1991) 359 zu den Verbindungen der Formel I mit
A= 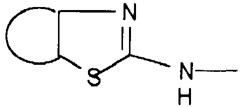
umgesetzt werden.

Verbindungen der Formel I, in denen D -C≡C- ist, können beispielsweise hergestellt werden, indem man eine Verbindung der Formel X

X-E-F-G X

in der X = I oder Br bedeutet, mit einer Verbindung des Typs A-B-C≡CH in einer Palladium-katalysierten Reaktion, wie z.B. bei A. Arcadi et al, Tetrahedron Lett. 1993, 34, 2813 oder E.C. Taylor et al., J. Org. Chem. 1990, 55, 3222 beschrieben, umsetzt.

Analog können Verbindungen der Formel I, in denen F gleich -C≡C- ist beispielsweise durch Verknüpfung von Verbindungen der Formel XI

A-B-D-E-X XI

in der X I oder Br bedeutet, mit einer Verbindung des Typs HC≡C-G in einer Palladium-katalysierten Reaktion hergestellt werden.

Die Synthese der Fibrinogenrezeptorantagonisten-Template E erfolgt wie in den jeweiligen Patenten, Patentanmeldungen oder Publikationen beschrieben, wobei während der Templatsynthese, oder anschließend, vorzugsweise bereits während der Templatsynthese, funktionelle Gruppen in das Templat eingebaut oder an das Templat geknüpft werden, die die spätere Anknüpfung von A-B-D und F-G durch Fragmentverknüpfung erlauben, wie nachfolgend für ein Templat aus WO 94/18981 exemplarisch beschrieben:

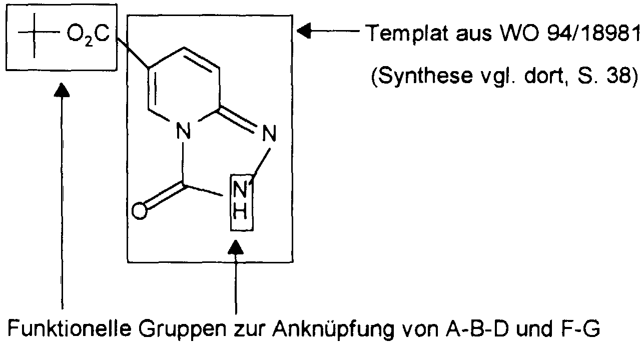

Beispiel für die Anknüpfung von A-B-D und F-G: 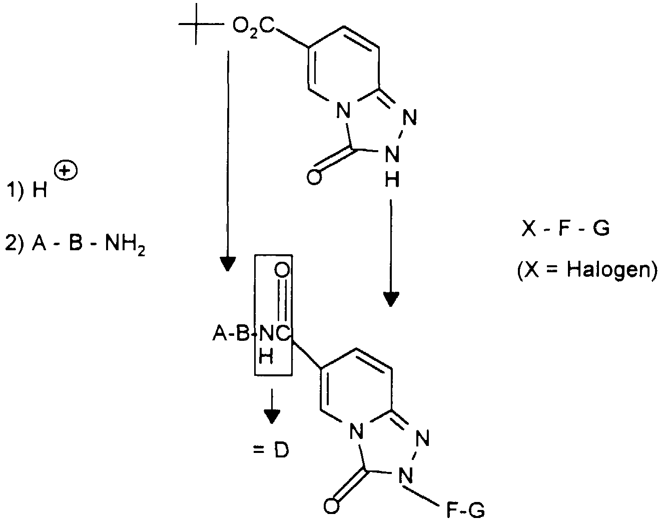 Literaturbekannte Herstellungsverfahren sind z.B. in J. March, Advanced Organic Chemistry, Third Edition (John Wiley & Sons, 1985) beschrieben.

Die Verbindungen der Formel I und ihre physiologisch verträglichen Salze können am Tier, bevorzugt am Säugetier, und insbesondere am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Salzes davon, neben üblichen pharmazeutisch einwandfreien Träger- und Zusatzstoffen enthalten. Die Zubereitungen enthalten normalerweise etwa 0,5 bis 90 Gew.-% der therapeutisch wirksamen Verbindung.

Die Heilmittel können oral, z.B. in Form von Pillen, Tabletten, Lacktabletten, Dragees, Granulaten, Hart- und Weichgelatinekapseln, Lösungen, Sirupen, Emulsionen, Suspensionen oder Aerosolmischungen verabreicht werden. Die Verabreichung kann aber auch rektal, z.B. in Form von Suppositorien, oder parenteral, z.B. in Form von Injektions- oder Infusionslösungen, Mikrokapseln oder Rods, perkutan, z.B. in Form von Salben oder Tinkturen, oder nasal, z.B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z. B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z. B.Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate oder Rods eignen sich Mischpolymerisate aus Glykolsäure und Milchsäure.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder deren physiologisch verträglichen Salze enthalten; ferner neben mindestens einer Verbindung der Formel I noch einen oder mehrere andere therapeutisch wirksame Stoffe.

Die Dosis kann innerhalb weiter Grenzen variieren und ist in jedem einzelnen Fall den individuellen Gegebenheiten anzupassen.

Bei oraler Verabreichung kann die Tagesdosis zwischen 0,01 bis 100 mg/kg, vorzugsweise 0,1 bis 5 mg/kg, insbesondere 0,3 bis 0,5 mg/kg Körpergewicht zur Erzielung wirksamer Ergebnisse, betragen. Auch bei intravenöser Applikation beträgt die Tagesdosis im allgemeinen etwa 0,01 bis 100 mg/kg, vorzugsweise 0,05 bis 10 mg/kg Körpergewicht. Die Tagesdosis kann, insbesondere bei der Applikation größerer Mengen, in mehrere, z.B. 2, 3 oder 4 Teilverabreichungen aufgeteilt werden. Gegebenenfalls kann es, je nach individuellem Verhalten, erforderlich werden, von der angegebenen Tagesdosis nach oben oder nach unten abzuweichen.

Außer als Arzneimittelwirkstoffe können die Verbindungen der Formel I in Diagnoseverfahren, zum Beispiel in in vitro-Diagnosen, oder als Hilfsmittel bei biochemischen Untersuchungen eingesetzt werden, bei denen eine Hemmung des Vitronectinrezeptors beabsichtigt ist.

Die Hemmung der Knochenresorption durch die erfindungsgemäßen Verbindungen kann beispielsweise mit Hilfe eines Osteoclasten-Resorptions-Tests ("PIT ASSAY') beispielsweise analog WO 95/32710 bestimmt werden. Die Testmethoden, nach denen die antagonistische Wirkung der erfindungsgemäßen Verbindungen auf den Vitronectinrezeptor αᵥβ₃ bestimmt werden können, sind nachfolgend beschrieben.

### Testmethode 1:

### Inhibierung der Bindung von humanem Vitronectin (Vn) an humanen Vitronectinrezeptor (VnR) αᵥβ₃: ELISA-Test.

1. Reinigung von humanem Vitronectin
   Humanes Vitronectin wird aus menschlichem Plasma isoliert und durch Affinitätschromatographie nach der Methode von Yatohyo et al., Cell Structure and Function, 1988, 23, 281-292 gereinigt.
2. Reinigung von humanem Vitronectinrezeptor (αᵥβ₃)
   Humaner Vitronectinrezeptor wird aus der menschlichen Plazenta nach der Methode von Pytela et al., Methods Enzymol. 1987, 144, 475 gewonnen. Humaner Vitronectinrezeptor αᵥβ₃ kann auch aus einigen Zellinien (z.B. aus 293 Zellen, einer humanen embryonalen Nierenzellinie), die mit DNA Sequenzen für beide Untereinheiten αᵥ und β₃ des Vitronectinrezeptors cotransfiziert sind, gewonnen werden. Die Untereinheiten werden mit Octylglycosid extrahiert und anschließend über Concanavalin A, Heparin-Sepharose und S-300 chromatographiert.
3. Monoklonale Antikörper
   Murine monoklonale Antikörper, spezifisch für die β₃ Untereinheit des Vitronectinrezeptors, werden nach der Methode von Newman et al., Blood, 1985, 227-232, oder nach einem ähnlichen Verfahren hergestellt. Das Kaninchen Fab 2 anti-Maus Fc Konjugat an Meerrettich Peroxidase (anti-Maus Fc HRP) wurde von Pel Freeze (Katalog Nr. 715 305-1) bezogen.
4. ELISA Test
   Nunc Maxisorp 96 well-Mikrotiter-Platten werden mit einer Lösung von humanem Vitronectin (0,002 mg/ml, 0,05 ml/well) in PBS (Phosphatgepufferte Kochsalzlösung) über Nacht bei 4°C belegt. Die Platten werden zweimal mit PBS/0,05 % Tween 20 gewaschen und durch Inkubieren (60 min) mit Rinderserum-Albumin (BSA, 0,5 %, RIA Güteklasse oder besser) in Tris-HCl (50 mM), NaCl (100 mM), MgCl₂ (1 mM), CaCl₂ (1 mM), MnCl₂ (1 mM), pH 7, geblockt. Man stellt Lösungen von bekannten Inhibitoren und von den Testsubstanzen in Konzentration von 2x10¹² bis 2x10⁻⁶ Mol/l in Assay-Puffer [BSA (0,5 %, RIA-Güteklasse oder besser) in Tris-HCl (50 mM), NaCl (100 mM), MgCl₂ (1 mM), CaCl₂ (1 mM), MnCl₂ (1 mM), pH 7] her. Die geblockten Platten werden entleert, und jeweils 0,025 ml dieser Lösung, die eine definierte Konzentration (2x10⁻¹² bis 2x10⁻⁶) entweder an einem bekannten Inhibitor oder an einer Testsubstanz enthält, werden in jedes well gegeben. 0,025 ml einer Lösung des Vitronectinrezeptors im Testpuffer (0,03 mg/ml) werden in jedes well der Platte pipettiert und die Platte wird auf einem Schüttler 60-180 min bei Raumtemperatur inkubiert. In der Zwischenzeit wird eine Lösung (6 ml/Platte) eines für die β₃-Untereinheit des Vitronectinrezeptors spezifischen murinen monoklonalen Antikörpers im Assay-Puffer (0,0015 mg/ml) hergestellt. Zu dieser Lösung gibt man einen zweiten Kaninchen- Antikörper (0,001 ml Stammlösung/6 ml der murinen monoklonalen anti-β₃-Antikörper Lösung), der ein anti-Maus Fc-HRP-Antikörper-Konjugat darstellt, und dieses Gemisch aus murinem anti-β₃ Antikörper und Kaninchen-anti-Maus-Fc-HRP-Antikörper-Konjugat läßt man während der Zeit der Rezeptor-Inhibitor-Inkubation inkubieren.
   Die Testplatten werden 4 mal mit PBS-Lösung, die 0,05 % Tween-20 enthält, gewaschen und man pipettiert jeweils 0,05 ml/well der Antikörpermischung in jedes well der Platte und inkubiert 60-180 min.. Die Platte wird 4 mal mit PBS/0,05 % Tween-20 gewaschen und anschließend mit 0,05 ml/well einer PBS-Lösung, die 0,67 mg/ml o-Phenylendiamin und 0,012 % H₂O₂ enthält, entwickelt. Alternativ hierzu kann o-Phenylendiamin in einem Puffer (pH 5) eingesetzt werden, der Na₃PO₄ (50 mM) und Zitronensäure enthält. Die Farbentwicklung wird mit 1 N H₂SO₄ (0,05 ml/well) gestoppt. Die Absorption jedes well wird bei 492-405 nm gemessen und die Daten werden nach Standardmethoden ausgewertet.

### Testmethode 2:

### Inhibierung der Bindung von Kistrin an humanen Vitronectinrezeptor (VnR) αᵥβ₃: ELISA-Test

1. Reinigung von Kistrin
   Kistrin wird nach den Methoden von Dennis et al., wie in Proc. Natl. Acad. Sci. USA 1989, 87, 2471-2475 und PRO-TEINS: Structure, Function and Genetics 1993, 15, 312-321, beschrieben, gereinigt.
2. Reinigung von humanem Vitronectinrezeptor (αᵥβ₃)
   siehe Testmethode 1.
3. Monoklonale Antikörper
   siehe Testmethode 1.
4. ELISA-Test
   Die Fähigkeit von Substanzen, die Bindung von Kistrin an den Vitronectinrezeptor zu inhibieren, kann mit einem ELISA-Test ermittelt werden. Zu diesem Zweck werden Nunc 96 well-Mikrotiterplatten mit einer Lösung von Kistrin (0,002 mg/ml) nach der Methode von Dennis et al., wie in PROTEINS: Structure, Function and Genetics 1993, 15, 312-321, beschrieben, belegt. Die weitere experimentelle Durchführung des ELISA-Test erfolgt wie bei Testmethode 1, Punkt 4, beschrieben.

### Testmethode 3:

### Inhibierung der Bindung von mit αᵥβ₃ transfizierten 293 Zellen an humanes Vitronectin:

Zelltest
293 Zellen, eine humane embryonale Nierenzellinie, die mit DNA-Sequenzen für die αᵥ und β₃ Untereinheiten des Vitronectinrezeptors αᵥβ₃ cotransfiziert sind, werden nach der FACS Methode unter dem Gesichtspunkt einer hohen Expressionsrate (> 500000 αᵥβ₃ Rezeptoren/Zelle) selektiert. Die ausgewählten Zellen werden kultiviert und erneut mittels FACS aussortiert, um eine stabile Zellinie (15 D) mit Expressionsraten > 1000000 Kopien an αᵥβ₃ pro Zelle zu erhalten.

Eine Limbro 96 well-Gewebekulturplatte mit flachem Boden wird mit humanem Vitronectin (0,01 mg/ml, 0,05 ml/well) in Phosphat-gepufferter Kochsalzlösung (PBS) über Nacht bei 4°C belegt und anschließend mit 0,5 %igem BSA geblockt. Man stellt Lösungen der Testsubstanzen von 10⁻¹⁰ bis 2 x 10⁻³ Mol/l in glucosehaltigem DMEM Medium her und gibt jeweils 0,05 ml/well der Lösung auf die Platte. Die Zellen, die hohe Level an αᵥβ₃ exprimieren (z.B. 15 D), werden in glucosehaltigem DMEM Medium suspendiert und die Suspension wird auf einem Gehalt von 25000 Zellen/0,05 ml Medium eingestellt. 0,05 ml dieser Zellsuspension werden in jedes well gegeben und die Platte bei 37°C 90 min inkubiert. Die Platte wird 3 x mit warmen PBS gewaschen um nichtgebundene Zellen zu entfernen. Die gebundenen Zellen werden in Zitratpuffer (25 mM, pH 5,0), der 0,25 % Triton X-100 enthält, lysiert. Anschließend wird das Hexoseamidase-Substrat p-Nitrophenyl-N-acetyl-β-D-glucosaminid zugegeben und die Platte 90 min bei 37°C inkubiert. Die Reaktion wird mit einem Glycin (50 mM)/EDTA (5 mM)-Puffer (pH 10,4) gestoppt und die Absorption von jedem well bei 405-650 nm gemessen.

Die antagonistische Wirkung der erfindungsgemäßen Verbindungen auf den Fibrinogenrezeptor α_{∥b}β₃, insbesondere zur Selektivitätsbestimmung, kann wie in US 5 403 836, S. 237 beschrieben, durchgeführt werden.

## Patentansprüche

1. Verbindung der Formel I,
A-B-D-E-F-G (I)
worin bedeuten:
A 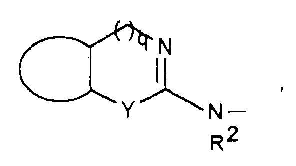 wobei 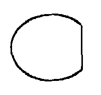
ein 5- bis 10-gliedriges mono- oder polycyclisches, aromatisches oder nicht aromatisches Ringsystem darstellt, das 1 bis 4 Heteroatome aus der Reihe N, O, S enthalten kann und gegebenenfalls ein- oder mehrfach mit R¹², R¹³, R¹⁴ und R¹⁵ substituiert sein kann;
B eine direkte Bindung, (C₁-C₈)-Alkandiyl, (C₅-C₁₀)-Argen, (C₃-C₈)-Cycloalkylen, -C≡C-, -NR²-, -NR²-C(O)-, -NR²-C(O)-NR²-, -NR²-C(S)-NR²-, -O-C(O)-, -NR²-S(O)-, -NR²-S(O)₂-, -O-, -S-, -CR²=CR³-, die jeweils ein-oder zweifach durch (C₁-C₈)-Alkyl substituiert sein können;
D eine direkte Bindung, (C₁-C₈)-Alkandiyl, (C₅-C₁₀)-Arylen, -O-, -NR²-, -CO-NR²-, -NR²-CO-, -NR²-C(O)-NR²-, -NR²-C(S)-NR²-, -OC(O)-, -C(O)O-, -S(O)-, -S(O)2-, -S(O)₂-NR²-, -S(O)-NR²-, -NR²-S(O)-, -NR²-S(O)₂-, -S-, -CR²=CR³-, -C≡C-, die jeweils ein- oder zweifach durch (C₁-C₈)-Alkyl, -CR²=CR³-, (C₅-C₆)-Aryl substituiert sein können,
wobei, wenn B eine direkte Bindung ist, D nicht gleich -CO-NR²-, -C(O)O-, -S(O)-, -S(O)₂-, -S(O)-NR²-, -S(O)₂-NR²- sein kann;
E ein Templat aus der Reihe der Fibrinogenrezeptorantagonisten bedeutet;
F ist wie D definiert;
G 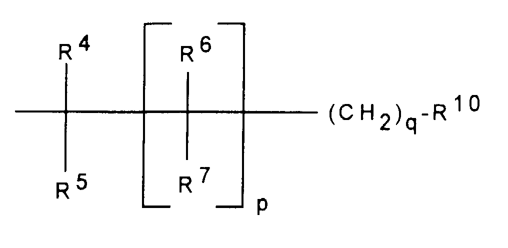
R², R³ unabhängig voneinander H, (C₁-C₁₀)-Alkyl, das gegebenenfalls durch Fluor ein- oder mehrfach substituiert ist, (C₃-C₁₂)-Cycloalkyl, (C₃-C₁₂)-Cycloalkyl-(C₁-C₈-alkyl, (C₅-C₁₄)-Aryl, (C₅-C₁₄)-Aryl-(C₁-C₈)-alkyl, R⁸OC(O)R⁹, R⁸R⁸NC(O)R⁹, R⁸C(O)R⁹;
R⁴, R⁵, R⁶, R⁷ unabhängig voneinander H, Fluor, OH, (C₁-C₈)-Alkyl, (C₃-C₁₄)-Cycloalkyl, (C₃-C₁₄)-Cycloalkyl-(C₁-C₈)-alkyl, oder R⁸OR⁹, R⁸SR⁹, R³CO₂R⁹, R⁸OC(O)R⁹, R⁸-(C₅-C₁₄)-Aryl-R⁹, R¹N(R²)R⁹, R⁸R⁸NR⁹, R⁸N(R²)C(O)OR⁹, R⁸S(O)ₙN(R²)R⁹, R⁸OC(O)N(R²)R⁹, R⁸C(O)N(R²)R⁹, R⁸N(R²)C(O)N(R²)R⁹, R⁸N(R²)S(O)ₙN(R²)R⁹, R⁸S(O)ₙR⁹, R⁸SC(O)N(R²)R⁹, R⁸C(O)R⁹, R⁸N(R²)C(O)R⁹, R⁸N(R²)S(O)ₙR⁹;
R⁸ H, (C₁-C₈)-Alkyl, (C₃-C₁₄)-Cycloalkyl, (C₃-C₁₄)-Cycloalkyl-(C₁-C₈)-alkyl, (C₅-C₁₄)-Aryl, (C₅-C₁₄)-Aryl-(C₁-C₈)-alkyl, wobei die Alkylreste durch Fluor ein- oder mehrfach substituiert sein können;
R⁹ eine direkte Bindung oder (C₁-C₈)-Alkandiyl;
R¹⁰ C(O)R¹¹, C(S)R¹¹, S(O)ₙR¹¹, P(O)(R¹¹)ₙ oder ein vier- bis achtgliedriger, gesättigter oder ungesättigter Heterocyclus, der 1, 2, 3 oder 4 Heteroatome aus der Reihe N, O, S enthält, wie z.B. Tetrazolyl, Imidazolyl, Pyrazolyl, Oxazolyl, Thiadiazolyl;
R¹¹ OH, (C₁-C₈)-Alkoxy, (C₅-C₁₄)Aryl-(C₁-C₈)-alkoxy, (C₅-C₁₄)-Aryloxy, (C₁-C₈)-Alkylcarbonyloxy-(C₁-C₄)-alkoxy, (C₅-C₁₄)-Aryl-(C₁-C₈)-alkylcarbonyloxy-(C₁-C₆)-alkoxy, NH₂, Mono- oder Di-((C₁-C₈)-alkyl)-amino, (C₅-C₁₄)-Aryl-(C₁-C₈)-alkylamino, (C₁-C₈)-Dialkylaminocarbonylmethyloxy, (C₅-C₁₄)-Aryl-(C₁-C₈)-dialkylaminocarbonylmethyloxy oder (C₅-C₁₄)-Arylamino oder den Rest einer L- oder D-Aminosäure;
R¹² R¹³, R¹⁴, R¹⁵ unabhängig voneinander H, (C₁-C₁₀)-Alkyl, das gegebenenfalls durch Fluor ein- oder mehrfach substituiert ist, (C₃-C₁₂)-Cycloalkyl, (C₃-C₁₂)-Cycloalkyl-(C₁-C₈)-alkyl, (C₅-C₁₄)-Aryl, (C₅-C₁₄)-Aryl-(C₁-C₈)-alkyl, H₂N, R⁸ONR⁹, R⁸OR⁹, R⁸OC(O)R⁹, R⁸R⁸NR⁹, R⁸-(C₅-C₁₄)-Arg-R⁹, HO-(C₁ -C₈)-Alkyl-N(R²)R⁹, R⁸N(R²)C(O)R⁹, R⁸C(O)N(R²)R⁹, R⁸C(O)R⁹, R²R³N-C(=NR²)-NR², R²R³N-C(=NR²), =O, =S; wobei zwei benachbarte Substituenten aus R¹² bis R¹⁵ ferner -OCH₂O-, -OCH₂CH₂O-bedeuten können;
Y NR², O, S;
n 1 oder 2;
p, q unabhängig voneinander 0 oder 1;
in allen ihren stereoisomeren Formen und Mischungen davon in allen Verhältnissen, und ihre physiologisch verträglichen Salze,
wobei Verbindungen ausgenommen sind, in denen E
a) ein 6-gliedriges aromatisches Ringsystem, das bis zu 4 N-Atome enthalten kann und mit 1 bis 4 gleichen oder verschiedenen beliebigen Substituenten substituiert sein kann, bedeutet, oder
b) 4-Methyl-3-oxo-2,3,4,5-tetrahydro-1-H-1,4-benzodiazepin bedeutet.

2. Verbindung der Formel I gemäß Anspruch 1, in der bedeuten:
A 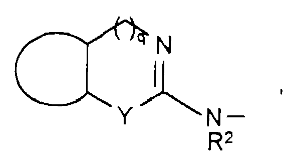 wobei 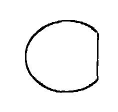
ein 5- bis 10-gliedriges mono- oder polycyclisches, aromatisches oder nicht aromatisches Ringsystem darstellt, das 1 bis 4 Heteroatome aus der Reihe N, O, S enthalten kann und gegebenenfalls ein- oder mehrfach mit R¹², R¹³, R¹⁴ und R¹⁵ substituiert sein kann;
B eine direkte Bindung, (C₁-C₆)-Alkandiyl, (C₅-C₈)-Arylen, (C₃-C₈)-Cycloalkylen, -C≡C-, -NR²-, -NR²-C(O)-, - NR²-C(O)-NR²-, -NR²-S(O)-, -NR²-S(O)₂-, -O-, -CR²=CR³-, die jeweils ein- oder zweifach durch (C₁-C₆)-Alkyl substituiert sein können;
D eine direkte Bindung, (C₁-C₈)-Alkandiyl, (C₅-C₈)-Arylen, -O-, -NR²-, -CO-NR²-, -NR²-CO-, -NR²-C(O)-NR²- , -OC(O)-, -C(O)O-, -S(O)₂-, -S(O)₂-NR²-, -NR²-S(O)₂-, -S-, -CR²=CR³, -C≡C-, die jeweils ein- oder zweifach durch (C₁-C₈)-Alkyl, -CR²=CR³ , (C₅-C₆)-Aryl substituiert sein können, wobei, wenn B eine direkte Bindung ist, D nicht gleich -CO-NR²-, -C(O)O-, -SO₂-, -S(O)₂-NR²- sein kann;
F ist wie D definiert:
G 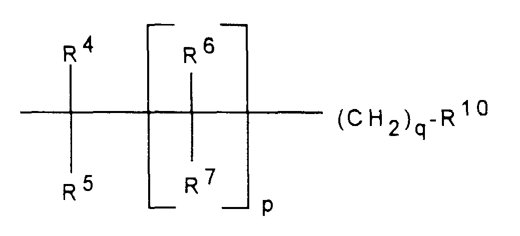
R² R³ unabhängig voneinander H, (C₁-C₁₀)-Alkyl, das gegebenenfalls durch Fluor ein- oder mehrfach substituiert ist, (C₃-C₈)-Cycloalkyl, (C₃-C₈)-Cycloalkyl-(C₁-C₆)-alkyl, (C₅-C₁₂)-Aryl, (C₅-C₁₂)-Aryl-(C₁-C₆)-alkyl, R⁸OC(O)R⁹, R⁸R⁸NC(O)R⁹, R⁸C(O)R⁹;
R⁴, R⁵, R⁶, R⁷ unabhängig voneinander H, Fluor, OH, (C₁-C₈)-Alkyl, (C₅-C₁₄)-Cycloalkyl, (C₅-C₁₄)-Cycloalkyl-(C₁-C₈)-alkyl, oder R⁸OR⁹, R⁸SR⁹, R⁸CO₂R⁹, R⁸OC(O)R⁹, R⁸-(C₅-C₁₄)-Aryl-R⁹, R⁸N(R²)R⁹, R⁸R⁸NR⁹, R⁸N(R²)C(O)OR⁹, R⁸S(O)ₙN(R²)R⁹, R⁸OC(O)N(R²)R⁹, R⁸C(O)N(R²)R⁹, R⁸N(R²)C(O)N(R²)R⁹, R⁸N(R²)S(O)ₙN(R²)R⁹, R⁸S(O)ₙR⁹, R⁸SC(O)N(R²)R⁹, R⁸C(O)R⁹, R⁸N(R²)C(O)R⁹, R⁸N(R²)S(O)ₙR⁹;
R⁸ H, (C₁-C₆)-Alkyl, (C₅-C₁₄)-Cycloalkyl, (C₅-C₁₄)-Cycloalkyl-(C₁-C₆)-alkyl, (C₅-C₁₂)-Aryl, (C₅-C₁₂)-Aryl-(C₁-C₆)-alkyl, wobei die Alkylreste durch Fluor ein- oder mehrfach substituiert sein können;
R⁹ eine direkte Bindung oder (C₁-C₆)-Alkandiyl;
R¹⁰ C(O)R¹¹, C(S)R¹¹, S(O)ₙR¹¹, P(O)(R¹¹)ₙ oder ein vier- bis achtgliedriger, gesättigter oder ungesättigter Heterocyclus, der 1, 2, 3 oder 4 Heteroatome aus der Reihe N, O, S enthält;
R¹¹ OH, (C₁-C₆)-Alkoxy, (C₅-C₁₂)-Aryl-(C₁-C₆)-alkoxy, (C₅-C₁₂)-Aryloxy, (C₁-C₆)-Alkylcarbonyloxy-(C₁-C₄)-alkoxy, (C₅-C₁₂)-Aryl-(C₁-C₆)-alkylcarbonyloxy-(C₁-C₆)-alkoxy, NH₂, Mono- oder Di-((C₁-C₆)-alkyl)-amino, (C₅-C₁₂)-Aryl-(C₁-C₆)-alkylamino, (C₁-C₆)-Dialkylaminocarbonylmethyloxy;
R¹², R¹³, R¹⁴, R¹⁵ unabhängig voneinander H, (C₁-C₈)-Alkyl, das gegebenenfalls durch Fluor ein- oder mehrfach substituiert ist, (C₃-C₈)-Cycloalkyl, (C₃-C₈)-Cycloalkyl-(C₁-C₆)-alkyl, (C₅-C₁₂)-Aryl, (C₅-C₁₂)-Aryl-(C₁-C₆)-alkyl, H₂N, R⁸ONR⁹, R⁸OR⁹, R⁸OC(O)R⁹, R⁸-(C₅-C₁₂)-Aryl-R⁹, R⁸R⁸NR⁹, HO-(C₁-C₅)-Alkyl-N(R²)R⁹, R⁸N(R²)C(O)R⁹, R⁸C(O)N(R²)R⁹, R⁸C(O)R⁹, R²R³N-C(=NR²), R²R³N-C(=NR²)-NR², =O, =S;
wobei zwei benachbarte Substituenten aus R¹² bis R¹⁵ zusammen ferner -OCH₂O-, - OCH₂CH₂-O-, -OC(CH₃)₂O- bedeuten können;
Y NR², O, S;
n 1 oder 2;
p, q unabhängig voneinander 0 oder 1;
und E wie in Anspruch 1 definiert ist;
in allen ihren stereoisomeren Formen und Mischungen davon in allen Verhältnissen, und ihre physiologisch
verträglichen Salze.

3. Verbindung der Formel I gemäß Anspruch 1 und/oder 2, in der bedeuten:
A einen der Reste 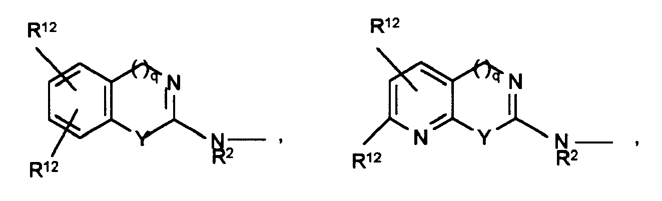 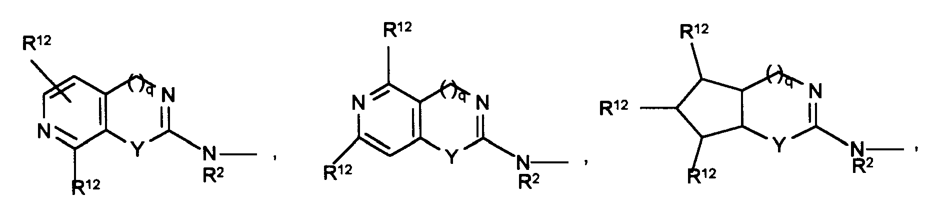 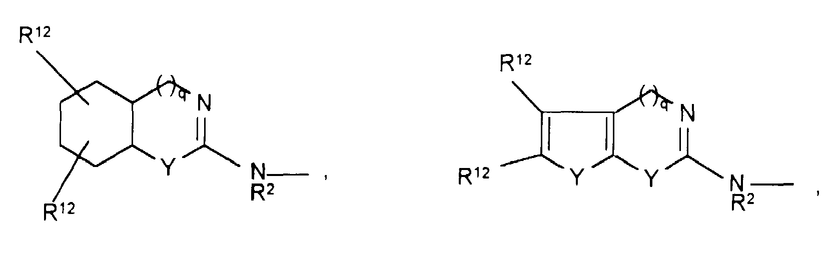 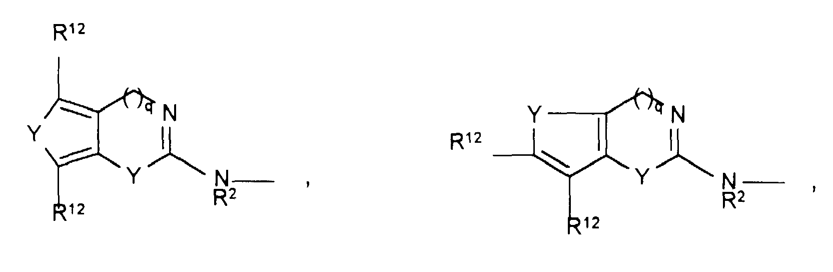 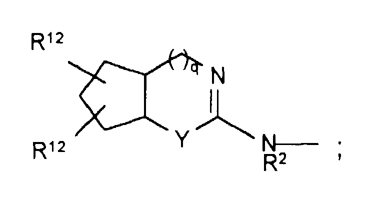
B eine direkte Bindung, (C₁-C₆)-Alkandiyl, (C₅-C₆)-Arylen, (C₅-C₆)-Cycloalkylen, -C≡C-, -NR²-, -NR²-C(O)-, - NR²-S(O)₂-, -O-, -CR²=CR³-, die jeweils ein- oder zweifach durch (C₁-C₆)-Alkyl substituiert sein können;
D eine direkte Bindung, (C₁-C₆)-Alkandiyl, (C₅-C₆)-Arylen, -O-, -NR²-, -NR²-C(O)-, -C(O)NR²-, -NR²-C(O)-NR²-, -OC(O)-, -S(O)₂-NR²-, -NR²-S(O)₂-, -CR²=CR³-, die jeweils ein- oder zweifach durch (C₁-C₆)-Alkyl substituiert sein können, wobei, wenn B eine direkte Bindung ist, D nicht gleich -C(O)NR²-, -S(O)₂NR²- sein kann;
E
a) 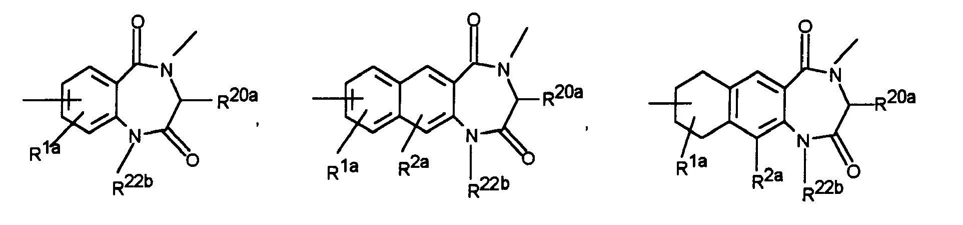 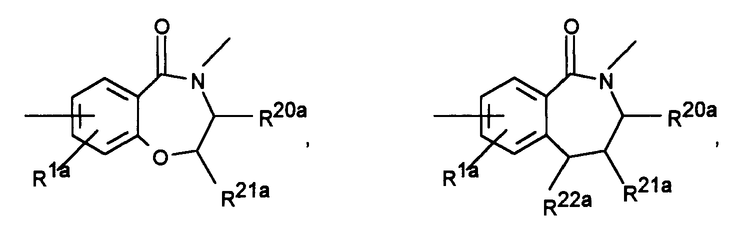 bedeutet,
wobei R^{1a}, R^{2a}, R^{20a}, R^{21a}, R^{22a} bedeuten:
R^{1a}, R^{2a}, unabhängig voneinander eine bis drei Gruppen aus der Reihe Wasserstoff, Halogen, Cyano, Carboxamido, Carbamoyloxy, Formyloxy, Formyl, Azido, Nitro, Ureido, Thioureido, Hydroxy, Mercapto, Sulfonamido oder einen gegebenenfalls substituierten Rest aus der Reihe C₁-C₁₂-Alkyl, C₂-C₁₂-Alkenyl, C₃-C₁₂-Alkinyl, C₃-C₁₂-Cycloalkyl, C₆-C₁₄-Aryl, C₆-C₁₀-Aryl-C₁-C₈-alkyl, C₁-C₁₂-Alkyloxy,C₆-C₁₄-Aryloxy und C₁-C₁₂-Acylamino, wobei die Substituenten einen Rest aus der Reihe Halogen, Cyano, Azido, Nitro, Hydroxy, Mercapto, Sulfonamido, Ureido, Thioureido, Carboxamido, Carbamoyloxy, Formyloxy, Formyl, C₁-C₄-Alkoxy, Phenyl und Phenoxy bedeuten;
R^{20a} Wasserstoff, Halogen (Fluor, Chlor, Brom, Iod), C₁-C₄-Alkoxy, C₁-C₄-Alkyl, Phenyl, Benzyl oder Halogen-C₁-C₄-Alkyl bedeutet,
R^{21a}, R^{22a}, unabhängig voneinander
1. Wasserstoff
2. (C₁-C₁₂)-Alkyl
3. (C₆-C₁₄)-Aryl,
4. (C₃-C₁₄)-Cycloalkyl,
5. (C₁-C₁₂)-Alkyl-(C₆-C₁₄)-aryl,
6. (C₁-C₁₂)-Alkyl-(C₃-C₁₄)-cycloalkyl bedeutet, wobei die unter 2. bis 6. definierten Reste substituiert sein können mit einem oder mehreren Resten aus der Reihe Halogen (Fluor, Chlor, Brom, Iod); Nitro; Hydroxyl; Carboxyl; Tetrazol; Hydroxamat; Sulfonamid; Trifluorimid; Phosphonat; C₁-C₆-Alkyl; C₆-C₁₄-Aryl; Benzyl; C₃ -C₁₄-Cycloalkyl; COR^{24a}; CONR²⁵R²⁶; wobei
R^{24a} einen Rest aus der Reihe C₁-C₈-Alkoxy; C₃-C₁₂-Alkenoxy; C₆-C₁₂-Aryloxy; di-C₁-C₈-Alkylamino-C₁-C₈-alkoxy; Acylamino-C₁-C₈-Alkoxy; Acetylaminoethoxy; Nicotinoylaminoethoxy; Succinamidethoxy; Pivaloylethoxy; C₆-C₁₂-Aryl-C₁-C₈-alkoxy, wobei die Arylgruppe gegebenenfalls mit ein bis drei Resten aus der Reihe Nitro, Halogen, C₁-C₄-Alkoxy, Amino, Hydroxyl, Hydroxy-C₂-C₈-Alkoxy, Dihydroxy-C₃-C₈-Alkoxy substituiert sein kann;
R²⁵ und R²⁶ unabhängig voneinander Wasserstoff, C₁-C₁₀-Alkyl, C₃-C₁₀-Alkenyl, C₆-C₁₄-Aryl, C₁-C₆-Alkyl-C₆-C₁₀-aryl bedeuten oder
R²⁵ und R²⁶ zusammen einen Trimethylen-, Tetramethylen-, Pentamethylen- oder 3-Oxopentamethylenrest bilden;
7. Q²-L³ bedeutet, wobei
Q² Wasserstoff oder Q¹ ist; und
L³ eine chemische Bindung, L¹ oder L² bedeutet;
Q¹ ein substituierter oder unsubstituierter, positiv geladener, Stickstoff-ent-haltender Rest ist,
L¹ ein zweiwertiger Rest ist, der 3 bis 9 Methylengruppen enthält, wobei eine bis alle Methylengruppen durch eine oder mehrere Alkengruppe, Alkingruppe, Arylgruppe oder funktionelle Gruppe enthaltend Heteroatome aus der Reihe N, O oder S, ersetzt sein können, und
L² ein gegebenenfalls substituierter, zweiwertiger Rest ist;
und R^{22b}
1. Wasserstoff
2. (C₁-C₁₂)-Alkyl
3. (C₆-C₁₄)-Aryl,
4. (C₃-C₁₄)-Cycloalkyl,
5. (C₁-C₁₂)-Alkyl-(C₆-C₁₄)-aryl,
6. (C₁-C₁₂)-Alkyl-(C₃-C₁₄)-cycloalkyl bedeutet, wobei die unter 2. bis 6. definierten Reste substituiert sein können mit einem oder mehreren Resten aus der Reihe Halo-gen (Fluor, Chlor, Brom, Iod); Nitro; Hydroxyl; Carboxyl; Tetrazol; Hydroxamat; Sulfonamid; Trifluorimid; Phosphonat; C₁-C₆-Alkyl; C₆-C₁₄-Aryl; Benzyl; C₃-C₁₄-Cycloalkyl; COR^{24a}; CONR²⁵R²⁶; wobei
R^{24a} einen Rest aus der Reihe C₁-C₈-Alkoxy; C₃-C₁₂-Alkenoxy; C₆-C₁₂-Aryloxy; di-C₁-C₈-Alkylamino-C₁-C₈-alkoxy; Acylamino-C₁-C₈-Alkoxy; Acetylaminoethoxy; Nicotinoylaminoethoxy; Succinamidethoxy; Pivaloylethoxy; C₆-C₁₂-Aryl-C₁-C₈-alkoxy, wobei die Arylgruppe gegebenenfalls mit ein bis drei Resten aus der Reihe Nitro, Halogen, C₁-C₄-Alkoxy, Amino, Hydroxyl, Hydroxy-C₂-C₈-Alkoxy, Dihydroxy-C₃-C₈-Alkoxy substituiert sein kann;
R²⁵ und R²⁶ unabhängig voneinander Wasserstoff, C₁-C₁₀-Alkyl, C₃-C₁-Alkenyl, C₆-C₁₄-Aryl, C₁-C₆-Alkyl-C₆-C₁₀-aryl bedeuten oder
R²⁵ und R²⁶ zusammen einen Trimethylen-, Tetramethylen-, Pentamethylen- oder 3-Oxopentamethylenrest bilden;
7. Q²-L³ bedeutet, wobei
Q² Wasserstoff oder Q¹ ist; und
L³ eine chemische Bindung, L¹ oder L² bedeutet;
Q¹ ein substituierter oder unsubstituierter, positiv geladener, Stickstoff-enthaltender Rest ist,
L¹ ein zweiwertiger Rest ist, der 3 bis 9 Methylengruppen enthält, wobei eine bis alle Methyenlgruppen durch eine oder mehrere Alkenreste, Alkinreste, Arylreste oder funktionelle Gruppen enthaltend Heteroatome aus der Reihe N, O oder S ersetzt sein können, und
L² ein gegebenenfalls substituierter, zweiwertiger Rest ist;
oder b) 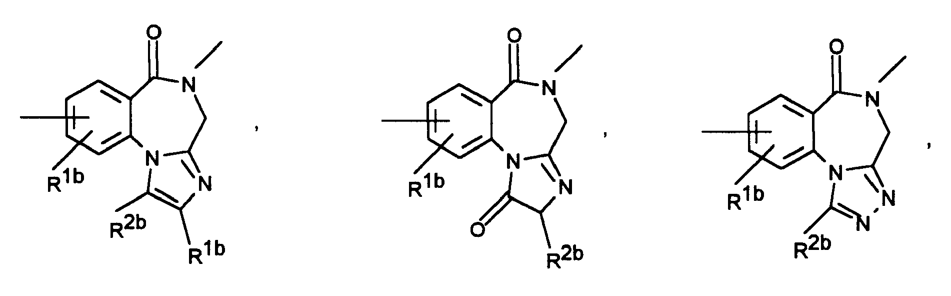 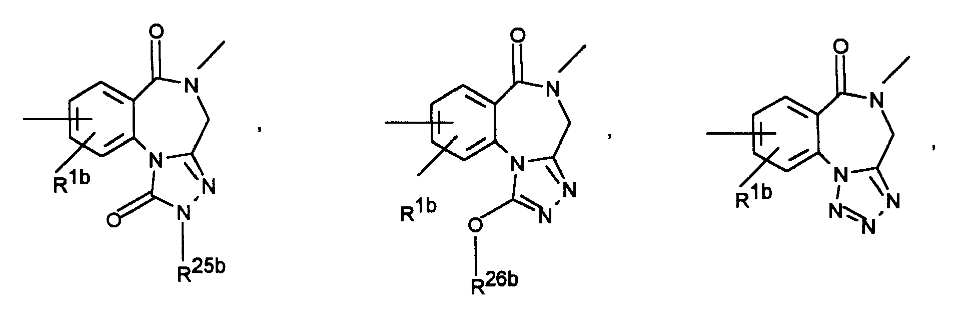 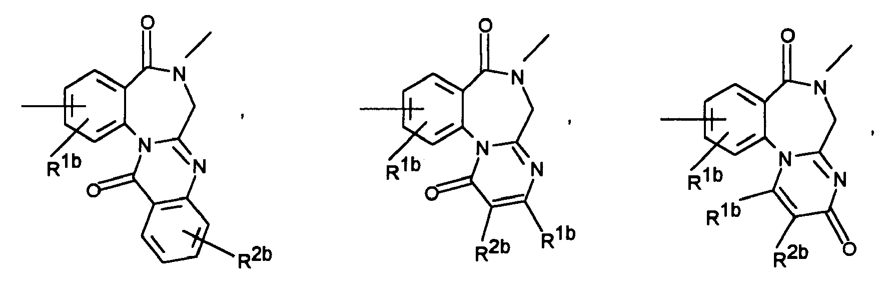 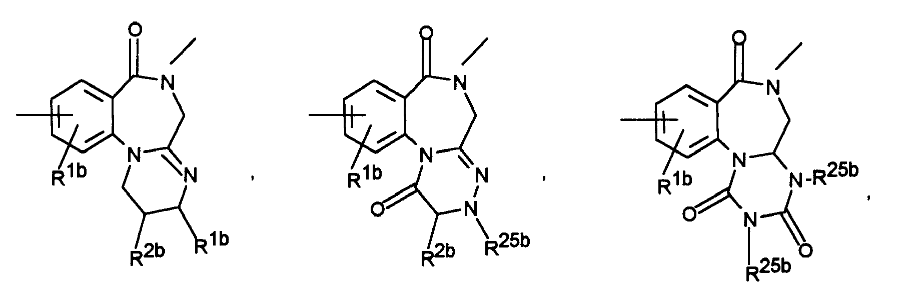 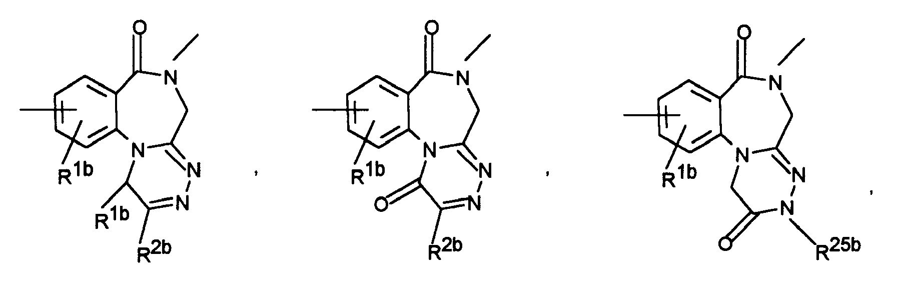 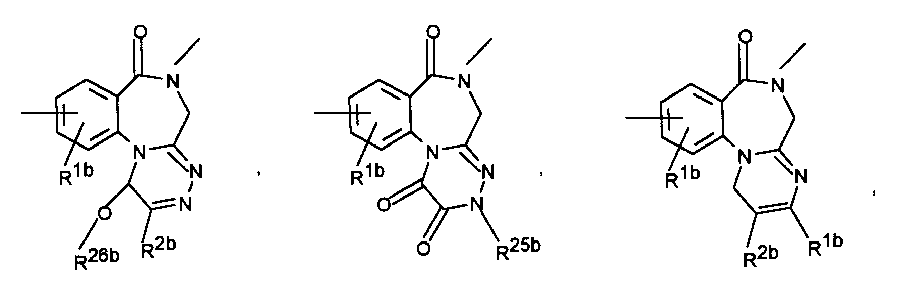 wobei R^{1b} und R^{2b} bedeuten:
R^{1b}, R^{2b} unabhängig voneinander ein bis drei Gruppen aus der Reihe Wasserstoff, Halogen, Cyano, Carboxamido, Carbamoyloxy, Formyloxy, Formyl, Azido, Nitro, Ureido, Thioureido, Hydroxy, Mercapto, Sulfonamido oder ein gegebenenfalls substituierter Rest aus der Reihe C₁-C₁₂-Alkyl, C₂-C₁₂-Alkenyl, C₃-C₁₂-Alkinyl, C₃-C₁₂-Cycloalkyl, C₆-C₁₄-Aryl, C₆-C₁₀-Aryl-C₁-C₈-alkyl, C₁-C₁₂-AlkoxyC₆-C₁₄-Aryloxy und C₁-C₁₂-Acylamino, wobei die Substituenten einen Rest aus der Reihe Halogen, Cyano, Azido, Nitro, Hydroxy, Mercapto, Sulfonamido, Ureido, Thioureido, Carboxamido, Carbamoyloxy, Formyloxy, Formyl, C₁-C₄-Alkoxy, Phenyl und Phenoxy darstellen; und
R^{25b} und R^{26b} unabhängig voneinander Wasserstoff, C₁-C₁₀-Alkyl, C₃-C₁₀-Alkenyl, C₆-C₁₄-Aryl, C₁-C₆-Alkyl-C₆-C₁₀-aryl bedeuten oder
R^{25b} und R^{26b} zusammen einen Trimethylen-, Tetramethylen-, Pentamethylen- oder 3-Oxopentamethylenrest bilden;
oder c) 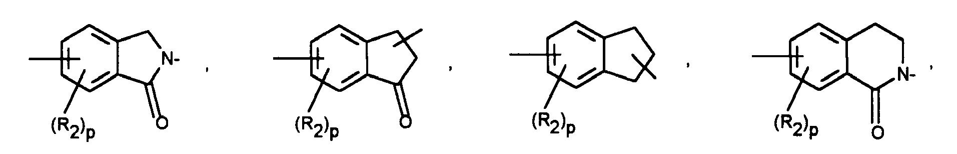 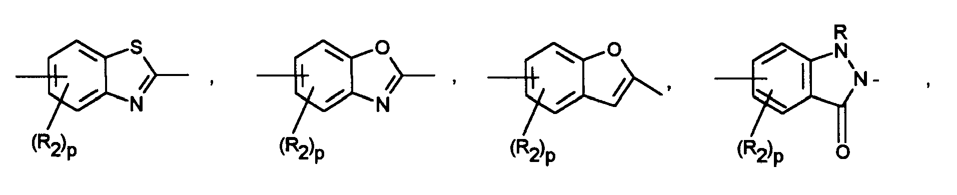 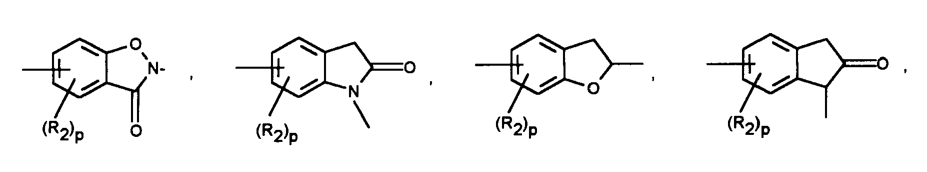 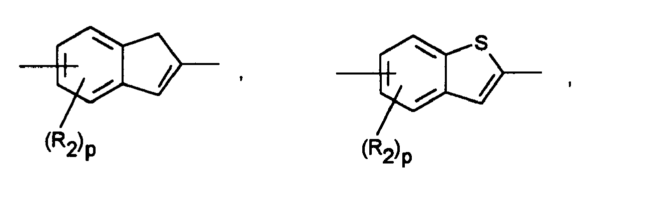 bedeutet,
wobei
(R₂)ₚ an ein oder mehrere C-Atomen des 6-gliedrigen Rings gebunden ist und unabhängig voneinander einen Rest aus der Reihe H, Alkyl, halogensubstituiertes Alkyl, Hydroxyalkyl, Alkenyl, Alkinyl, Cycloalkyl, Aryl, Aryloxy, Aralkyl, Hydroxy, Alkoxy, Aralkoxy, Carbamyl, Amino, substituiertes Amino, Acyl, Cyano, Halogen, Nitro und Sulfo bedeutet;
R (C₁-C₄)-Alkyl bedeutet
p eine ganze Zahl von 1 bis 3 ist,
oder d) 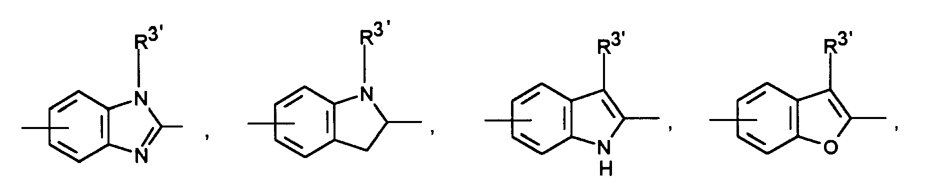 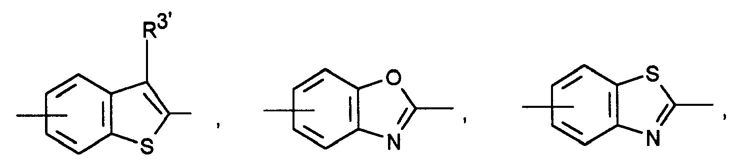 bedeutet wobei R^{3'} Wasserstoff, (C₁-C₆)-Alkyl, Aryl-C₁-C₆-alkyl ist,
oder e) 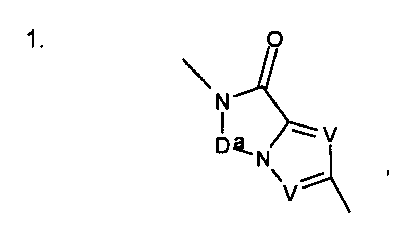
worin V CR^{7a} oder N ist, und
D^{a} CH₂, CH₂-CH₂, CH₂C(R^{7a})₂CH₂ oder 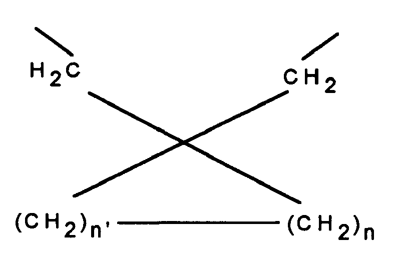
bedeutet; 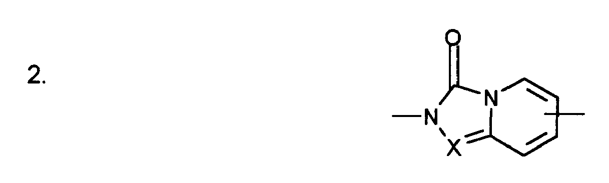
worin X CR^{3a} oder N ist,
wobei R^{3a} CN, C(O)N(R^{7a})R^{8a}, 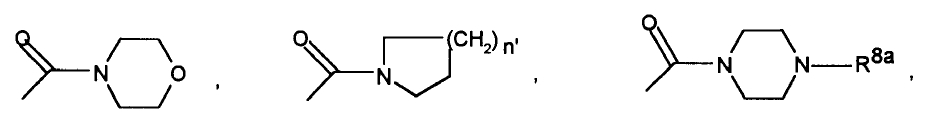 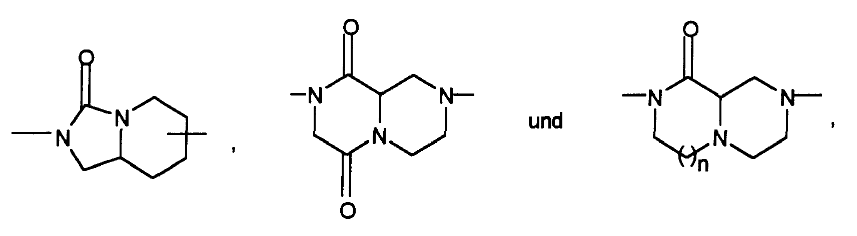
bedeutet; 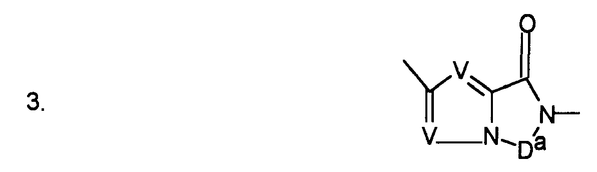
worin V CR⁷, oder N bedeutet, und
D^{a} CH₂, CH₂-CH₂, CH₂C(R^{7a})₂CH₂ oder bedeutet; 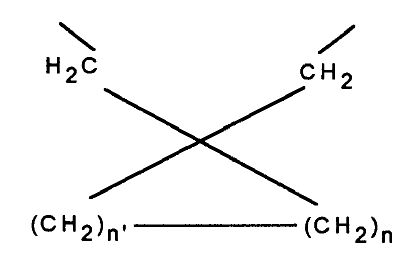 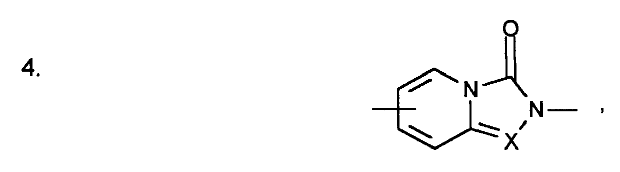
worin X CR^{3a} oder N bedeutet, worin
R^{3a} CN, C(O)N(R^{7a})R^{8a}, 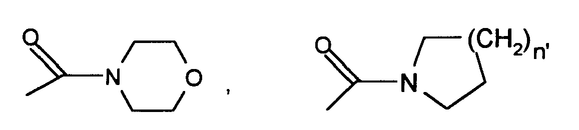
bedeutet; 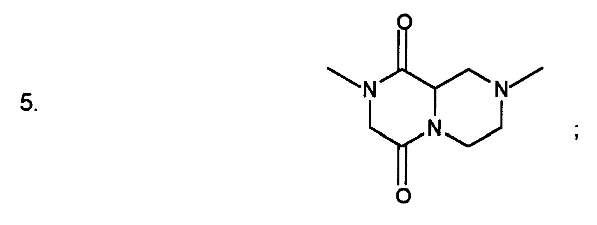 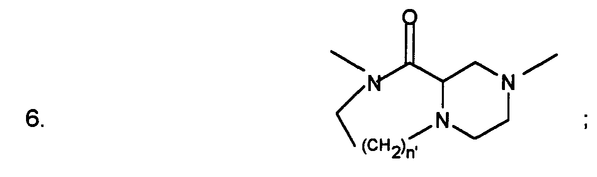 und 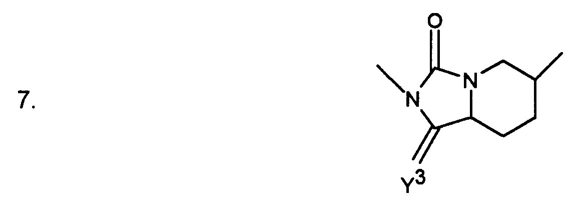
wobei Y³ O oder H₂ ist und
R^{7a} Wasserstoff; C₁-C₄-Alkyl, gegebenenfalls substituiert mit OH oder (C₁-C₄)-Alkoxy; C₂-C₆-Alkenyl, gegebenenfalls substituiert mit (C₁-C₄)-Alkoxy; oder OH (C₁-C₄)-Alkylaryl; Aryl gegebenenfalls substituiert mit gleichen oder verschiedenen Resten aus der Reihe Halogen, (C₁-C₄)-Alkoxy, Hydroxy oder (C₁-C₄)-Alkyl, bedeutet,
R^{8a} Wasserstoff oder C₁-C₄-Alkyl bedeutet
n eine ganze Zahl von 0 bis 7 und
n' eine ganze Zahl von 0 bis 3 ist;
oder f) 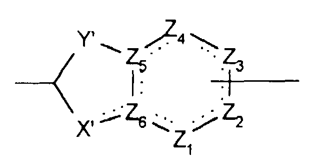 bedeutet,
wobei bedeuten:
X' ein Sauerstoff-, Schwefel- oder Stickstoffatom oder eine -NR^{2b}-Gruppe, wobei
R^{2b} ein Wasserstoffatom, eine geradkettige oder verzweigte Alkylgruppe mit 1 bis 15 C-Atomen, eine geradkettige oder verzweigte Alkenyl- oder Alkinylgruppe mit jeweils 3 bis 10 Kohlenstoffatomen, wobei die Doppel- oder Dreifachbindung nicht unmittelbar an das Stickstoffatom anschließen kann, eine Cycloalkyl- oder Cycloalkylalkylgruppe mit jeweils 3 bis 7 Kohlenstoffatomen im Cycloalkylteil, eine Arylgruppe, eine Alkylgruppe mit 2 bis 6 Kohlenstoffatomen, die ab der β-Position zu dem Stickstoffatom der -NR^{2b}-Gruppe durch eine R^{3b}O-, (R^{3b})₂N-, R^{4b}CO-NR^{3b}-, Alkylsulfonyl-NR^{3b} -, Arylsulfonyl-NR^{3b}-, Alkylsulfenyl-, Alkylsulfinyl-, Alkylsulfonyl- oder R^{5b}-Gruppe substituiert ist, oder eine Alkylgruppe mit 1 bis 6 Kohlenstoffatomen, die durch eine oder zwei Arylgruppen, R^{6b}OCO-, (R^{3b})₂NCO-, R^{5b}-CO-, R^{3b}O-CO-alkylen-NR₃-CO-, (R^{3b})₂N-CO-alkylen-NR^{3b}-CO- oder R^{5b}CO-alkylen-NR^{3b}-CO-Gruppe substituiert ist, in denen R^{3b} und R^{5b} wie nachstehend definiert sind und R^{6b} ein Wasserstoffatom, eine Alkylgruppe mit 1 bis 6 Kohlenstoffatomen, eine Cycloalkylgruppe mit 5 bis 7 Kohlenstoffatomen oder eine Aralkylgruppe darstellt,
Y' eine NO-Gruppe, ein Stickstoffatom oder eine gegebenenfalls durch eine Alkylgruppe substituierte Methingruppe,
Z₁, Z₂, Z₃ und Z₄, die gleich oder verschieden sein können, Methingruppen, Kohlenstoffatome, Iminogruppen oder Stickstoffatome, wobei mindestens einer der Reste Z₁ bis Z₄ ein Kohlenstoffatom enthalten muß und eine oder zwei zu einem Stickstoffatom benachbarte Methingruppen jeweils durch Carbonylgruppen ersetzt sein können,
Z₅ und Z₆ jeweils ein Kohlenstoffatom oder auch einer der Reste Z₅ oder Z₆ ein Stickstoffatom und der andere der Reste Z₅ oder Z₆ ein Kohlenstoffatom,
R^{3b} ein Wasserstoffatom, eine Alkylgruppe mit 1 bis 6 Kohlenstoffatomen, eine Aryl-, Aralkyl-, Carboxyalkyl- oder Alkoxycarbonylalkylgruppe,
R^{4b} ein Wasserstoffatom, eine Alkyl- oder Alkoxygruppe mit jeweils 1 bis 6 Kohlenstoffatomen, eine Aryl- oder Aralkylgruppe mit 1 bis 6 Kohlenstoffatomen im Alkylteil und
R^{5b} eine Azetidino-, Pyrrolidino-, Hexamethylenimino- oder Heptamethyleniminogruppe oder eine Piperidinogruppe, in der die Methylengruppe in 4-Stellung durch ein Sauerstoffatom, eine Sulfenyl-, Sulfinyl- oder Sulfonylgruppe oder durch eine durch eine R₃-, R₄CO-, Alkylsulfonyl- oder Arylsulfonylgruppe substituierte Iminogruppe ersetzt sein kann, wobei R₃ und R₄ wie vorstehend erwähnt, definiert sind, darstellen;
F eine direkte Bindung, (C₁-C₆)-Alkandiyl, -O-, -CO-NR²-, -NR²-CO-, -NR²-C(O)-NR²-, -OC(O)-, -C(O)O-, - CO, S(O)₂-, -S(O)₂-NR²-, -NR²-S(O)₂-, -CR²=CR³-, -C≡C-, die jeweils ein- oder zweifach durch (C₁-C₆)-Alkyl substituiert sein können;
G 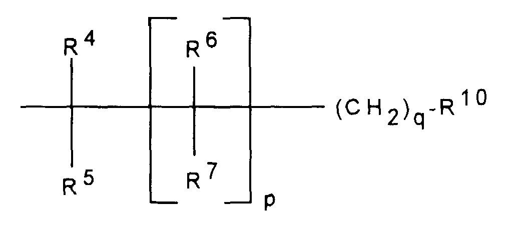
R², R³ unabhängig voneinander H, (C₁-C₆)-Alkyl, das gegebenenfalls durch Fluor ein- oder mehrfach substituiert ist, (C₅-C₆)-Cycloalkyl, (C₅-C₆)-Cycloalkyl-(C₁-C₄)-alkyl, (C₅-C₁₀)-Aryl, (C₅-C₁₀)-Aryl-(C₁-C₄)-alkyl, R⁸OC(O)R⁹, R⁸R⁸NC(O)R⁹, R⁸C(O)R⁹;
R⁴, R⁵, R⁶, R⁷ unabhängig voneinander H, Fluor, OH, (C₁-C₆)-Alkyl, (C₅-C₁₄)-Cycloalkyl, (C₅-C₁₄)-Cycloalkyl-(C₁-C₆)-alkyl, oder R⁸OR⁹, R⁸CO₂R⁹, R⁸OC(O)R⁹, R⁸-(C₅-C₁₀)-Aryl-R⁹, R⁸NHR⁹, R⁸R⁸NR⁹, R⁸NHC(O)OR⁹, R⁸S(O)ₙNHR⁹, R⁸OC(O)NHR⁹, R⁸C(O)NHR⁹, R⁸C(O)R⁹, R⁸NHC(O)NHR⁹, R⁸NHS(O)ₙNHR⁹, R⁸NHC(O)R⁹, R⁸NHS(O)ₙR⁹, wobei mindestens ein Rest aus der Reihe R⁴, R⁵, R⁶, R⁷ einen lipophilen Rest bedeutet;
R⁸ H, (C₁-C₆)-Alkyl, (C₅-C₁₄)-Cycloalkyl, (C₅-C₁₄)-Cycloalkyl-(C₁-C₄)-alkyl, (C₅-C₁₀)-Aryl, (C₅-C₁₀)-Aryl-(C₁-C₄)-alkyl, wobei die Alkylreste mit 1 bis 6 Fluor-Atomen substituiert sein können;
R⁹ eine direkte Bindung oder (C₁-C₆)-Alkandiyl;
R¹⁰ C(O)R¹¹;
R¹¹ OH, (C₁-C₆)-Alkoxy, (C₅-C₁₀)-Aryl-(C₁-C₆)-alkoxy, (C₅-C₁₀)-Aryloxy, (C₁-C₆)-Alkylcarbonyloxy-(C₁-C₄)-alkoxy, (C₅-C₁₀)-Aryl-(C₁-C₄)-alkylcarbonyloxy-(C₁-C₄)-alkoxy, NH₂, Mono-oder Di-((C₁-C₆)-alkyl)-amino;
R¹² H, (C₁-C₆)-Alkyl, das gegebenenfalls durch Fluor ein- oder mehrfach substituiert ist, (C₃-C₆)-Cycloalkyl, (C₃-C₆)-Cycloalkyl-(C₁-C₄)-alkyl, (C₅-C₁₀)-Aryl, (C₅-C₁₀)-Aryl-(C₁-C₄)-alkyl, H₂N, R⁸OR⁹, R⁸OC(O)R⁹, R⁸-(C₅-C₁₀)-Aryl-R⁹, R⁸R⁸NR⁹, R⁸NHC(O)R⁹, R⁸C(O)NHR⁹, H₂N-C(=NH)-, H₂N-C(=NH)-NH-, =O;
wobei zwei benachbarte Substituenten R¹² ferner -OCH₂O-, -OCH₂CH₂O-bedeuten können;
Y NR², O, S;
n 1 oder 2; und
p, q unabhängig voneinander 0 oder 1;
in allen ihren stereoisomeren Formen und Mischungen davon in allen Verhältnissen, und ihre physiologisch verträglichen Salze.

4. Verbindung der Formel I gemäß einem oder mehreren der Ansprüche 1 bis 3, worin bedeuten:
A einen der Reste 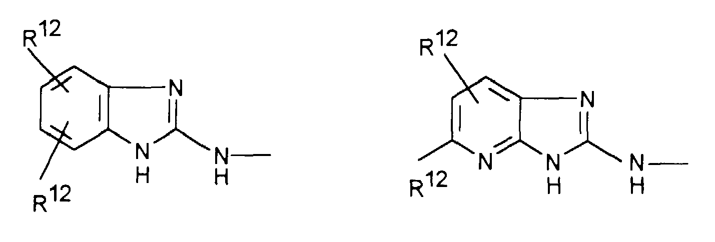 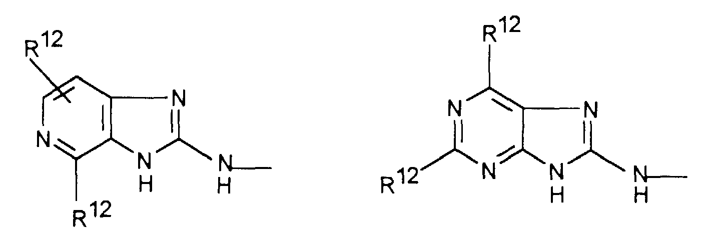 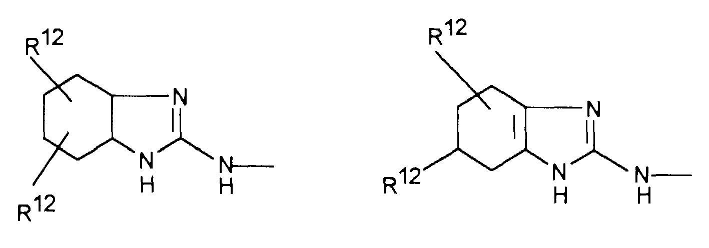
B eine direkte Bindung, (C₁-C₄)-Alkandiyl, Phenylen, Pyridindiyl, Thiophendiyl, Furandiyl, Cyclohexylen, Cyclopentylen, -C≡C-, -CR²=CR³-, die jeweils ein- oder zweifach durch (C₁-C₄)-Alkyl substituiert sein können;
D eine direkte Bindung, (C₁-C₄)-Alkandiyl oder Phenylen, -O-, -NR²-, -NR²-C(O)-, -C(O)-NR²-, -NR²-S(O)₂, - NR²-C(O)-NR²-, -CR²=CR³-, die jeweils ein- oder zweifach durch (C₁-C₄)-Alkyl substituiert sein können, wobei, wenn B eine direkte Bindung ist, D nicht gleich -C(O)-NR²- sein kann;
E
a) 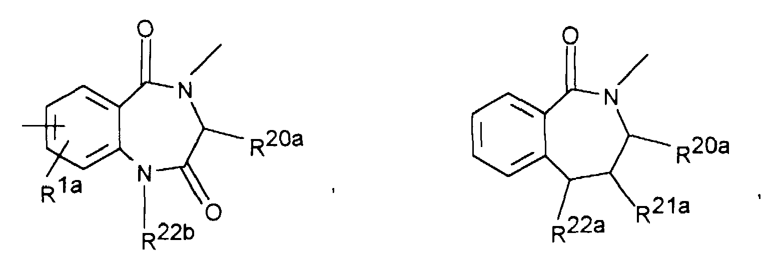 wobei hier R^{1a}, R^{20a}, R^{21a}, R^{22a} und R^{22b} bedeuten:
R^{1a} unabhängig (Fluor, voneinander eins bis drei Gruppen aus der Reihe Wasserstoff und Halogen Chlor, Brom, Iod);
R^{20a} Wasserstoff;
R^{21a}, R^{22a}, unabhängig voneinander
1. Wasserstoff,
2. (C₁-C₆)-Alkyl,
3. (C₆-C₁₂)-Aryl,
4. (C₆-C₁₂)-Cycloalkyl,
5. (C₁-C₆)-Alkyl-(C₆-C₁₂)-aryl,
6. (C₁-C₆)-Alkyl-(C₆-C₁₂)-cycloalkyl bedeutet,
wobei die unter 2. bis 6. definierten Reste substituiert sein können mit einem oder mehreren Resten aus der Reihe
Fluor, Chlor, Hydroxyl, Hydroxamat, Sulfonamid, (C₁-C₆)-Alkyl, (C₆-C₁₂)-Aryl, Benzyl, (C₆-C₁₂)-Cycloalkyl ;
R^{22b}
1. Wasserstoff
2. (C₁-C₁₂)-Alkyl
3. (C₆-C₁₄)-Aryl,
4. (C₃-C₁₄)-Cycloalkyl,
5. (C₁-C₁₂)-Alkyl-(C₆-C₁₄)-aryl,
6. (C₁-C₁₂)-Alkyl-(C₃-C₁₄)-cycloalkyl bedeutet, wobei die unter 2. bis 6. definierten Reste substituiert sein können mit einem oder mehreren Resten aus der Reihe Halogen (Fluor, Chlor, Brom, Iod); Nitro; Hydroxyl; Carboxyl; Tetrazol; Hydroxamat; Sulfonamid; Trifluorimid; Phosphonat; C₁-C₆-Alkyl; C₆-C₁₄-Aryl; Benzyl; C₃ -C₁₄-Cycloalkyl; COR^{24a}; CONR²⁵R²⁶; wobei
R^{24a} einen Rest aus der Reihe C₁-C₈-Alkoxy; C₃-C₁₂-Alkenoxy; C₆-C₁₂-Aryloxy; di-C₁-C₈-Alkylamino-C₁-C₈-alkoxy; Acylamino-C₁-C₈-Alkoxy; Acetylaminoethoxy; Nicotinoylaminoethoxy; Succinamidethoxy; Pivaloylethoxy; C₆-C₁₂-Aryl-C₁-C₈-alkoxy, wobei die Arylgruppe gegebenenfalls mit ein bis drei Resten aus der Reihe Nitro, Halogen, C₁-C₄-Alkoxy, Amino, Hydroxyl, Hydroxy-C₂-C₈-Alkoxy, Dihydroxy-C₃-C₈-Alkoxy substituiert sein kann;
R²⁵ und R²⁶ unabhängig voneinander Wasserstoff, C₁-C₁₀-Alkyl, C₃-C₁₀-Alkenyl, C₆-C₁₄-Aryl, C₁-C₆-Alkyl-C₆-C₁₀-aryl bedeuten oder
R²⁵ und R²⁶ zusammen einen Trimethylen-, Tetramethylen-, Pentamethylen- oder 3-Oxopentamethylenrest bilden;
7. Q²-L³ bedeutet, wobei
Q² Wasserstoff oder Q¹ ist; und
L³ eine chemische Bindung oder L¹ bedeutet;
Q¹ eine Amino-, Amidino-, Aminoalkylenimino-, Iminoalkylenamino- oder Guanidinogruppe, vorzugsweise eine Amidinogruppe bedeutet;
L¹ C₆-C₁₄-Aryl-C₂-C₄-alkinylen; C₆-C₁₄-Aryl-C₁-C₃-alkylen; C₆-C₁₄-Aryl-C₁-C₃-alkyloxyen oder -R^{14c}-CO-NR^{6c}R^{15c}- bedeutet, wobei
R^{6c} Wasserstoff, C₁-C₄-Alkoxy, C₁-C₄-Alkyl oder Halogen-C₁-C₄-alkyl bedeutet;
R^{14c} eine chemische Bindung, C₁-C₈-Alkylen, C₃-C₇-Cycloalkylen, C₂-C₅-Alkenylen, C₃-C₅-Alkinylen, C₆-C₁₀-Arylen, C₁-C₃-Alkyl-C₆-C₁₂-arylen, C₁-C₂-Alkyl-C₆-C₁₀-aryl-C₁-C₂-alkylen, C₆-C₁₀-aryl-C₁-C₂-alkylen oder C₆-C₁₀-Aryloxy-C₁-C₂-alkylen bedeutet, und
R^{15c} eine chemische Bindung, C₁-C₄-Alkylen, C₂-C₄-Alkenylen, C₂-C₄-Alkinylen, C₆-C₁₀-Arylen oder C₁-C₃-Alkyl-C₆-C₁₂-arylen bedeutet;
oder b) 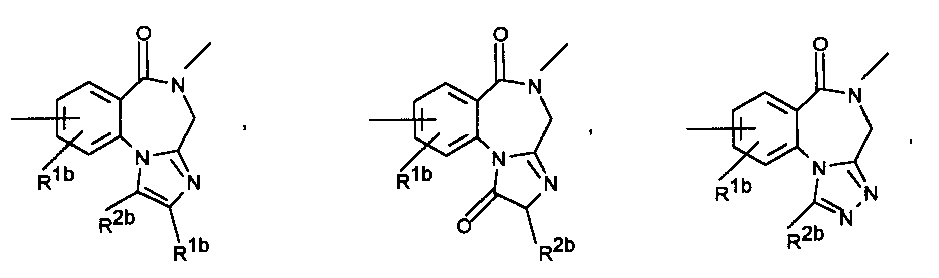 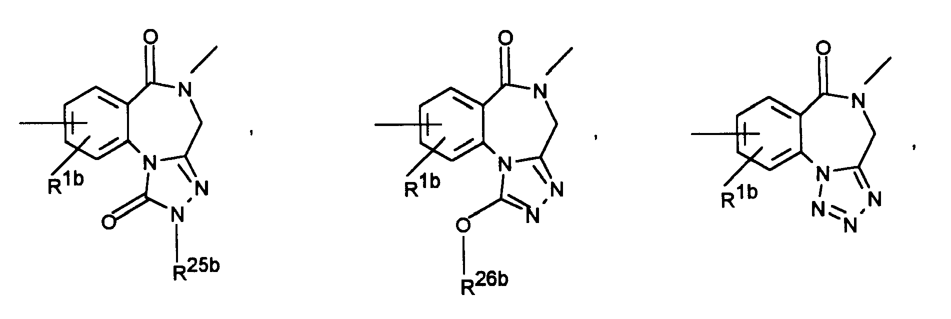 wobei hier R^{1b}, R^{2b}, R^{25b} und R^{26b} bedeuten:
R^{1b}, R^{2b} unabhängig voneinander eine bis drei Gruppen aus der Reihe Wasserstoff und Halogen (Fluor, Chlor, Brom, Iod); und
R^{25b} und R^{26b} unabhängig voneinander Wasserstoff, C₁-C₁₀-Alkyl, C₃-C₁₀-Alkenyl, C₆-C₁₄-Aryl, C₁-C₆-Alkyl-C₆-C₁₀-aryl bedeuten oder
R^{25b} und R^{26b} zusammen einen Trimethylen-, Tetramethylen-, Pentamethylen- oder 3-Oxopentamethylenrest bilden;
oder c) 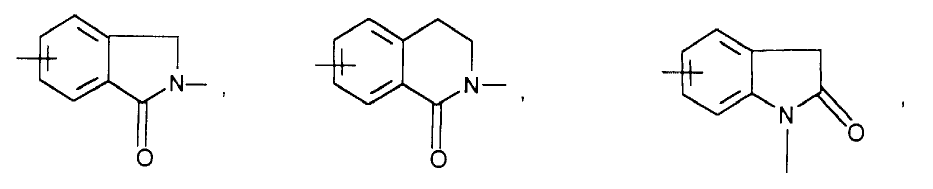 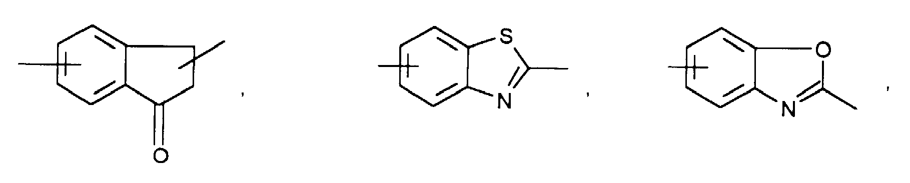 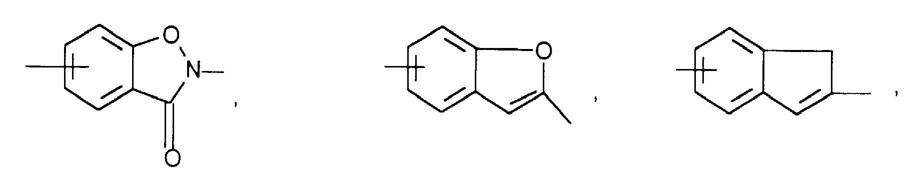 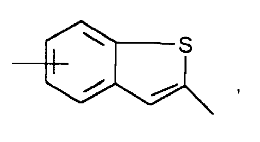
oder d) 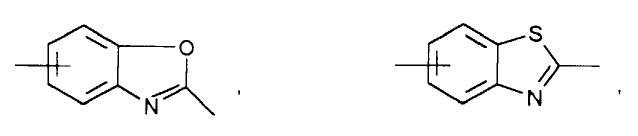 oder e) 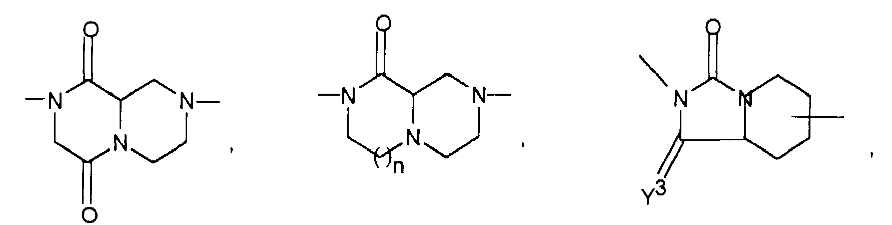 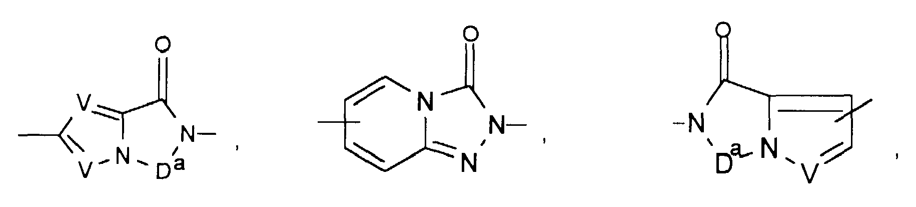 wobei Y³, V und D^{a} wie oben beschrieben, definiert sind;
oder f) 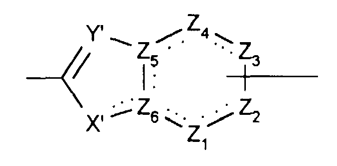 wobei bedeuten:
X' ein Sauerstoff-, Schwefel- oder Stickstoffatom oder eine -NR^{2b}-Gruppe, wobei
R^{2b} ein Wasserstoffatom, eine geradkettige oder verzweigte Alkylgruppe mit 1 bis 15 C-Atomen, eine geradkettige oder verzweigte Alkenyl- oder Alkinylgruppe mit jeweils 3 bis 10 Kohlenstoffatomen, wobei die Doppel- oder Dreifachbindung nicht unmittel-bar an das Stickstoffatom anschließen kann, eine Cycloalkyl- oder Cycloalkylalkylgruppe mit jeweils 3 bis 7 Kohlenstoffatomen im Cycloalkylteil, eine Arylgruppe, eine Alkylgruppe mit 2 bis 6 Kohlenstoffatomen, die ab der β-Position zu dem Stickstoffatom der -NR^{2b}-Gruppe durch eine R^{3b}O-, (R^{3b})₂N-, R^{4b}CO-NR^{3b}-, Alkylsulfonyl-NR^{3b}-, Arylsulfonyl-NR^{3b}-, Alkylsulfenyl-, Alkylsulfinyl-, Alkylsulfonyl- oder R^{5b}-Gruppe substituiert ist, oder eine Alkylgruppe mit 1 bis 6 Kohlenstoffatomen, die durch eine oder zwei Arylgruppen, R^{6b}OCO-, (R^{3b})₂NCO-, R^{5b} -CO-, R^{3b}O-CO-alkylen-NR^{3b}-CO-, (R^{3b})₂N-CO-alkylen-NR^{3b}-CO- oder R^{5b}CO-alkylen-NR^{3b}-Co-Gruppe substituiert ist, in denen R^{3b} und R^{5b} wie nachstehend definiert sind und R^{6b} ein Wasserstoffatom, eine Alkylgruppe mit 1 bis 6 Kohlenstoffatomen, eine Cycloalkylgruppe mit 5 bis 7 Kohlenstoffatomen oder eine Aralkylgruppe darstellt,
Y' eine NO-Gruppe, ein Stickstoffatom oder eine gegebenenfalls durch eine Alkyl-gruppe substituierte Methingruppe,
Z₁, Z₂, Z₃ und Z₄, die gleich oder verschieden sein können, Methingruppen, Kohlenstoffatome, Iminogruppen oder Stickstoffatome, wobei mindestens einer der Reste Z₁ bis Z₄ ein Kohlenstoffatom enthalten muß und eine oder zwei zu einem Stickstoffatom benachbarte Methingruppe jeweils durch Carbonylgruppen ersetzt sein können,
Z₅ und Z₆ jeweils ein Kohlenstoffatom oder auch einer der Reste Z₅ oder Z₆ ein Stickstoffatom und der andere der Reste Z₅ oder Z₆ ein Kohlenstoffatom bedeuten,
R^{3b} ein Wasserstoffatom, eine Alkylgruppe mit 1 bis 6 Kohlenstoffatomen, eine Aryl-, Aralkyl-, Carboxyalkyl- oder Alkoxycarbonylalkylgruppe,
R^{4b} ein Wasserstoffatom, eine Alkyl- oder Alkoxygruppe mit jeweils 1 bis 6 Kohlenstoffatomen, eine Aryl- oder Aralkylgruppe mit 1 bis 6 Kohlenstoffatomen im Alkylteil und
R^{5b} eine Azetidino-, Pyrrolidino-, Hexamethylenimino- oder Heptamethyleniminogruppe oder eine Piperidinogruppe, in der die Methylengruppe in 4-Stellung durch ein Sauerstoffatom, eine Sulfenyl-, Sulfinyl- oder Sulfonylgruppe oder durch eine durch eine R^{3b} R^{4b}CO-, Alkylsulfonyl- oder Arylsulfonylgruppe substituierte Iminogruppe ersetzt sein kann, wobei R^{3b} und R^{4b} wie vorstehend erwähnt, definiert sind, darstellen,
F eine direkte Bindung, (C₁-C₆)-Alkandiyl, -O-, -CO-NR²-, -NR²-CO-, -NR²-C(O)-NR²-, -S(O)₂-NR², -NR²-S(O)₂-, -CR²=CR³, -C≡C-, die jeweils ein- oder zweifach durch (C₁-C₄)-Alkyl substituiert sein können;
G 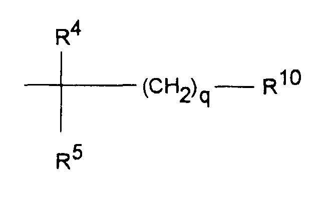
R², R³ unabhängig voneinander H, (C₁-C₄)-Alkyl, Trifluormethyl, Pentafluorethyl, (C₅-C₆)-Cycloalkyl, (C₅-C₆)-Cycloalkyl-(C₁-C₄)-alkyl, Phenyl, Benzyl;
R⁴ (C₁₀-C₁₄)-Cycloalkyl, (C₁₀-C₁₄)-Cycloalkyl-(C₁-C₄)-alkyl, oder R¹⁶OR⁹, R¹⁶NHR⁹, R¹⁶NHC(O)OR⁹, R¹⁶S(O)ₙNHR⁹, R¹⁶OC(O)NHR⁹, R¹⁶C(O)NHR⁹, R¹⁶C(O)R⁹, R¹⁶NHC(O)R⁹, R¹⁶NHS(O)ₙR⁹;
R⁵ H, (C₁-C₆)-Alkyl, (C₅-C₆)-Cycloalkyl, (C₅-C₆)-Cycloalkyl-(C₁-C₄)-alkyl, Trifluormethyl, Pentafluorethyl, Phenyl, Benzyl;
R⁸ H, (C₁-C₄)-Alkyl, (C₅-C₆)-Cycloalkyl, (C₅-C₆)-Cycloalkyl-(C₁-C₂)-alkyl, Phenyl, Benzyl, Trifluormethyl, Pentafluorethyl;
R⁹ eine direkte Bindung oder (C₁-C₄)-Alkandiyl;
R¹⁰ C(O)R¹¹;
R¹¹ OH, (C₁-C₆)-Alkoxy, Phenoxy, Benzyloxy, (C₁-C₄)-Alkylcarbonyloxy-(C₁-C₄)-alkoxy, NH₂, Mono-oder Di-((C₁-C₆)-alkyl)-amino;
R¹² H, (C₁-C₄)-Alkyl, Trifluormethyl, Pentafluorethyl, (C₅-C₆)-Cycloalkyl, (C₅-C₆)-Cycloalkyl-(C₁-C₂)-alkyl, (C₅-C₆)-Aryl, (C₅-C₆)-Aryl-(C₁-C₂)-alkyl, H₂N, R⁸R⁸NR⁹, R⁸NHC(O)R⁹, H₂N-C(=NH), H₂N-C(=NH)-NH-, wobei zwei benachbarte Substituenten R¹² ferner -OCH₂O-, -OCH₂CH₂O-bedeuten können;
R¹⁶ (C₁₀-C₁₄)-Cycloalkyl, (C₁₀-C₁₀)-Cycloalkyl-(C₁-C₄)-alkyl, die gegebenenfalls 1 oder 2-fach durch (C₁-C₄)-Alkyl, Trifluormethyl, Phenyl, Benzyl, (C₁-C₄)-Alkoxy, Phenoxy, Benzyloxy, =O oder Mono-oder Di-((C₁-C₄)-alkyl)-amino substituiert sein können, wobei die Cycloalkylenreste bevorzugt 1 - Adamantyl oder 2-Adamantyl sind, die wie vorstehend beschrieben, substituiert sein können;
n 1 oder 2; und
q 0 oder 1;
in allen ihren stereoisomeren Formen und Mischungen davon in allen Verhältnissen, und ihre physiologisch verträglichen Salze.

5. Verbindung der Formel I gemäß einem oder mehreren Ansprüche 1 bis 4, in denen der Abstand zwischen R¹⁰ und dem ersten N-Atom in A entlang des kürzesten Weges zwischen diesen Atomen 12 bis 13 kovalente Bindungen beträgt, in allen ihren stereoisomeren Formen und Mischungen davon in allen Verhältnissen, und ihre physiologisch verträglichen Salze

6. Verfahren zur Herstellung einer Verbindung der Formel I gemäß einem oder mehreren der Ansprüche 1 bis 5, **dadurch gekennzeichnet, daß** man durch Fragmentkondensation zwei oder mehrere Fragmente, die sich retrosynthetisch aus der Formel I ableiten lassen, verknüpft.

7. Verbindung der Formel I gemäß einem oder mehreren der Ansprüche 1 bis 5 und/oder ihre physiologisch verträglichen Salze zur Verwendung als Arzneimittel.

8. Verbindung der Formel I gemäß einem oder mehreren der Ansprüche 1 bis 5 und/oder ihre physiologisch verträglichen Salze zur Verwendung als Inhibitor der Knochenresorption durch Osteoclasten, als Inhibitor von Tumorwachstum oder Tumormetasierung, als Entzündungshemmer, zur Behandlung oder Prophylaxe von cardiovaskulären Erkrankungen, zur Behandlung oder Prophylaxe von Nephropathien oder Retinopathien, oder als Vitronectinrezeptor-Antagonist zur Behandlung oder Prophylaxe von Krankheiten, die auf der Wechselwirkung zwischen Vitronectinrezeptoren und deren Liganden bei Zell-Zell- oder Zell-Matrix-Interaktionsprozessen beruhen.

9. Pharmazeutische Zubereitung, enthaltend mindestens eine Verbindung der Formel I gemäß einem oder mehreren der Ansprüche 1 bis 5 und/oder ihre physiologisch verträglichen Salze neben pharmazeutisch einwandfreien Trägerstoffen und/oder Zusatzstoffen.
